# EUROPEAN PATENT SPECIFICATION

(11) **EP 4 065 725 B1**
(45) Date of publication and mention of the grant of the patent: **21.05.2025**
(21) Application number: 20828204.6
(22) Date of filing: 25.11.2020
(51) Int. Cl.: C12Q 1/68

(54) **METHODS, COMPOSITIONS AND SYSTEMS FOR IMPROVING THE BINDING OF METHYLATED POLYNUCLEOTIDES**
VERFAHREN, ZUSAMMENSETZUNGEN UND SYSTEME ZUM VERBESSERN DER BINDUNG VON METHYLIERTEN POLYNUKLEOTIDEN
PROCÉDÉS, COMPOSITIONS ET SYSTÈMES POUR AMÉLIORER LA FIXATION DE POLYNUCLÉOTIDES MÉTHYLÉS

(30) Priority: 26.11.2019 US 201962940853 P
(43) Date of publication of application: 05.10.2022
(73) Proprietor: Guardant Health, Inc., Palo Alto, CA 94304 (US)
(72) Inventor: HITE, Dustin Howard, Redwood City, California 94063 (US); GHADIRI, Farsheed, Redwood City, California 94063 (US); MORTIMER, Stefanie Ann Ward, Morgan Hill, California 95037 (US)
(74) Representative: Carpmaels & Ransford LLP
(86) International application number: PCT/US2020/062419
(87) International publication number: WO 2021/108708

(56) References cited:
- WO-A1-2017/190215
- WO-A1-2018/165366
- WO-A1-2019/006269
- DE-A1- 102012 219 142
- US-A1- 2018 220 176
- CLARE STIRZAKER ET AL: "Methylome sequencing in triple-negative breast cancer reveals distinct methylation clusters with prognostic value", NATURE COMMUNICATIONS, vol. 6, no. 5899, 1 January 2015 (2015-01-01), GB, XP055418632, ISSN: 2041-1723, DOI: 10.1038/ncomms6899
- OZREN BOGDANOVIC ET AL: "Affinity-based enrichment strategies to assay methyl-CpG binding activity and DNA methylation in early Xenopus embryos", BMC RESEARCH NOTES, BIOMED CENTRAL LTD, GB, vol. 4, no. 1, 18 August 2011 (2011-08-18), pages 300, XP021093075, ISSN: 1756-0500, DOI: 10.1186/1756-0500-4-300

## Description

### CROSS-REFERENCE TO RELATED APPLICATIONS

This application claims the benefit of priority to US Provisional Patent Application No. 62/940,853, filed November 26, 2019

### BACKGROUND

Cancer is a major cause of disease worldwide. Each year, tens of millions of people are diagnosed with cancer around the world, and more than half eventually die from it. In many countries, cancer ranks as the second most common cause of death following cardiovascular diseases. Early detection is associated with improved outcomes for many cancers.

Cancer can be caused by the accumulation of genetic variations within an individual's normal cells, at least some of which result in improperly regulated cell division. Such variations commonly include copy number variations (CNVs), single nucleotide variations (SNVs), gene fusions, insertions and/or deletions (indels), epigenetic variations include 5-methylation of cytosine (5-methylcytosine) and association of DNA with chromatin and transcription factors.

Cancers are often detected by biopsies of tumors followed by analysis of cells, markers or DNA extracted from cells. But more recently it has been proposed that cancers can also be detected from cell-free nucleic acids in body fluids, such as blood or urine. Such tests have the advantage that they are noninvasive and can be performed without identifying suspected cancer cells in biopsy. However, such liquid biopsy tests are complicated by the fact that amount of nucleic acids in body fluids is very low and what nucleic acid are present are heterogeneous in form (e.g., RNA and DNA, single-stranded and double-stranded, and various states of post-replication modification and association with proteins, such as histones).

### SUMMARY

In an aspect, the present disclosure provides a method of detecting the presence or absence of tumor in a subject comprises: (i) obtaining a polynucleotide sample from the subject; (ii) adding a set of carrier nucleic acid molecules to the polynucleotide sample to generate a first sample; wherein the set of carrier nucleic acid molecules comprises: (a) at least one subset of unmethylated carrier nucleic acid molecules; and (b) at least one subset of methylated carrier nucleic acid molecules, wherein at least one end of the carrier nucleic acid molecules is modified to prevent ligation, wherein the unmethylated carrier nucleic acid molecule does not comprise methylated nucleotides, and the methylated carrier nucleic acid molecule comprises one or more methylated nucleotides; (iii) partitioning the first sample into at least two partitioned sets using capturing agent that binds selectively to methylated polynucleotides, thereby generating a partitioned sample; (iv) processing at least a portion of the partitioned sample to generate processed sample, wherein the processing comprises at least one of the following: (a) tagging, (b) amplifying and (c) enriching molecules for specific regions of interest; (v) sequencing at least a portion of the processed sample to generate a set of sequencing reads; and (vi) analyzing at least a portion of the set of sequencing reads to detect the presence or absence of tumor. In some embodiments, sequencing step comprises sequencing at least a portion of the processed sample from at least two partitioned sets.

In another aspect, the present disclosure provides a method for analyzing polynucleotides comprises: (i) obtaining a polynucleotide sample from a subject; (ii) adding a set of carrier nucleic acid molecules to the polynucleotide sample to generate a first sample; wherein the set of carrier nucleic acid molecules comprises: (a) at least one subset of unmethylated carrier nucleic acid molecules; and (b) at least one subset of methylated carrier nucleic acid molecules, wherein at least one end of the carrier nucleic acid molecules is modified to prevent ligation, wherein the unmethylated carrier nucleic acid molecule does not comprise methylated nucleotides, and the methylated carrier nucleic acid molecule comprises one or more methylated nucleotides; (iii) partitioning the first sample into at least two partitioned sets using capturing agent that binds selectively to methylated polynucleotides, thereby generating a partitioned sample; (iv) processing at least a portion of the partitioned sample to generate processed sample, wherein the processing comprises at least one of the following: (a) tagging, (b) amplifying and (c) enriching molecules for specific regions of interest; (v) sequencing at least a portion of the processed sample to generate a set of sequencing reads; and (vi) analyzing at least a portion of the set of sequencing reads to detect the presence or absence of tumor. In some embodiments, sequencing step comprises sequencing at least a portion of the processed sample from at least two partitioned sets.

In another aspect, the present disclosure provides a method for analyzing polynucleotides comprises: (i) adding a set of carrier nucleic acid molecules to a polynucleotide sample from a subject to generate a first sample; wherein the set of carrier nucleic acid molecules comprises: (a) at least one subset of unmethylated carrier nucleic acid molecules; and (b) at least one subset of methylated carrier nucleic acid molecules, wherein at least one end of the carrier nucleic acid molecules is modified to prevent ligation, wherein the unmethylated carrier nucleic acid molecule does not comprise methylated nucleotides, and the methylated carrier nucleic acid molecule comprises one or more methylated nucleotides; (iii) partitioning the first sample into at least two partitioned sets using capturing agent that binds selectively to methylated polynucleotides, thereby generating a partitioned sample; (iv) processing at least a portion of the partitioned sample to generate processed sample, wherein the processing comprises at least one of the following: (a) tagging, (b) amplifying and (c) enriching molecules for specific regions of interest; (v) sequencing at least a portion of the processed sample to generate a set of sequencing reads; and (vi) analyzing at least a portion of the set of sequencing reads to detect the presence or absence of tumor. In some embodiments, sequencing step comprises sequencing at least a portion of the processed sample from at least two partitioned sets.

In another aspect, the present disclosure provides a method for analyzing polynucleotides comprises: (i) obtaining a polynucleotide sample from a subject; (ii) adding a set of carrier nucleic acid molecules to the polynucleotide sample to generate a first sample; wherein the set of carrier nucleic acid molecules comprises: (a) at least one subset of unmethylated carrier nucleic acid molecules; and (b) at least one subset of methylated carrier nucleic acid molecules, wherein at least one end of the carrier nucleic acid molecules is modified to prevent ligation, wherein the unmethylated carrier nucleic acid molecule does not comprise methylated nucleotides, and the methylated carrier nucleic acid molecule comprises one or more methylated nucleotides; (iii) partitioning the first sample into at least two partitioned sets using capturing agent that binds selectively to methylated polynucleotides, thereby generating a partitioned sample; (iv) processing at least a portion of the partitioned sample to generate processed sample, wherein the processing comprises at least one of the following: (a) tagging and (b) amplifying polynucleotides; (v) sequencing at least a portion of the processed sample to generate a set of sequencing reads; and (vi) analyzing at least a portion of the set of sequencing reads to detect the presence or absence of tumor. In some embodiments, sequencing step comprises sequencing at least a portion of the processed sample from at least two partitioned sets. In some embodiments, the processing further comprises enriching polynucleotides for specific regions of interest.

In another aspect, the present disclosure provides a method of detecting methylation status of polynucleotides comprises: (i) obtaining a polynucleotide sample from a subject; (ii) adding a set of carrier nucleic acid molecules to the polynucleotide sample to generate a first sample; wherein the set of carrier nucleic acid molecules comprises: (a) at least one subset of unmethylated carrier nucleic acid molecules; and (b) at least one subset of methylated carrier nucleic acid molecules, wherein at least one end of the carrier nucleic acid molecules is modified to prevent ligation, wherein the unmethylated carrier nucleic acid molecule does not comprise methylated nucleotides, and the methylated carrier nucleic acid molecule comprises one or more methylated nucleotides; (iii) partitioning the first sample into at least two partitioned sets using capturing agent that binds selectively to methylated polynucleotides, thereby generating a partitioned sample; (iv) processing at least a portion of the partitioned sample to generate processed sample, wherein the processing comprises at least one of the following: (a) tagging, (b) amplifying and (c) enriching molecules for specific regions of interest; (v) sequencing at least a portion of the processed sample to generate a set of sequencing reads; and (vi) analyzing at least a portion of the set of sequencing reads to detect the presence or absence of tumor. In some embodiments, sequencing step comprises sequencing at least a portion of the processed sample from at least two partitioned sets.

In some embodiments, analyzing at least a portion of the set of sequencing reads comprises detecting one or more somatic variations. In some embodiments, analyzing at least a portion of the set of sequencing reads comprises determining methylation status (i.e., whether the polynucleotide is methylated or not) of the polynucleotides based on the number of CpG residues (or another methylated nucleotides) and the partitioned set in which the polynucleotide gets partitioned. In some embodiments, prior to sequencing, polynucleotides in at leasttwo partitioned sets can be subjected to a chemical procedure that converts a nucleotide base selectively so as to provide information of the modification (e.g. methylation) of the nucleotide base. In these embodiments, the chemical procedure can be a bisulfite treatment, TAB-Seq, ACE-Seq, EM-Seq, hmC-Seal, TAPS or TAPSB. In these embodiments, analyzing at least a portion of the set of sequencing reads comprises determining the modification of the nucleotide base dependent upon the nucleotide base conversion. In some embodiments, the methods and systems used for partitioning may be found in PCT Patent Application No. PCT/US2020/053610.

Not claimed but described is a set of carrier nucleic acid molecules, comprising: (i) at least one subset of unmethylated carrier nucleic acid molecules; and/or (ii) at least one subset of methylated carrier nucleic acid molecules, wherein at least one end of the carrier nucleic acid molecules is modified to prevent ligation, wherein the unmethylated carrier nucleic acid molecule does not comprise methylated nucleotides and the methylated carrier nucleic acid molecule comprises one or more methylated nucleotides.

Also described is a population of nucleic acids, comprising: (i) a set of carrier nucleic acid molecules, comprising: (a) at least one subset of unmethylated carrier nucleic acid molecules; and/or (b) at least one subset of methylated carrier nucleic acid molecules, wherein at least one end of the carrier nucleic acid molecules is modified to prevent ligation, wherein the unmethylated carrier nucleic acid molecule does not comprise methylated nucleotides and the methylated carrier nucleic acid molecule comprises one or more methylated nucleotides; and (ii) a polynucleotide sample obtained from a subject.

Also described but not claimed is a system comprising a controller comprising or capable of accessing, computer readable media comprising non-transitory computer-executable instructions which, when executed by at least one electronic processor perform a method comprising: (i) adding a set of carrier nucleic acid molecules to polynucleotide sample to generate a first sample; wherein the set of carrier nucleic acid molecules comprises: (a) at least one subset of unmethylated carrier nucleic acid molecules; and/or (b) at least one subset of methylated carrier nucleic acid molecules, wherein at least one end of the carrier nucleic acid molecules is modified to prevent ligation, wherein the unmethylated carrier nucleic acid molecule does not comprise methylated nucleotides, and the methylated carrier nucleic acid molecule comprises one or more methylated nucleotides; (ii) partitioning the first sample into at least two partitioned sets using capturing agent that binds selectively to methylated polynucleotides, thereby generating a partitioned sample; (iii) processing at least a portion of the partitioned sample to generate processed sample, wherein the processing comprises at least one of the following: (a) tagging, (b) amplifying and (c) enriching molecules for specific regions of interest; (iv) sequencing at least a portion of the processed sample to generate a set of sequencing reads; and (v) analyzing at least a portion of the set of sequencing reads to detect the presence or absence of tumor

In some embodiments, the carrier nucleic acid molecules are between 25 bp and 325 bp in length. In some embodiments, a first subset and a second subset of the at least one subset of unmethylated carrier nucleic acid molecules comprise same nucleotide sequence. In some embodiments, a first subset and a second subset of the at least one subset of unmethylated carrier nucleic acid molecules comprise different nucleotide sequence.

In some embodiments, the first subset and the second subset of the at least one subset of unmethylated carrier nucleic acid molecules comprise one or more CpG dinucleotides in the nucleotide sequence. In some embodiments, position of the one or more CpG dinucleotides in the first subset is different from position of the one or more CpG dinucleotides in the second subset of the at least one subset of unmethylated carrier nucleic acid molecules. In some embodiments, number of the CpG dinucleotides in the first subset is different from number of CpG dinucleotides in the second subset of the at least one subset of unmethylated carrier nucleic acid molecules. In some embodiments, sequence of nucleotides adjacent to the one or more CpG dinucleotides in the first subset is different from sequence of nucleotides adjacent to the one or more CpG dinucleotides in the second subset of the at least one subset of unmethylated carrier nucleic acid molecules. In some embodiments, the sequence of nucleotides adjacent to the one or more methylated nucleotides in the first subset and/or the second subset can be the sequence of 1 nucleotide, 2 nucleotides, 3 nucleotides, 4 nucleotides or 5 nucleotides adjacent to the one or more methylated nucleotides. In some embodiments, a first subset and a second subset of the at least one subset of unmethylated carrier nucleic acid molecules are of different lengths.

In some embodiments, the one or more methylated nucleotides is selected from the group consisting of: (i) 5-methylcytosine, (ii) 6-methyladenine, (iii) hydroxymethyl cytosine, (iv) methyl uracil, and (v) any other methylated nucleotide. In some embodiments, number of methylated nucleotides is 1, 2, 3, 4, 5, 6, 7, 8, 9, 10, 12, 14, 15, 16, 17, 18, 19 or at least 20.

In some embodiments, a first subset and a second subset of the at least one subset of methylated carrier nucleic acid molecules comprise same nucleotide sequence. In some embodiments, a first subset and a second subset of the at least one subset of methylated carrier nucleic acid molecules comprise different nucleotide sequence.

In some embodiments, the first subset and the second subset of the at least one subset of methylated carrier nucleic acid molecules comprise one or more CpG dinucleotides in the nucleotide sequence. In some embodiments, the one or more CpG dinucleotides comprises one or more methylated cytosines.

In some embodiments, position of the one or more methylated nucleotides in the first subset is different from position of the one or more methylated nucleotides in the second subset of the at least one subset of methylated carrier nucleic acid molecules. In some embodiments, number of the methylated nucleotides in the first subset is different from number of methylated nucleotides in the second subset of the at least one subset of methylated carrier nucleic acid molecules. In some embodiments, sequence of nucleotides adjacent to the one or more methylated nucleotides in the first subset is different from sequence of nucleotides adjacent to the one or more methylated nucleotides in the second subset of the at least one subset of methylated carrier nucleic acid molecules. In some embodiments, the sequence of nucleotides adjacent to the one or more methylated nucleotides in the first subset and/or the second subset can be the sequence of 1 nucleotide, 2 nucleotides, 3 nucleotides, 4 nucleotides or 5 nucleotides adjacent to the one or more methylated nucleotides.

In some embodiments, a first subset and a second subset of the at least one subset of methylated carrier nucleic acid molecules are of different lengths.

In some embodiments, amount of the at least one subset of methylated carrier nucleic acid molecules to the at least one subset of unmethylated carrier nucleic acid molecules is about 0:1, 0.1:99.9, 0.5:99.5, 0.75:99.25, 1:99, 1:95, 1:90, 1:80, 1:75, 1:70, 1:60, 1:50, 1:40, 1:30, 1:25, 1:20, 1:10, 1:5, 1:2, 1:1.15, 1:1, 1.15:1, 2:1, 5:1, 10:1, 20:1, 25:1, 30:1, 40:1, 50:1, 60:1, 70:1, 75:1, 80:1, 90:1, 95:1, 99:1,99.25:0.75, 99.5:0.5, 99.9:0.1 or 1:0 ratio. In some embodiments, amount of the at least one subset of methylated carrier nucleic acid molecules to the at least one subset of unmethylated carrier nucleic acid molecules is about 1:1 ratio. In some embodiments, amount of the at least one subset of methylated carrier nucleic acid molecules to the at least one subset of unmethylated carrier nucleic acid molecules is about 2:1 ratio. In some embodiments, amount of the at least one subset of methylated carrier nucleic acid molecules to the at least one subset of unmethylated carrier nucleic acid molecules is about 1:2 ratio. In some embodiments, amount of the polynucleotide sample to the set of carrier nucleic acid molecules is about 1: 0.1; 1:0.2, 1:0.3, 1:4, 1:0.5, 1:6, 1:7, 1:8, 1:0.9, 1:1, 1:1, 1:2, 1:3, 1:4, 1:5, 1:6, 1:7, 1:8, 1:9, 1:10 ratio, 1:20, 1:30, 1:40, 1:50, 1:60, 1:70, 1:80, 1:90, 1:100, 1:200, 1:300; 1:400, 1:500, 1:600, 1:700, 1:800, 1:900, 1:1000, 1:5000, 1:10,000, 1:100,000, 1:500,000, 1:106, 1:107, 1:108 or 1:109. In some embodiments, the amount is in terms of mass. In some embodiments, the amount is in terms of molarity. In some embodiments, the polynucleotide sample is up to 1 µg. In some embodiments, the polynucleotide sample is up to 200 ng. In some embodiments, the polynucleotide sample is up to 150 ng. In some embodiments, the polynucleotide sample is up to 100 ng.

In some embodiments, the set of carrier nucleic acid molecules is added in a sufficient amount such that total amount of the polynucleotide sample and the set of carrier nucleic acid molecules is about 175 ng, 200 ng, 225 ng, 250 ng, 275 ng, 300 ng, 350 ng, 400 ng, 450 ng, 500 ng, 600 ng, 700 ng, 750 ng, 800 ng, 900 ng, 1 µg, 1.1 µg, 1.25 µg or 1.5 µg.

In some embodiments, sequence of the carrier nucleic acid molecule is selected from the group consisting of: (i) a sequence from a viral genome, (ii) a sequence from a bacterial genome, (iii) a sequence from a lambda genome, and (iv) a sequence from a non-human genome. In some embodiments, the carrier nucleic acid molecule is a synthetic DNA. In some embodiments, the carrier nucleic acid molecules comprise uracil nucleosides. In some embodiments, the method further comprises, adding uracil deglycosylase and DNA glycosylase-lyase prior to the amplification *(*Kropachev KT et al., Biochemistry (2006); 45(39):12039-12049*).* In some embodiments, the carrier DNA molecules can be generated by PCR. In some embodiments, the carrier nucleic acid molecules generated by PCR can be further modified either by treatment with a methyl transferase to incorporate the methyl group to one or more nucleotides in the carrier nucleic acid molecules. In some embodiments, the carrier nucleic acid molecules can be end labelled with a polymerase to incorporate a modified nucleoside so as to prevent the ligation of the carrier nucleic acid molecules to adapters. In some embodiments, the carrier nucleic acid molecules comprise non-naturally occurring nucleoside derivatives. In some embodiments, the carrier nucleic acid molecules can be labeled with biotin or fluorophore.

In some embodiments, the at least one end of the carrier nucleic acid molecules comprises C3 (propyl group) spacers. The C3 spacers help to block the ligation. In some embodiments, the at least one end of the carrier nucleic acid molecules comprises dideoxy nucleotides. In some embodiments, the at least one end of the carrier nucleic acid molecules comprises any chemical modification that prevents the hydroxyl group from acting as a nucleophile. In some embodiments, 5' end of the carrier nucleic acid molecules comprises at least one of the following modifications: (i) inverted (5'- 5') - dideoxy thymine, dideoxy cytosine, dideoxy guanine or dideoxy adenine; (ii) propyl group, or (iii) other organic functional groups, for example, but not limited to, benzyl, ethyl or methyl. In some embodiments, 3' end of the carrier nucleic acid molecules comprises at least one of the following modifications: (i) any dideoxy base such as dideoxy thymine, dideoxy cytosine, dideoxy guanine or dideoxy adenine, that can be added enzymatically or during synthesis; (ii) propyl group, or (iii) other organic functional groups, for example, but not limited to, benzyl, ethyl or methyl.

In some embodiments, the polynucleotide sample is obtained from tissue, blood, plasma, serum, urine, saliva, stool, cerebral spinal fluid, buccal swab or pleural tap. In some embodiments, the polynucleotide sample is obtained from tissue. In some embodiments, the polynucleotide sample obtained from the tissue is fragmented by enzymatic or mechanical means. In some embodiments, the polynucleotide sample is obtained from blood. In some embodiments, the polynucleotide sample from the blood is a cell-free DNA sample. In some embodiments, the polynucleotide sample is a cell-free DNA sample. In some embodiments, the carrier nucleic acid molecules are double stranded molecules. In some embodiments, the methods disclosed herein does not have a denaturing step prior to the ligation of adapters - i.e., the first sample is not subjected to a denaturing step (in other words, the double stranded carrier nucleic acid molecules are subjected to ligation).

In some embodiments, the results of the systems and/or methods disclosed herein are used as an input to generate a report. The report may be in a paper or electronic format. For example, information on, and/or information derived from, the partitioning of nucleic acid molecules, as determined by the methods or systems disclosed herein, can be displayed in such a report. The methods or systems disclosed herein may further comprise a step of communicating the report to a third party, such as the subject from whom the sample derived or a health care practitioner.

The various steps of the methods disclosed herein, or the steps carried out by the systems disclosed herein, may be carried out at the same time or different times, and/or in the same geographical location or different geographical locations, e.g. countries. The various steps of the methods disclosed herein can be performed by the same person or different people.

Described but not claimed are kits for performing the subject methods. The kit comprises: (a) a set of carrier nucleic acid molecules, wherein the set of carrier nucleic acid molecules comprises: (i) at least one subset of unmethylated carrier nucleic acid molecules; and/or (ii) at least one subset of methylated carrier nucleic acid molecules, wherein at least one end of the carrier nucleic acid molecules is modified to prevent ligation, wherein the unmethylated carrier nucleic acid molecule does not comprise methylated nucleotides and the methylated carrier nucleic acid molecule comprises one or more methylated nucleotides; and (b) a capturing agent that binds selectively to methylated polynucleotides.

### BRIEF DESCRIPTION OF THE DRAWINGS

The accompanying drawings, which are incorporated in and constitute a part of this specification, illustrate certain embodiments, and together with the written description, serve to explain certain principles of the methods, computer readable media, and systems disclosed herein. The description provided herein is better understood when read in conjunction with the accompanying drawings which are included by way of example and not by way of limitation. It will be understood that like reference numerals identify like components throughout the drawings, unless the context indicates otherwise. It will also be understood that some or all of the figures may be schematic representations for purposes of illustration and do not necessarily depict the actual relative sizes or locations of the elements shown.
**FIG. 1** is a flow chart representation of a method for detecting the presence or absence of tumor in a subject according to an embodiment of the disclosure.
**FIG. 2** is a schematic representation of a carrier nucleic acid molecule suitable for use with some embodiments of the disclosure.
**FIG. 3** is a schematic representation of the set of carrier nucleic acid molecules suitable for use with some embodiments of the disclosure.
**FIG. 4** is a schematic representation of the set of carrier nucleic acid molecules suitable for use with some embodiments of the disclosure.
**FIG. 5** is a schematic representation of the set of carrier nucleic acid molecules suitable for use with some embodiments of the disclosure.
**FIG. 6** is a schematic representation of the set of carrier nucleic acid molecules suitable for use with some embodiments of the disclosure.
**FIG. 7** is a schematic diagram of an example of a system suitable for use with some embodiments of the disclosure.
**FIG. 8A** and **FIG. 8B** are graphical representations of cell-free DNA molecules from the samples in the presence and absence of carrier DNA molecules in the hyper partitioned set.

### DEFINITIONS

In order for the present disclosure to be more readily understood, certain terms are first defined below. Additional definitions for the following terms and other terms may be set forth through the specification. If a definition of a term set forth below is inconsistent with a definition in an application or patent that is incorporated by reference, the definition set forth in this application should be used to understand the meaning of the term.

As used in this specification and the appended claims, the singular forms "a", "an", and "the" include plural references unless the context clearly dictates otherwise. Thus, for example, a reference to "a method" includes one or more methods, and/or steps of the type described herein and/or which will become apparent to those persons of ordinary skill in the art upon reading this disclosure and so forth.

It is also to be understood that the terminology used herein is for the purpose of describing particular embodiments only, and is not intended to be limiting. Further, unless defined otherwise, all technical and scientific terms used herein have the same meaning as commonly understood by one of ordinary skill in the art to which this disclosure pertains. In describing and claiming the methods, computer readable media, and systems, the following terminology, and grammatical variants thereof, will be used in accordance with the definitions set forth below.

***About*:** As used herein, "about" or "approximately" as applied to one or more values or elements of interest, refers to a value or element that is similar to a stated reference value or element. In certain embodiments, the term "about" or "approximately" refers to a range of values or elements that falls within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value or element unless otherwise stated or otherwise evident from the context (except where such number would exceed 100% of a possible value or element).

***Adapter*:** As used herein, "adapter" refers to a short nucleic acid (e.g., less than about 500 nucleotides, less than about 100 nucleotides, or less than about 50 nucleotides in length) that is typically at least partially double-stranded and is attached to either or both ends of a given sample nucleic acid molecule. Adapters can include nucleic acid primer binding sites to permit amplification of a nucleic acid molecule flanked by adapters at both ends, and/or a sequencing primer binding site, including primer binding sites for sequencing applications, such as various next-generation sequencing (NGS) applications. Adapters can also include binding sites for capture probes, such as an oligonucleotide attached to a flow cell support or the like. Adapters can also include a nucleic acid tag as described herein. Nucleic acid tags are typically positioned relative to amplification primer and sequencing primer binding sites, such that a nucleic acid tag is included in amplicons and sequence reads of a given nucleic acid molecule. Adapters of the same or different sequences can be linked to the respective ends of a nucleic acid molecule. In some embodiments, adapters of the same sequence is linked to the respective ends of the nucleic acid molecule except that the nucleic acid tag differs. In some embodiments, the adapter is a Y-shaped adapter in which one end is blunt ended or tailed as described herein, for joining to a nucleic acid molecule, which is also blunt ended or tailed with one or more complementary nucleotides. In still other example embodiments, an adapter is a bell-shaped adapter that includes a blunt or tailed end for joining to a nucleic acid molecule to be analyzed. Other examples of adapters include T-tailed and C-tailed adapters.

***Amplify*:** As used herein, "amplify" or "amplification" in the context of nucleic acids refers to the production of multiple copies of a polynucleotide, or a portion of the polynucleotide, typically starting from a small amount of the polynucleotide (e.g., a single polynucleotide molecule), where the amplification products or amplicons are generally detectable. Amplification of polynucleotides encompasses a variety of chemical and enzymatic processes. Amplification includes but is not limited to polymerase chain reaction (PCR).

***Barcode:*** As used herein, "barcode" or "molecular barcode" in the context of nucleic acids refers to a nucleic acid molecule comprising a sequence that can serve as a molecular identifier. Barcode or molecular barcode is a type of nucleic acid tag. For example, individual "barcode" sequences are typically added to the DNA fragment during next-generation sequencing (NGS) library preparation so that the sequencing read can be identified and sorted before the final data analysis.

***Cancer Type:*** As used herein, "cancer type" refers to a type or subtype of cancer defined, e.g., by histopathology. Cancer type can be defined by any conventional criterion, such as on the basis of occurrence in a given tissue (e.g., blood cancers, central nervous system (CNS), brain cancers, lung cancers (small cell and non-small cell), skin cancers, nose cancers, throat cancers, liver cancers, bone cancers, lymphomas, pancreatic cancers, bowel cancers, rectal cancers, thyroid cancers, bladder cancers, kidney cancers, mouth cancers, stomach cancers, breast cancers, prostate cancers, ovarian cancers, lung cancers, intestinal cancers, soft tissue cancers, neuroendocrine cancers, gastroesophageal cancers, head and neck cancers, gynecological cancers, colorectal cancers, urothelial cancers, solid state cancers, heterogeneous cancers, homogenous cancers), unknown primary origin and the like, and/or of the same cell lineage (e.g., carcinoma, sarcoma, lymphoma, cholangiocarcinoma, leukemia, mesothelioma, melanoma, or glioblastoma) and/or cancers exhibiting cancer markers, such as, but not limited to, Her2, CA15-3, CA19-9, CA-125, CEA, AFP, PSA, HCG, hormone receptor and NMP-22. Cancers can also be classified by stage (e.g., stage 1, 2, 3, or 4) and whether of primary or secondary origin.

***Carrier nucleic acid molecules:*** As used herein, "carrier nucleic acid molecules" refers to a set of nucleic acid molecules that can be added to polynucleotide sample obtained from a subject to improve the detection of the presence or absence of tumor in the subject. In some embodiments, the carrier nucleic acid molecules can be single stranded or double stranded. In some embodiments, the carrier nucleic acid molecules can be DNA or RNA. In some embodiments, the carrier nucleic acid molecules can be synthetic oligonucleotides. In some embodiments, carrier nucleic acid molecules can be generated by PCR via amplifying one or specific regions of interest from a genome. In some embodiments, the carrier nucleic acid molecules generated by PCR can be further modified either by treatment with a methyl transferase to incorporate the methyl group to one or more nucleotides in the carrier nucleic acid molecules. In some embodiments, the carrier nucleic acid molecules can be end labelled with a polymerase to incorporate a modified nucleoside so as to prevent the ligation of the carrier nucleic acid molecules to adapters. In some embodiments, the carrier nucleic acid molecules can have a non-naturally occurring nucleic acid sequence. In some embodiments, the carrier nucleic acid molecules can have a naturally occurring nucleic acid sequence. In some embodiments, carrier nucleic acid molecules can have a nucleic acid sequence corresponding to a non-human genome. As non-limiting examples, these carrier nucleic acid molecules may either have (i) a sequence corresponding to regions of lambda phage DNA or human genome, (ii) a sequence corresponding to regions of viral genome, (iii) a sequence corresponding to regions of bacterial genome, (iv) a non-naturally occurring sequence, and/or (v) a combination of any of the above. In some embodiments, the carrier nucleic acid molecules comprise non-naturally occurring nucleoside derivatives. In some embodiments, the carrier DNA molecules can be generated by PCR. In some embodiments, the carrier nucleic acid molecules generated by PCR can be further modified either by treatment with a methyl transferase to incorporate the methyl group to one or more nucleotides in the carrier nucleic acid molecules. In some embodiments, the carrier nucleic acid molecules can be end labelled with a polymerase to incorporate a modified nucleoside so as to prevent the ligation of the carrier nucleic acid molecules to adapters. In some embodiments, the carrier nucleic acid molecules comprise non-naturally occurring nucleoside derivatives. In some embodiments, the carrier nucleic acid molecules can be labeled with biotin or fluorophore.

***Cell-Free Nucleic Acid*:** As used herein, "cell-free nucleic acid" refers to nucleic acids not contained within or otherwise bound to a cell or, in some embodiments, nucleic acids remaining in a sample following the removal of intact cells. Cell-free nucleic acids can include, for example, all non-encapsulated nucleic acids sourced from a bodily fluid (e.g., blood, plasma, serum, urine, cerebrospinal fluid (CSF), etc.) from a subject. Cell-free nucleic acids include DNA (cfDNA), RNA (cfRNA), and hybrids thereof, including genomic DNA, mitochondrial DNA, circulating DNA, siRNA, miRNA, circulating RNA (cRNA), tRNA, rRNA, small nucleolar RNA (snoRNA), Piwi-interacting RNA (piRNA), long non-coding RNA (long ncRNA), and/or fragments of any of these. Cell-free nucleic acids can be double-stranded, single-stranded, or a hybrid thereof. A cell-free nucleic acid can be released into bodily fluid through secretion or cell death processes, e.g., cellular necrosis, apoptosis, or the like. Some cell-free nucleic acids are released into bodily fluid from cancer cells, e.g., circulating tumor DNA (ctDNA). Others are released from healthy cells. CtDNA can be non-encapsulated tumor-derived fragmented DNA. A cell-free nucleic acid can have one or more epigenetic modifications, for example, a cell-free nucleic acid can be acetylated, 5-methylated, and/or hydroxy methylated.

***Cellular Nucleic Acids*:** As used herein, "cellular nucleic acids" means nucleic acids that are disposed within one or more cells from which the nucleic acids have originated, at least at the point a sample is taken or collected from a subject, even if those nucleic acids are subsequently removed (e.g., via cell lysis) as part of a given analytical process.

***Coverage:*** As used herein, the terms "coverage", "total molecule count" or "total allele count" are used interchangeably. They refer to the total number of DNA molecules at a particular genomic position in a given sample.

***Deoxyribonucleic Acid*** or ***Ribonucleic Acid*:** As used herein, "deoxyribonucleic acid" or "DNA" refers to a natural or modified nucleotide which has a hydrogen group at the 2'-position of the sugar moiety. DNA typically includes a chain of nucleotides comprising four types of nucleotide bases; adenine (A), thymine (T), cytosine (C), and guanine (G). As used herein, "ribonucleic acid" or "RNA" refers to a natural or modified nucleotide which has a hydroxyl group at the 2'-position of the sugar moiety. RNA typically includes a chain of nucleotides comprising four types of nucleotide bases; A, uracil (U), G, and C. As used herein, the term "nucleotide" refers to a natural nucleotide or a modified nucleotide. Certain pairs of nucleotides specifically bind to one another in a complementary fashion (called complementary base pairing). In DNA, adenine (A) pairs with thymine (T) and cytosine (C) pairs with guanine (G). In RNA, adenine (A) pairs with uracil (U) and cytosine (C) pairs with guanine (G). When a first nucleic acid strand binds to a second nucleic acid strand made up of nucleotides that are complementary to those in the first strand, the two strands bind to form a double strand. As used herein, "nucleic acid sequencing data," "nucleic acid sequencing information," "sequence information," "nucleic acid sequence," "nucleotide sequence", "genomic sequence," "genetic sequence," or "fragment sequence," or "nucleic acid sequencing read" denotes any information or data that is indicative of the order and/or identity of the nucleotide bases (e.g., adenine, guanine, cytosine, and thymine or uracil) in a molecule (e.g., a whole genome, whole transcriptome, exome, oligonucleotide, polynucleotide, or fragment) of a nucleic acid such as DNA or RNA. It should be understood that the present teachings contemplate sequence information obtained using all available varieties of techniques, platforms or technologies, including, but not limited to: capillary electrophoresis, microarrays, ligation-based systems, polymerase-based systems, hybridization-based systems, direct or indirect nucleotide identification systems, pyrosequencing, ion- or pH-based detection systems, and electronic signature-based systems.

***Mutation:*** As used herein, "mutation" refers to a variation from a known reference sequence and includes mutations such as, for example, single nucleotide variants (SNVs), and insertions or deletions (indels). A mutation can be a germline or somatic mutation. In some embodiments, a reference sequence for purposes of comparison is a wildtype genomic sequence of the species of the subject providing a test sample, typically the human genome.

***Mutation Caller:*** As used herein, "mutation caller" means an algorithm (typically, embodied in software or otherwise computer implemented) that is used to identify mutations in test sample data (e.g., sequence information obtained from a subject).

***Neoplasm:*** As used herein, the terms "neoplasm" and "tumor" are used interchangeably. They refer to abnormal growth of cells in a subject. A neoplasm or tumor can be benign, potentially malignant, or malignant. A malignant tumor is a referred to as a cancer or a cancerous tumor.

***Next Generation Sequencing:*** As used herein, "next generation sequencing" or "NGS" refers to sequencing technologies having increased throughput as compared to traditional Sanger- and capillary electrophoresis-based approaches, for example, with the ability to generate hundreds of thousands of relatively small sequence reads at a time. Some examples of next generation sequencing techniques include, but are not limited to, sequencing by synthesis, sequencing by ligation, and sequencing by hybridization. In some embodiments, next generation sequencing includes the use of instruments capable of sequencing single molecules.

***Nucleic Acid Tag:*** As used herein, "nucleic acid tag" refers to a short nucleic acid (e.g., less than about 500 nucleotides, about 100 nucleotides, about 50 nucleotides, or about 10 nucleotides in length), used to distinguish nucleic acids from different samples (e.g., representing a sample index), or different nucleic acid molecules in the same sample (e.g., representing a molecular barcode), of different types, or which have undergone different processing. The nucleic acid tag comprises a predetermined, fixed, non-random, random or semi-random oligonucleotide sequence. Such nucleic acid tags may be used to label different nucleic acid molecules or different nucleic acid samples or sub-samples. Nucleic acid tags can be single-stranded, double-stranded, or at least partially double-stranded. Nucleic acid tags optionally have the same length or varied lengths. Nucleic acid tags can also include double-stranded molecules having one or more blunt-ends, include 5' or 3' single-stranded regions (e.g., an overhang), and/or include one or more other single-stranded regions at other locations within a given molecule. Nucleic acid tags can be attached to one end or to both ends of the other nucleic acids (e.g., sample nucleic acids to be amplified and/or sequenced). Nucleic acid tags can be decoded to reveal information such as the sample of origin, form, or processing of a given nucleic acid. For example, nucleic acid tags can also be used to enable pooling and/or parallel processing of multiple samples comprising nucleic acids bearing different molecular barcodes and/or sample indexes in which the nucleic acids are subsequently being deconvolved by detecting (e.g., reading) the nucleic acid tags. Nucleic acid tags can also be referred to as identifiers (e.g. molecular identifier, sample identifier). Additionally, or alternatively, nucleic acid tags can be used as molecular identifiers (e.g., to distinguish between different molecules or amplicons of different parent molecules in the same sample or sub-sample). This includes, for example, uniquely tagging different nucleic acid molecules in a given sample, or non-uniquely tagging such molecules. In the case of non-unique tagging applications, a limited number of tags (i.e., molecular barcodes) may be used to tag each nucleic acid molecule such that different molecules can be distinguished based on their endogenous sequence information (for example, start and/or stop positions where they map to a selected reference genome, a sub-sequence of one or both ends of a sequence, and/or length of a sequence) in combination with at least one molecular barcode. Typically, a sufficient number of different molecular barcodes are used such that there is a low probability (e.g., less than about a 10%, less than about a 5%, less than about a 1%, or less than about a 0.1% chance) that any two molecules may have the same endogenous sequence information (e.g., start and/or stop positions, subsequences of one or both ends of a sequence, and/or lengths) and also have the same molecular barcode.

***Partitioning:*** As used herein, the "partitioning" and "epigenetic partitioning" are used interchangeably. It refers to separating or fractionating the nucleic acid molecules based on a characteristic (e.g. the level/degree of epigenetic modification) of the nucleic acid molecules. The partitioning can be physical partitioning of molecules. Partitioning can involve separating the nucleic acid molecules into groups or sets based on the level of epigenetic modification (e.g. methylation). For example, the nucleic acid molecules can be partitioned based on the level of methylation of the nucleic acid molecules. In some embodiments, the methods and systems used for partitioning may be found in PCT Patent Application No. PCT/US2017/068329 Also, the methods, systems and compositions may be found in PCT Patent Application Nos. PCT/US2019/059217 and PCT/US2020/016120.

***Partitioned set:*** As used herein, "partitioned set" refers to a set of nucleic acid molecules partitioned into a set/group based on the differential binding affinity of the nucleic acid molecules to a binding agent. The binding agent binds preferentially to the nucleic acid molecules comprising nucleotides with epigenetic modification. For example, if the epigenetic modification is methylation, the binding agent can be a methyl binding domain (MBD) protein. In some embodiments, a partitioned set can comprise nucleic acid molecules belonging to a particular level/degree of epigenetic modification. For example, the nucleic acid molecules can be partitioned into three sets: one set for highly methylated nucleic acid molecules (or hypermethylated nucleic acid molecules), which can be referred as hypermethylated partitioned set or hyper partitioned set, another set for low methylated nucleic acid molecules (or hypomethylated nucleic acid molecules), which can be referred as hypomethylated partitioned set or hypo partitioned set and a third set for intermediately methylated nucleic acid molecules, which can be referred as intermediately methylated partitioned set or intermediate partitioned set. In another example, the nucleic acid molecules can be partitioned based on the number of methylated nucleotides - one partitioned set can have nucleic acid molecules with nine methylated nucleotides and another partitioned set can have unmethylated nucleic acid molecules (i.e., zero methylated nucleotides).

***Polynucleotide:*** As used herein, "polynucleotide", "nucleic acid", "nucleic acid molecule", or "oligonucleotide" refers to a linear polymer of nucleosides (including deoxyribonucleosides, ribonucleosides, or analogs thereof) joined by inter-nucleosidic linkages. Typically, a polynucleotide comprises at least three nucleosides. Oligonucleotides often range in size from a few monomeric units, e.g., 3-4, to hundreds of monomeric units. Whenever a polynucleotide is represented by a sequence of letters, such as "ATGCCTG", it will be understood that the nucleotides are in 5' → 3' order from left to right and that in the case of DNA, "A" denotes deoxyadenosine, "C" denotes deoxycytidine, "G" denotes deoxyguanosine, and "T" denotes deoxythymidine, unless otherwise noted. The letters A, C, G, and T may be used to refer to the bases themselves, to nucleosides, or to nucleotides comprising the bases, as is standard in the art.

***Reference Sequence:*** As used herein, "reference sequence" refers to a known sequence used for purposes of comparison with experimentally determined sequences. For example, a known sequence can be an entire genome, a chromosome, or any segment thereof. A reference typically includes at least about 20, at least about 50, at least about 100, at least about 200, at least about 250, at least about 300, at least about 350, at least about 400, at least about 450, at least about 500, at least about 1000, or more than 1000 nucleotides. In some embodiments, the reference sequence can be human genome. A reference sequence can align with a single contiguous sequence of a genome or chromosome or can include non-contiguous segments that align with different regions of a genome or chromosome. Examples of reference sequences include, for example, human genomes, such as, hG19 and hG38.

***Sample:*** As used herein, "sample" means anything capable of being analyzed by the methods and/or systems disclosed herein.

***Sequencing:*** As used herein, "sequencing" refers to any of a number of technologies used to determine the sequence (e.g., the identity and/or order of monomer units) of a biomolecule, e.g., a nucleic acid such as DNA or RNA. Examples of sequencing methods include, but are not limited to, targeted sequencing, single molecule real-time sequencing, exon or exome sequencing, intron sequencing, electron microscopy-based sequencing, panel sequencing, transistor-mediated sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, co-amplification at lower denaturation temperature-PCR (COLD-PCR), multiplex PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD^{™} sequencing, MS-PET sequencing, and a combination thereof. In some embodiments, sequencing can be performed by a gene analyzer such as, for example, gene analyzers commercially available from Illumina, Inc., Pacific Biosciences, Inc., or Applied Biosystems/Thermo Fisher Scientific, among many others.

***Sequence Information:*** As used herein, "sequence information" in the context of a nucleic acid molecule means the order and/or identity of monomer units (e.g., nucleotides, etc.) in that molecule and can also include start and stop genomic coordinates of the nucleic acid molecule mapping to the reference sequence.

***Somatic Mutation:*** As used herein, the terms "somatic mutation" or "somatic variation" are used interchangeably. They refer to a mutation in the genome that occurs after conception. Somatic mutations can occur in any cell of the body except germ cells and accordingly, are not passed on to progeny.

***Subject:*** As used herein, "subject" refers to an animal, such as a mammalian species (e.g., human) or avian (e.g., bird) species, or other organism, such as a plant. More specifically, a subject can be a vertebrate, e.g., a mammal such as a mouse, a primate, a simian or a human. Animals include farm animals (e.g., production cattle, dairy cattle, poultry, horses, pigs, and the like), sport animals, and companion animals (e.g., pets or support animals). A subject can be a healthy individual, an individual that has or is suspected of having a disease or a predisposition to the disease, or an individual in need of therapy or suspected of needing therapy. The terms "individual" or "patient" are intended to be interchangeable with "subject."

For example, a subject can be an individual who has been diagnosed with having a cancer, is going to receive a cancer therapy, and/or has received at least one cancer therapy. The subject can be in remission of a cancer. As another example, the subject can be an individual who is diagnosed of having an autoimmune disease. As another example, the subject can be a female individual who is pregnant or who is planning on getting pregnant, who may have been diagnosed of or suspected of having a disease, e.g., a cancer, an auto-immune disease.

### DETAILED DESCRIPTION

### I. Overview

Genomic/epigenetic partitioning-based methods can allow for multi-analyte, simultaneous signal detection in one assay. However, detected signals of the partitioning-based analyte may have poor resolution and are subject to variable assay conditions that alter signal sensitivity and specificity. It is desirable to increase the sensitivity of liquid biopsy assays while reducing the loss of cell-free nucleic acid (original material) or data in the process. It is also desirable to provide for the ability to compare results across different experiments by controlling for assay variability by using one or more controls as described herein.

The present disclosure provides methods, compositions and systems for analyzing polynucleotides in partitioning assays. The invention comprises using a set of carrier nucleic acid molecules. In some embodiments, the use of carrier nucleic acid molecules can increase the specificity of partitioning of methylated nucleic acid molecules by preventing non-specific binding of unmethylated nucleic acid molecules to the capturing agent that selectively binds to methylated nucleic acid molecules.

Nucleic acids molecules, such as cell-free polynucleotides, can differ based on epigenetic characteristics such as methylation. Nucleic acids can possess different nucleotide sequences, e.g., specific genes or genetic loci. Characteristics can differ in terms of degree. For example, DNA molecules can differ in their extent of epigenetic modification. Extent of modification can refer to a number of modifying events to which a molecule has been subject, such as number of methylation groups (extent of methylation) or other epigenetic changes. For example, DNA may be hypomethylated or hypermethylated.

A characteristic of nucleic acid molecules may be a modification, which may include various chemical modifications (i.e. epigenetic modifications). Non-limiting examples of chemical modification may include, but are not limited to, covalent DNA modifications, including DNA methylation. In some embodiments, DNA methylation comprises addition of a methyl group to a cytosine at a CpG site (cytosine-phosphate-guanine site (i.e., a cytosine followed by a guanine in a 5' → 3' direction of the nucleic acid sequence)). In some embodiments, DNA methylation comprises addition of a methyl group to adenine, such as in N⁶-methyladenine. In some embodiments, DNA methylation is 5-methylation (modification of the carbon at the fifth position in the six-membered ring of cytosine). In some embodiments, 5-methylation comprises addition of a methyl group to the 5C position of the cytosine to create 5-methylcytosine (m5c). In some embodiments, methylation comprises a derivative of m5c. Derivatives of m5c include, but are not limited to, 5-hydroxymethylcytosine (5-hmC), 5-formylcytosine (5-fC), and 5-caryboxylcytosine (5-caC). In some embodiments, DNA methylation is 3C methylation (modification of the carbon at the third position in the six-membered ring of cytosine). In some embodiments, 3C methylation comprises addition of a methyl group to the 3C position of the cytosine to generate 3-methylcytosine (3mC). Methylation can also occur at non CpG sites, for example, methylation can occur at a CpA, CpT, or CpC site. DNA methylation can change the activity of methylated DNA region. For example, when DNA in a promoter region is methylated, transcription of the gene may be repressed. DNA methylation is critical for normal development and abnormality in methylation may disrupt epigenetic regulation. The disruption, e.g., repression, in epigenetic regulation may cause diseases, such as cancer. Promoter methylation in DNA may be indicative of cancer.

A CpG dyad is the dinucleotide CpG (cytosine-phosphate-guanine, i.e. a cytosine followed by a guanine in a 5' → 3' direction of the nucleic acid sequence) on the sense strand and its complementary CpG on the antisense strand of a double-stranded DNA molecule. CpG dyads can be either fully methylated or hemi-methylated (methylated on one strand only).

The CpG dinucleotide is underrepresented in the normal human genome, with the majority of CpG dinucleotide sequences being transcriptionally inert (e.g. DNA heterochromatic regions in pericentromeric parts of the chromosome and in repeat elements) and methylated. However, many CpG islands are protected from such methylation especially around transcription start sites (TSS).

Cancer can be indicated by epigenetic variations, such as methylation. Examples of methylation changes in cancer include local gains of DNA methylation in the CpG islands at the TSS of genes involved in normal growth control, DNA repair, cell cycle regulation, and/or cell differentiation. This hypermethylation can be associated with an aberrant loss of transcriptional capacity of involved genes and occurs at least as frequently as point mutations and deletions as a cause of altered gene expression. DNA methylation profiling can be used to detect regions with different extents of methylation ("differentially methylated regions" or "DMRs") of the genome that are altered during development or that are perturbed by disease, for example, cancer or any cancer-associated disease.

Methylation profiling can involve determining methylation patterns across different regions of the genome. For example, after partitioning molecules based on extent of methylation (e.g., relative number of methylated nucleotides per molecule) and sequencing, the sequences of molecules in the different partitions can be mapped to a reference genome. This can show regions of the genome that, compared with other regions, are more highly methylated or are less highly methylated. In this way, genomic regions, in contrast to individual molecules, may differ in their extent of methylation. In addition to methylation, other epigenetic modifications may be similarly profiled.

Nucleic acid molecules in a sample may be fractionated or partitioned based on one or more characteristics. Partitioning nucleic acid molecules in a sample can increase a rare signal. For example, a genetic variation present in hypermethylated DNA but less (or not) present in hypomethylated DNA can be more easily detected by partitioning a sample into hypermethylated and hypomethylated nucleic acid molecules. By analyzing multiple fractions of a sample, a multi-dimensional analysis of a single molecule can be performed and hence, greater sensitivity can be achieved. Partitioning may include physically partitioning nucleic acid molecules into subsets or groups based on the presence or absence of a genomic characteristic. Fractionation may include physically partitioning nucleic acid molecules into partition groups based on the degree to which a genomic characteristic, such as an epigenetic modification, is present. A sample may be fractionated or partitioned into one or more groups partitions based on a characteristic that is indicative of differential gene expression or a disease state. A sample may be fractionated based on a characteristic, or combination thereof that provides a difference in signal between a normal and diseased state during analysis of nucleic acids, e.g., cell free DNA ("cfDNA"), non-cfDNA, tumor DNA, circulating tumor DNA ("ctDNA") and cell free nucleic acids ("cfNA").

The present disclosure provides methods, compositions and systems for analyzing polynucleotides using partitioning assay to detect the presence or absence of tumor. The methods may include adding carrier nucleic acid molecules to the polynucleotide sample obtained from the subject. In some embodiments, the use of carrier nucleic acid molecules helps in improving the partitioning of the polynucleotides using methyl binding proteins. The methyl binding domain proteins have low affinity for unmethylated molecules. Thus, when incubated with DNA obtained a subject, molecules not containing methylated cytosines can inadvertently be captured. This reduces the ability to separate efficiently the methylated molecules from unmethylated molecules. Hence, by including carrier nucleic acid molecules in the assay, the carrier nucleic acid molecules will bind to the methyl binding proteins and the unmethylated molecules from the polynucleotide sample will not be able to bind to the methyl binding proteins. Thus improving the specificity of the partitioning of methylated molecules. In some embodiments, the carrier nucleic acid molecules improve the library preparation of the molecules for next generation sequencing.

In some embodiments, carrier nucleic acid molecules are added to the polynucleotide sample and partitioned into different partitioned sets based on methylation level of molecules, followed by sequencing (alone or together) and analyzing the nucleic acid molecules in each partition. In some embodiments, the partitions of nucleic acids are enriched for specific target genomic regions. In some embodiments, the partitions of nucleic acid molecules are amplified prior to and/or after enriching. In some embodiments, the enrichment may be performed after the partitioned sets have been differentially tagged with molecular barcodes and recombined into a mixture of the differentially tagged partitioned sets. The methods can be used in various applications, such as prognosis, diagnosis and/or for monitoring of a disease. In some embodiments, the disease is cancer.

Accordingly, in one aspect, the present disclosure provides a method of detecting the presence or absence of tumor in a subject comprising: (i) obtaining a polynucleotide sample from the subject; (ii) adding a set of carrier nucleic acid molecules to the polynucleotide sample to generate a first sample; wherein the set of carrier nucleic acid molecules comprises: (a) at least one subset of unmethylated carrier nucleic acid molecules; and/or (b) at least one subset of methylated carrier nucleic acid molecules, , wherein the unmethylated carrier nucleic acid molecule does not comprise methylated nucleotides and the methylated carrier nucleic acid molecule comprises one or more methylated nucleotides; (iii) partitioning the first sample into at least two partitioned sets using capturing agent that binds selectively to methylated polynucleotides, thereby generating a partitioned sample; (iv) processing at least a portion of the partitioned sample to generate processed sample, wherein the processing comprises at least one of the following: (a) tagging, (b) amplifying and (c) enriching DNA molecules for specific regions of interest; (v) sequencing at least a portion of the processed sample to generate a set of sequencing reads; and (vi) analyzing at least a portion of the set of sequencing reads to detect the presence or absence of tumor.

In another aspect, the present disclosure provides a method of detecting the presence or absence of tumor in a subject comprising: (i) obtaining a polynucleotide sample from the subject; (ii) adding a set of carrier nucleic acid molecules to the polynucleotide sample to generate a first sample; wherein the set of carrier nucleic acid molecules comprises: (a) at least one subset of unmethylated carrier nucleic acid molecules; and/or (b) at least one subset of methylated carrier nucleic acid molecules, wherein at least one end of the carrier nucleic acid molecules is modified to prevent ligation, wherein the unmethylated carrier nucleic acid molecule does not comprise methylated nucleotides and the methylated carrier nucleic acid molecule comprises one or more methylated nucleotides; (iii) partitioning the first sample into at least two partitioned sets using capturing agent that binds selectively to methylated polynucleotides, thereby generating a partitioned sample; (iv) processing at least a portion of the partitioned sample to generate processed sample, wherein the processing comprises at least one of the following: (a) tagging, (b) amplifying and (c) enriching DNA molecules for specific regions of interest; (v) sequencing at least a portion of the processed sample to generate a set of sequencing reads; and (vi) analyzing at least a portion of the set of sequencing reads to detect the presence or absence of tumor.

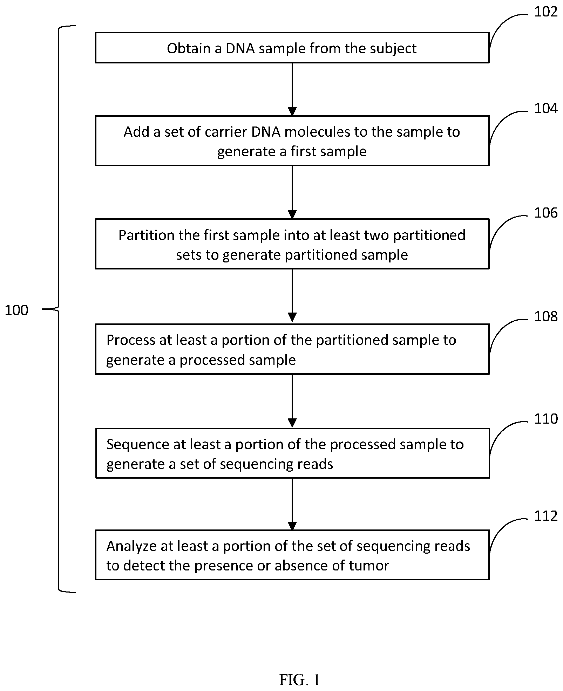

FIG. 1 illustrates an example embodiment of a method 100 for detecting the presence or absence of tumor in a subject. In 102, a polynucleotide sample from the subject is obtained. In some embodiments, the polynucleotide sample is obtained from tissues, blood, plasma, serum, urine, saliva, stool, cerebral spinal fluid, buccal swab or pleural tap of the subject. In some embodiments, the polynucleotide sample is obtained from the tissue. In some embodiments, the polynucleotide sample obtained from the tissue is fragmented either by enzymatic or mechanical means/methods. In some embodiments, fragmentase enzyme can be used to fragment the DNA obtained from the tissue. In some embodiments, the polynucleotide sample is obtained from the blood. In some embodiments, the polynucleotide sample obtained from the blood is a cell-free DNA sample. In some embodiments, the polynucleotide sample is a cell-free DNA sample.

In 104, a set of carrier nucleic acid molecules are added to the polynucleotide sample to generate a first sample. In some embodiments, the set of carrier nucleic acid molecules comprises at least one subset of unmethylated carrier nucleic acid molecules and/or at least one subset of methylated carrier nucleic acid molecules, wherein the unmethylated carrier nucleic acid molecule does not comprise methylated nucleotides, and the methylated carrier nucleic acid molecule comprises one or more methylated nucleotides. In some embodiments, at least one end of the carrier nucleic acid molecule is modified to prevent ligation. In some embodiments, at least one end of the carrier nucleic acid molecules comprises C3 (propyl group) spacers. In some embodiments, at least one end of the carrier nucleic acid molecules comprises dideoxy nucleotides. In some embodiments, at least one end of the carrier nucleic acid molecules comprises any chemical modification that prevents the hydroxyl group from acting as a nucleophile. In some embodiments, the 5'end of the carrier nucleic acid molecules comprises at least one of the following modifications: (i) inverted (5'- 5') - dideoxy thymine, dideoxy cytosine, dideoxy guanine or dideoxy adenine; (ii) propyl group, or (iii) other organic functional groups, for example, but not limited to, benzyl, ethyl or methyl. In some embodiments, the 3'end of the carrier nucleic acid molecules comprises at least one of the following modifications: (i) any dideoxy base such as dideoxy thymine, dideoxy cytosine, dideoxy guanine or dideoxy adenine, that can be added enzymatically or during synthesis; (ii) propyl group, or (iii) other organic functional groups, for example, but not limited to, benzyl, ethyl or methyl.

In some embodiments, the one or more methylated nucleotides is at least one of the following: (i) 5-methylcytosine, (ii) 6-methyladenine, (iii) hydroxymethyl cytosine, (iv) methyl uracil, or (v) any other methylated nucleotide. In some embodiments, the methylated carrier nucleic acid molecules can have 1, 2, 3, 4, 5, 6, 7, 8, 9, 10, 12, 14, 15, 16, 17, 18, 19 or at least 20 methylated nucleotides. In some embodiments, the carrier nucleic acid molecules can be between 25 bp and 325 bp in length. In some embodiments, the unmethylated carrier nucleic acid molecules of one subset can have the same sequence as the unmethylated carrier nucleic acid molecules of another subset. In some embodiments, the methylated carrier nucleic acid molecules of one subset can have the same sequence as the methylated carrier nucleic acid molecules of another subset. In some embodiments, the sequence of the unmethylated carrier nucleic acid molecules in one subset is different from the sequence of the unmethylated carrier nucleic acid molecules in the other subset(s). In some embodiments, the sequence of the methylated carrier nucleic acid molecules in one subset is different from the sequence of the methylated carrier nucleic acid molecules in the other subset(s).

In some embodiments, the one or more subsets of unmethylated carrier nucleic acid molecules and/or methylated carrier nucleic acid molecules can have one or more CpG dinucleotides in the nucleotide sequence. In some embodiments, the one or CpG dinucleotides in the methylated carrier nucleic acid molecules can have one or more methylated cytosines.

In some embodiments, position of the one or more CpG dinucleotides in one subset is different from the position of the one or more CpG dinucleotides in the other subset(s) of the unmethylated carrier nucleic acid molecules. In some embodiments, the number of the CpG dinucleotides in one subset is different from the number of CpG dinucleotides in the other subset(s) of the unmethylated carrier nucleic acid molecules. In some embodiments, the sequence of nucleotides adjacent to the one or more CpG dinucleotides in one subset is different from the sequence of nucleotides adjacent to the one or more CpG dinucleotides in the other subset(s) of unmethylated carrier nucleic acid molecules. In some embodiments, the length of the unmethylated carrier nucleic acid molecules in one subset is different from the length of the unmethylated carrier nucleic acid molecules in the other subset(s) of unmethylated carrier nucleic acid molecules.

In some embodiments, position (i.e., relative to the end of the molecule) of the one or more methylated nucleotides in one subset is different from position of the one or more methylated nucleotides in the other subset(s) of the methylated carrier nucleic acid molecules. In some embodiments, the number of the methylated nucleotides in one subset is different from the number of methylated nucleotides in the other subset(s) of the methylated carrier nucleic acid molecules. In some embodiments, the sequence of nucleotides adjacent to the one or more methylated nucleotides in one subset is different from the sequence of nucleotides adjacent to the one or more methylated nucleotides in the other subset(s) of unmethylated carrier nucleic acid molecules. In some embodiments, the length of the methylated carrier nucleic acid molecules in one subset is different from the length of the methylated carrier nucleic acid molecules in the other subset(s) of methylated carrier nucleic acid molecules. In some embodiments, the carrier nucleic acid molecules comprise uracil nucleosides. In these embodiments, the method further comprises adding a uracil deglycosylase and a DNA glycosylase-lyase (e.g. endonuclease VIII) prior to the amplification step.

In some embodiments, the amount of the methylated carrier nucleic acid molecules to the unmethylated carrier nucleic acid molecules is about 0:1, 0.1:99.9, 0.5:99.5, 0.75:99.25, 1:99, 1:95, 1:90, 1:80, 1:75, 1:70, 1:60, 1:50, 1:40, 1:30, 1:25, 1:20, 1:10, 1:5, 1:2, 1:1.15, 1:1, 1.15:1, 2:1, 5:1, 10:1, 20:1, 25:1, 30:1, 40:1, 50:1, 60:1, 70:1, 75:1, 80:1, 90:1, 95:1, 99:1,99.25:0.75, 99.5:0.5, 99.9:0.1 or 1:0 ratio. In some embodiments, the amount of the polynucleotide sample to the set of carrier nucleic acid molecules is about 1: 0.1; 1:0.2, 1:0.3, 1:4, 1:0.5, 1:6, 1:7, 1:8, 1:0.9, 1:1, 1:1, 1:2, 1:3, 1:4, 1:5, 1:6, 1:7, 1:8, 1:9, 1:10 ratio, 1:20, 1:30, 1:40, 1:50, 1:60, 1:70, 1:80, 1:90, 1:100, 1:200, 1:300; 1:400, 1:500, 1:600, 1:700, 1:800, 1:900, 1:1000, 1:5000, 1:10,000, 1:100,000, 1:500,000, 1:10⁶, 1:10⁷, 1:10⁸ or 1:10⁹. In some embodiments, the amount is in terms of mass. In some embodiments, the amount is in terms of molarity. In some embodiments, the polynucleotide sample is up to 1 µg. In some embodiments, the polynucleotide sample is up to 200 ng. In some embodiments, the polynucleotide sample is up to 150 ng. In some embodiments, the polynucleotide sample is up to 100 ng. In some embodiments, the set of carrier nucleic acid molecules is added in a sufficient amount such that total amount of the polynucleotide sample and the set of carrier nucleic acid molecules is about 175 ng, 200 ng, 225 ng, 250 ng, 275 ng, 300 ng, 350 ng, 400 ng, 450 ng, 500 ng, 600 ng, 700 ng, 750 ng, 800 ng, 900 ng, 1 µg, 1.1 µg, 1.25 µg or 1.5 µg.

In some embodiments, the sequence of the carrier nucleic acid molecule is selected from one of the following: (i) a sequence from a viral genome, (ii) a sequence from a bacterial genome, (iii) a sequence from a lambda genome, or (iv) a sequence from a non-human genome. In some embodiments, the carrier nucleic acid molecule is a synthetic DNA. In some embodiments, the carrier nucleic acid molecules comprise uracil nucleosides. In some embodiments, the carrier DNA molecules can be generated by PCR. In some embodiments, the carrier nucleic acid molecules generated by PCR can be further modified either by treatment with a methyl transferase to incorporate the methyl group to one or more nucleotides in the carrier nucleic acid molecules. In some embodiments, the carrier nucleic acid molecules can be end labelled with a polymerase to incorporate a modified nucleoside so as to prevent the ligation of the carrier nucleic acid molecules to adapters. In some embodiments, the carrier nucleic acid molecules comprise non-naturally occurring nucleoside derivatives. In some embodiments, the carrier nucleic acid molecules can be labeled with biotin or fluorophore.

In 106, at least a portion of the first sample is partitioned or fractionated into at least two partitioned sets using a capturing agent that binds selectively to methylated polynucleotides, thereby generating a partitioned sample. In some embodiments, partitioning is based on the differential binding affinity of the nucleic acid molecules to a binding agent. Examples of binding agents include, but not limited to methyl binding domain (MBDs) and methyl binding proteins (MBPs). Examples of MBPs contemplated herein include, but are not limited to:
(a) MeCP2 is a protein preferentially binding to 5-methyl-cytosine over unmodified cytosine;
(b) RPL26, PRP8 and the DNA mismatch repair protein MHS6 preferentially bind to 5-hydroxymethyl-cytosine over unmodified cytosine;
(c) FOXK1, FOXK2, FOXP1, FOXP4 AND FOXI3 preferably bind to 5-formyl-cytosine over unmodified cytosine (Iurlaro et al., Genome Biol. 14, R119 (2013)).; and
(d) Antibodies specific to one or more methylated nucleotide bases.

Although for some affinity agents and modifications, binding to the agent may occur in an essentially all or none manner depending on whether a nucleic acid bears a modification (e.g., methylation), the separation may be one of degree. In such embodiments, nucleic acids overrepresented in a modification bind to the agent at a greater extent than nucleic acids underrepresented in the modification. Alternatively, nucleic acids having modifications may bind in an all or nothing manner. But then, various levels of modifications may be sequentially eluted from the binding agent.

For example, in some embodiments, partitioning can be binary or based on degree/level of methylation. For example, all methylated molecules can be partitioned from unmethylated molecules using methyl-binding domain proteins (e.g., MethylMiner Methylated DNA Enrichment Kit (ThermoFisher Scientific)). Subsequently, additional partitioning may involve eluting fragments having different levels of methylation by adjusting the salt concentration in a solution with the methyl-binding domain and bound fragments. As salt concentration increases, molecules having greater methylation levels are eluted.

In some embodiments, the partitioning comprises partitioning the nucleic acid molecules based on a differential binding affinity of the nucleic acid molecules to a binding agent that preferentially binds to nucleic acid molecules comprising methylated nucleotides.

In some embodiments, the partitioned sets are representatives of nucleic acids having different extents of modifications (over representative or under representative of modifications). Over representation and under representation can be defined by the number of methylated nucleotides born by a nucleic acid relative to the median number of methylated nucleotides per molecule in a population. For example, if the median number of 5-methylcytosine nucleotides in nucleic acid molecules in a sample is 2, a nucleic acid molecule including more than two 5-methylcytosine residues is over represented in this modification and a nucleic acid with 1 or zero 5-methylcytosine residues is under represented. The effect of the affinity separation is to partition for nucleic acids over represented in a modification in a bound phase and for nucleic acids underrepresented in a modification in an unbound phase (i.e., in solution). The nucleic acids in the bound phase can be eluted before subsequent processing.

When using MethylMiner Methylated DNA Enrichment Kit (ThermoFisher Scientific) various levels of methylation can be partitioned using sequential elutions. For example, a hypomethylated partition (no methylation) can be separated from a methylated partition by contacting the nucleic acid population with the MBD from the kit, which is attached to magnetic beads. The beads are used to separate out the methylated nucleic acids from the non-methylated nucleic acids. Subsequently, one or more elution steps are performed sequentially to elute nucleic acids having different levels of methylation. For example, a first set of methylated nucleic acids can be eluted at a salt concentration of about 150 mM or about 160 mM or higher, e.g., at least 150 mM, 200mM, 300 mM, 400mM, 500mM, 600mM, 700mM, 800mM, 900mM, 1000mM, or 2000mM. After such methylated nucleic acids are eluted, magnetic separation is once again used to separate higher level of methylated nucleic acids from those with lower level of methylation. The elution and magnetic separation steps can repeat themselves to create various partitions such as a hypomethylated partition (representative of no methylation), an intermediately methylated partition (representative of low level of methylation), and a hyper methylated partition (representative of high level of methylation).

In some methods, nucleic acids bound to an agent used for affinity separation are subjected to a wash step. The wash step washes off nucleic acids weakly bound to the affinity agent. Such nucleic acids can be enriched in nucleic acids having the modification to an extent close to the mean or median (i.e., intermediate between nucleic acids remaining bound to the solid phase and nucleic acids not binding to the solid phase on initial contacting of the sample with the agent). The affinity separation results in at least two, and sometimes three or more partitions of nucleic acids with different extents of a modification. The partitioning of the nucleic acid molecules can be analyzed by sequencing of the nucleic acid molecules partitioned or by digital droplet PCR (ddPCR) or by quantitative PCR (qPCR). In some embodiments, instead of adding the carrier nucleic acid molecules prior to partitioning step, the carrier nucleic acid molecules can be added either during the wash step (i.e., while collecting the intermediate partitioned set) or during the elution step (i.e., while collecting the hyper partitioned set)

In 108, at least a portion of the partitioned sample is processed to generate a processed sample. The processing step comprises tagging; amplifying and/or enriching molecules for specific regions of interest. In some embodiments, prior to the amplification, each of the at least two partitioned sets is differentially tagged. The tagged partitioned sets are then pooled together prior to amplification. Differential tagging of the partitioned sets helps in keeping track of the nucleic acid molecules belonging to a particular partitioned set. The tags are usually provided as components of adapters. The nucleic acid molecules in different partitioned sets receive different tags that can distinguish members of one partitioned set from another. The tags linked to nucleic acid molecules of the same partition set can be the same or different from one another. But if different from one another, the tags can have part of their sequence in common so as to identify the molecules to which they are attached as being of a particular partitioned set. For example, if the molecules of the first sample are partitioned into two partitioned sets - P1 and P2, then the molecules in P1 can be tagged with A1, A2, A3 and so forth and molecules in P2 can be tagged with B1, B2, B3 and so forth. Such a tagging system allows distinguishing the partitioned sets and between the molecules within a partitioned set. In some embodiments, the tags (i.e., molecular barcodes) are part of the adapters and the adapters comprise universal primer binding sites. The adapters comprising the tags are attached via ligation. In some embodiments, the ends of carrier nucleic acid molecules are modified to prevent ligation. In those embodiments, the adapters (that comprises tags - i.e., molecular barcodes) will not be attached to the carrier nucleic acid molecules. Hence, the carrier nucleic acid molecules cannot be amplified using the universal primers. Following tagging, the molecules are amplified using primers that bind to the primer binding region present in the adapters (that are ligated to the molecules). In this amplification, only the polynucleotides obtained from the subject are amplified and not the carrier nucleic acid molecules. Following amplification, the molecules are enriched for specific regions of interest.

In 110, at least a portion of the processed sample is sequenced to generate a set of sequencing reads. In some embodiments, at least a portion of the processed sample from at least two partitioned sets is sequenced to generate a set of sequencing reads. The sequence information obtained comprises sequence of the nucleic acid molecules and the tags (i.e., molecular barcodes) attached to the polynucleotides. From the sequence of the tags (i.e., molecular barcodes) attached to the polynucleotides, one can correlate the tag (i.e., molecular barcodes) with the partitioned set of the polynucleotide. The sequence information is used to identify the polynucleotides (obtained from the subject) and its corresponding partitioned sets. In 112, at least a portion of the set of sequencing reads is analyzed to detect the presence or absence of tumor. In some embodiments, the analysis step comprises determining the methylation status of the molecules. For example, specific regions of interest have been previously determined to be unmethylated in healthy individuals and methylated in individuals with malignant tumors. The analysis step comprises determining whether the molecules in these regions of interest are methylated or not. This is determined based on the number of CpG residues in the molecule and the partitioned set in which the molecule gets partitioned, which in turn will be used to detect the presence or absence of tumor.

### II. Carrier nucleic acid molecules

Carrier nucleic acid molecules are used in analyzing polynucleotides in partitioning assays. In some embodiments, the use of carrier nucleic acid molecules help in increasing the specificity of partitioning of methylated polynucleotides using methyl binding proteins. The methyl binding domain proteins have low affinity for unmethylated molecules. Thus, when incubated with DNA obtained a subject, molecules not containing methylated cytosines can inadvertently be captured. This imperfect specificity reduces the ability to separate efficiently the methylated molecules from unmethylated molecules. Hence, by including carrier nucleic acid molecules in the assay, the carrier nucleic acid molecules may bind to the methyl binding proteins and the unmethylated molecules from the polynucleotide sample will not be able to bind to the methyl binding proteins, thus improving the specificity of partitioning of methylated molecules. Similarly, carrier nucleic acid molecules may be used to improve the partitioning specificity of other methylated nucleic acid-specific binding reagents, such as antibodies, antibody derivative molecules, and the like. In some embodiments, the carrier nucleic acid molecules improve the library preparation of the molecules for next generation sequencing.

In some embodiments, the carrier nucleic acid molecules can have a naturally occurring nucleic acid sequence. In some embodiments, the carrier nucleic acid molecules can have a non-naturally occurring nucleic acid sequence. In some embodiments, the carrier nucleic acid molecules can be synthetic oligonucleotides. In some embodiments, carrier nucleic acid molecules can have a nucleic acid sequence corresponding to a non-human genome. For example, these molecules can either have (i) a sequence corresponding to regions of lambda phage DNA or human genome, (ii) a sequence corresponding to regions of viral genome or bacterial genome, (iii) a non-naturally occurring sequence, and/or (iv) a combination of any of the above. In some embodiments, the carrier DNA molecules can be generated by PCR. In some embodiments, the carrier nucleic acid molecules generated by PCR can be further modified either by treatment with a methyl transferase to incorporate the methyl group to one or more nucleotides in the carrier nucleic acid molecules. In some embodiments, the carrier nucleic acid molecules can be end labelled with a polymerase to incorporate a modified nucleoside so as to prevent the ligation of the carrier nucleic acid molecules to adapters. In some embodiments, the carrier nucleic acid molecules comprise non-naturally occurring nucleoside derivatives. In some embodiments, the carrier nucleic acid molecules can be labeled with biotin or fluorophore.

In another aspect, the present disclosure provides a set of carrier nucleic acid molecules, comprising: (i) at least one subset of unmethylated carrier nucleic acid molecules; and/or (ii) at least one subset of methylated carrier nucleic acid molecules, wherein the unmethylated carrier nucleic acid molecule does not comprise methylated nucleotides and the methylated carrier nucleic acid molecule comprises one or more methylated nucleotides. In some embodiments, at least one end of the carrier nucleic acid molecules is modified to prevent ligation.

In another aspect, the present disclosure provides a set of carrier nucleic acid molecules, comprising: (i) at least one subset of unmethylated carrier nucleic acid molecules; and/or (ii) at least one subset of methylated carrier nucleic acid molecules, wherein the at least one subset of unmethylated carrier nucleic acid molecules does not comprise methylated nucleotides and the at least one subset of methylated carrier nucleic acid molecules comprise methylated nucleotides and at least one end of the carrier nucleic acid molecules is modified to prevent ligation.

In some embodiments, the at least one end of the carrier nucleic acid molecules comprises C3 (propyl group) spacers. In some embodiments, the at least one end of the carrier nucleic acid molecules comprises dideoxy nucleotides. In some embodiments, the at least one end of the carrier nucleic acid molecules comprises any chemical modification that prevents the hydroxyl group from acting as a nucleophile. In some embodiments, the 5'end of the carrier nucleic acid molecules comprises at least one of the following modifications: (i) inverted (5'- 5') - dideoxy thymine, dideoxy cytosine, dideoxy guanine or dideoxy adenine; (ii) propyl group, or (iii) other organic functional groups, for example, but not limited to, benzyl, ethyl or methyl. In some embodiments, the 3'end of the carrier nucleic acid molecules comprises at least one of the following modifications: (i) any dideoxy base such as dideoxy thymine, dideoxy cytosine, dideoxy guanine or dideoxy adenine, that can be added enzymatically or during synthesis; (ii) propyl group, or (iii) other organic functional groups, for example, but not limited to, benzyl, ethyl or methyl.

In some embodiments, the one or more methylated nucleotides is at least one of the following: (i) 5-methylcytosine, (ii) 6-methyladenine, (iii) hydroxymethyl cytosine, (iv) methyl uracil, or (v) any other methylated nucleotide. In some embodiments, the methylated carrier nucleic acid molecules can have 1, 2, 3, 4, 5, 6, 7, 8, 9, 10, 12, 14, 15, 16, 17, 18, 19 or at least 20 methylated nucleotides. In some embodiments, the carrier nucleic acid molecules can be between 25 bp and 325 bp in length. In some embodiments, the unmethylated carrier nucleic acid molecules of one subset can have the same sequence as the unmethylated carrier nucleic acid molecules of another subset. In some embodiments, the methylated carrier nucleic acid molecules of one subset can have the same sequence as the methylated carrier nucleic acid molecules of another subset. In some embodiments, the sequence of the unmethylated carrier nucleic acid molecules in one subset is different from the sequence of the unmethylated carrier nucleic acid molecules in the other subset(s). In some embodiments, the sequence of the methylated carrier nucleic acid molecules in one subset is different from the sequence of the methylated carrier nucleic acid molecules in the other subset(s).

In some embodiments, the one or more subsets of unmethylated carrier nucleic acid molecules and/or methylated carrier nucleic acid molecules can have one or more CpG dinucleotides in the nucleotide sequence. In some embodiments, the one or CpG dinucleotides in the methylated carrier nucleic acid molecules can have one or more methylated cytosines.

In some embodiments, position of the one or more CpG dinucleotides in one subset is different from the position of the one or more CpG dinucleotides in the other subset(s) of the unmethylated carrier nucleic acid molecules. In some embodiments, the number of the CpG dinucleotides in one subset is different from the number of CpG dinucleotides in the other subset(s) of the unmethylated carrier nucleic acid molecules. In some embodiments, the sequence of nucleotides adjacent to the one or more CpG dinucleotides in one subset is different from the sequence of nucleotides adjacent to the one or more CpG dinucleotides in the other subset(s) of unmethylated carrier nucleic acid molecules. In some embodiments, the length of the unmethylated carrier nucleic acid molecules in one subset is different from the length of the unmethylated carrier nucleic acid molecules in the other subset(s) of unmethylated carrier nucleic acid molecules.

In some embodiments, position of the one or more methylated nucleotides in one subset is different from position of the one or more methylated nucleotides in the other subset(s) of the methylated carrier nucleic acid molecules. In some embodiments, the number of the methylated nucleotides in one subset is different from the number of methylated nucleotides in the other subset(s) of the methylated carrier nucleic acid molecules. In some embodiments, the sequence of nucleotides adjacent to the one or more methylated nucleotides in one subset is different from the sequence of nucleotides adjacent to the one or more methylated nucleotides in the other subset(s) of unmethylated carrier nucleic acid molecules. In some embodiments, the length of the methylated carrier nucleic acid molecules in one subset is different from the length of the methylated carrier nucleic acid molecules in the other subset(s) of methylated carrier nucleic acid molecules.

In some embodiments, the amount of the methylated carrier nucleic acid molecules to the unmethylated carrier nucleic acid molecules is about 0:1, 0.1:99.9, 0.5:99.5, 0.75:99.25, 1:99, 1:95, 1:90, 1:80, 1:75, 1:70, 1:60, 1:50, 1:40, 1:30, 1:25, 1:20, 1:10, 1:5, 1:2, 1:1.15, 1:1, 1.15:1, 2:1, 5:1, 10:1, 20:1, 25:1, 30:1, 40:1, 50:1, 60:1, 70:1, 75:1, 80:1, 90:1, 95:1, 99:1,99.25:0.75, 99.5:0.5, 99.9:0.1 or 1:0 ratio. In some embodiments, the amount is in terms of mass. In some embodiments, the amount is in terms of molarity. In some embodiments, the carrier nucleic acid molecules comprise uracil nucleosides.

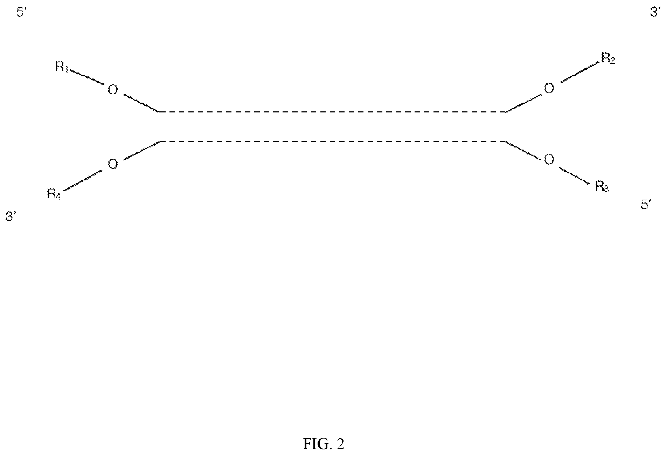

FIG. 2 is a schematic representation of a carrier nucleic acid molecule according to an embodiment of the disclosure. The carrier nucleic acid molecule in FIG. 2 is a double-stranded DNA molecule. '---' region in the double-stranded DNA sequence represent any DNA sequence. R₁ and R₃ represent the modification at the 5' ends of the carrier nucleic acid molecule that prevents the ligation of the carrier nucleic acid molecule. R₂ and R₄ represent the modification at the 3' ends of the carrier nucleic acid molecule that prevents the ligation of the carrier nucleic acid molecule. In some embodiments, the ends of the carrier nucleic acid molecules comprise C3 (propyl group) spacers. In some embodiments, the ends of the carrier nucleic acid molecules comprises dideoxy nucleotides or inverted dideoxy nucleotides. In some embodiments, the 5' end of the carrier nucleic acid molecules comprise inverted dideoxy nucleotides. In some embodiments, the 3' end of the carrier nucleic acid molecules comprise dideoxy nucleotides. In some embodiments, the ends of the carrier nucleic acid molecules comprises any chemical modification that prevents the hydroxyl group from acting as a nucleophile. In some embodiments, the 5'end of the carrier nucleic acid molecules comprises at least one of the following modifications: (i) inverted (5'- 5') - dideoxy thymine, dideoxy cytosine, dideoxy guanine or dideoxy adenine; (ii) propyl group, or (iii) other organic functional groups, for example, but not limited to, benzyl, ethyl or methyl. In some embodiments, the 3'end of the carrier nucleic acid molecules comprises at least one of the following modifications: (i) any dideoxy base such as dideoxy thymine, dideoxy cytosine, dideoxy guanine or dideoxy adenine, that can be added enzymatically or during synthesis; (ii) propyl group, or (iii) other organic functional groups, for example, but not limited to, benzyl, ethyl or methyl. In some embodiments, the DNA sequence of the carrier nucleic acid molecule comprises one or more CpG dinucleotides. In some embodiments, the carrier nucleic acid molecule comprises one or more methylated nucleotides.

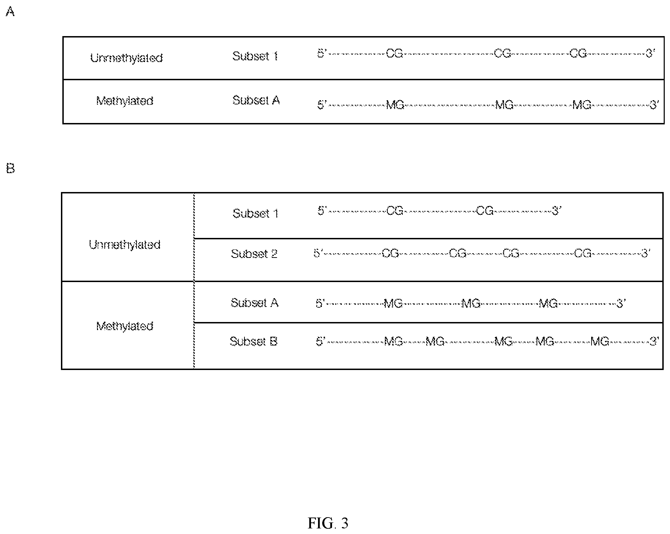

FIG. 3A is a schematic representation of a set of carrier nucleic acid molecules according to an embodiment of the disclosure. In this embodiment, the carrier nucleic acid molecules are double-stranded DNA molecules and will be referred as carrier DNA molecules. Also, in this embodiment, the ends of the carrier DNA molecules are modified to prevent ligation. In other embodiments, the ends of the carrier DNA molecules need not be modified to prevent ligation. For illustration purposes, only one strand of a single carrier DNA molecule for every subset is shown in the figure. In FIG. 3A, '---' region in the carrier DNA molecule represents any other sequence apart from CpG and M represents 5-methylcytosine, C represents cytosine and G represents guanine. In this embodiment, the carrier DNA molecules have one subset (Subset 1) of unmethylated carrier DNA molecules and one subset (Subset A) of methylated carrier DNA molecules. In this embodiment, the sequence of the carrier DNA molecules in both Subset 1 and Subset A is the same. Both Subset 1 and Subset A carrier DNA molecules have 3 CpG dinucleotides. In this embodiment, Subset A, the cytosines in all the three CpG dinucleotides are methylated (hence shown as MG in FIG. 3A). In some embodiments, the carrier DNA molecules can be of any length between 25 bp and 325 bp. In some embodiments, the carrier DNA molecules can be at least 50 bp, at least 60 bp, at least 80 bp, at least 100 bp, at least 120 bp, at least 150 bp, at least 200 bp, at least 250 bp or at least 300 bp.

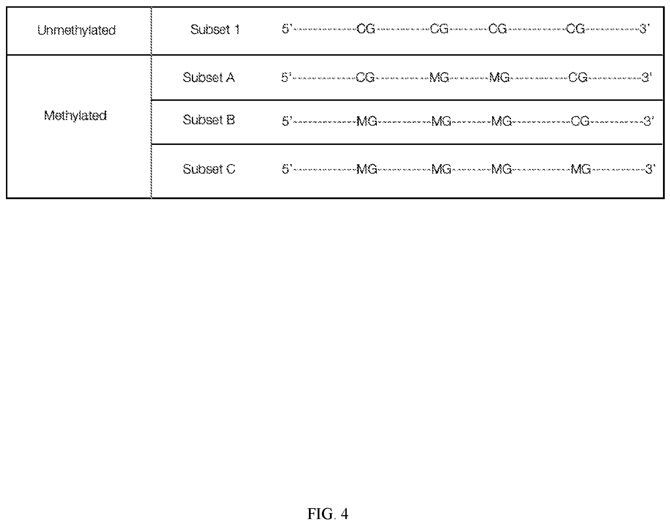

FIG. 3B is a schematic representation of a set of carrier nucleic acid molecules according to an embodiment of the disclosure. In this embodiment, the carrier nucleic acid molecules are double-stranded DNA molecules and will be referred as carrier DNA molecules. Also, in this embodiment, the ends of the carrier DNA molecules are modified to prevent ligation. In other embodiments, the ends of the carrier DNA molecules need not be modified to prevent ligation. For illustration purposes, only one strand of a single carrier DNA molecule for every subset is shown in the figure. In FIG. 3B, '---' region in the carrier DNA molecule represents any other sequence apart from CpG and M represents 5-methylcytosine, C represents cytosine and G represents guanine. In this embodiment, the carrier DNA molecules have two subsets (Subsets 1 and 2) of unmethylated carrier DNA molecules and two subsets (Subsets A and B) of methylated carrier DNA molecules. In this embodiment, each subset (Subsets 1, 2, A and B) have different nucleotide sequences and each subset have a different number of CpG dinucleotides - i.e., Subset 1 has two CpG dinucleotides, Subset 2 has four CpG dinucleotides, Subset A has three CpG dinucleotides and Subset B has five CpG dinucleotides. In this embodiment, all the cytosines of the CpG dinucleotides in the methylated carrier DNA molecules are methylated (hence shown as MG in FIG. 3B). In other embodiments, not all cytosines of the CpG dinucleotides have to be methylated in the methylated carrier DNA molecules (for example, see FIG. 4). In some embodiments, the carrier DNA molecules can be of any length between 25 bp and 325 bp. In some embodiments, the carrier DNA molecules can be at least 50 bp, at least 60 bp, at least 80 bp, at least 100 bp, at least 120 bp, at least 150 bp, at least 200 bp, at least 250 bp or at least 300 bp.

FIG. 4 is a schematic representation of a set of carrier nucleic acid molecules according to an embodiment of the disclosure. In this embodiment, the carrier nucleic acid molecules are double-stranded DNA molecules and will be referred as carrier DNA molecules. Also, in this embodiment, the ends of the carrier DNA molecules are modified to prevent ligation. In other embodiments, the ends of the carrier DNA molecules need not be modified to prevent ligation. In this embodiment, the carrier DNA molecules described herein may take into account of the methylation effect of the methylated cytosines in the CpG dinucleotides during partitioning of the nucleic acid molecules. For illustration purposes, only one strand of a single carrier DNA molecule for every subset is shown in the figure. In FIG. 4, '---' region in the carrier DNA molecule represents any other sequence apart from CpG and M represents 5-methylcytosine, C represents cytosine and G represents guanine. In this embodiment, the carrier DNA molecules have one subset (Subset 1) of unmethylated carrier DNA molecules and three subsets (Subsets A, B and C) of methylated carrier DNA molecules. In this embodiment, the sequence of the carrier DNA molecules in all the subsets are the same - i.e., Subsets 1, A, B and C have the same nucleotide sequence and they all have four CpG dinucleotides. But, the number of methylated nucleotides (in this embodiment, methylated cytosine of the CpG dinucleotides) differ in each subset of the methylated carrier DNA molecules. In this embodiment, Subset A has two methylated cytosines in the CpG nucleotides, Subset B has three methylated cytosines in the CpG nucleotides and Subset C has four methylated cytosines in the CpG nucleotides (shown as MG in FIG. 4). In some embodiments, the carrier DNA molecules can be of any length between 25 bp and 325 bp. In some embodiments, the carrier DNA molecules can be at least 50 bp, at least 60 bp, at least 80 bp, at least 100 bp, at least 120 bp, at least 150 bp, at least 200 bp, at least 250 bp or at least 300 bp.

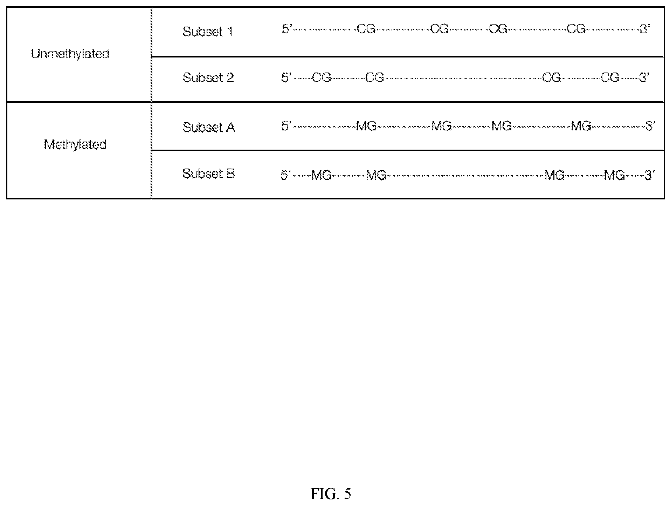

FIG. 5 is a schematic representation of a set of carrier nucleic acid molecules according to an embodiment of the disclosure. In this embodiment, the carrier nucleic acid molecules are double-stranded DNA molecules and will be referred as carrier DNA molecules. Also, in this embodiment, the ends of the carrier DNA molecules are modified to prevent ligation. In other embodiments, the ends of the carrier DNA molecules need not be modified to prevent ligation. In this embodiment, the carrier DNA molecules described herein may take into account of the position-specific effect and/or the methylation effect of the CpG dinucleotides during partitioning of the nucleic acid molecules. For illustration purposes, only one strand of a single carrier DNA molecule for every subset is shown in the figure. In FIG. 5, '---' region in the carrier DNA molecule represents any other sequence apart from CpG and M represents 5-methylcytosine, C represents cytosine and G represents guanine. In this embodiment, the set of carrier DNA molecules have two subsets (Subsets 1 and 2) of unmethylated carrier DNA molecules and two subsets (Subsets A and B) of methylated carrier DNA molecules. In this embodiment, the sequence of the carrier DNA molecules in each subset is different from the other subset - i.e., Subsets 1, 2, A and B have different nucleotide sequences. In this embodiment, the number of CpG dinucleotides is all the subsets is the same - i.e., all the subsets (Subsets 1, 2, A and B) have four CpG dinucleotides. In this embodiment, the number of methylated nucleotides (in this embodiment, methylated cytosine of the CpG dinucleotides) in each subset of the methylated carrier DNA molecules is the same and all the four cytosines in the four CpG dinucleotides are methylated (shown as MG in Subsets A and B of FIG. 5). In this embodiment, the position of CpG dinucleotides is different between Subset 1 and Subset 2 and is different between Subset A and Subset B. But the position of CpG dinucleotides in Subset 1 and Subset A is the same and the position of CpG dinucleotides in Subset 2 and Subset B is the same. In some embodiments, the carrier DNA molecules can be of any length between 25 bp and 325 bp. In some embodiments, the carrier DNA molecules can be at least 50 bp, at least 60 bp, at least 80 bp, at least 100 bp, at least 120 bp, at least 150 bp, at least 200 bp, at least 250 bp or at least 300 bp.

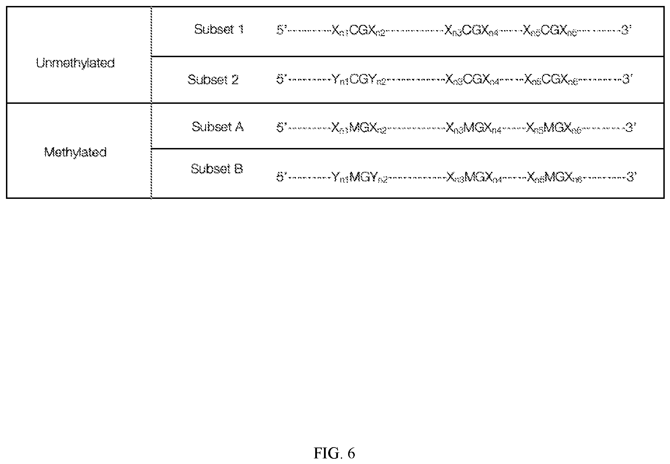

FIG. 6 is a schematic representation of a set of carrier nucleic acid molecules according to an embodiment of the disclosure. In this embodiment, the carrier nucleic acid molecules are double-stranded DNA molecules and will be referred as carrier DNA molecules. Also, in this embodiment, the ends of the carrier DNA molecules are modified to prevent ligation. In other embodiments, the ends of the carrier DNA molecules need not be modified to prevent ligation. In this embodiment, the carrier DNA molecules described herein may take into account of the sequence-specific effect of the nucleotides adjacent to the CpG dinucleotides during partitioning of the nucleic acid molecules. For illustration purposes, only one strand of a single carrier DNA molecule for every subset is shown in the figure. In FIG. 6, '---' region in the carrier DNA molecule represents any other sequence apart from CpG dinucleotide; M represents 5-methylcytosine, C represents cytosine and G represents guanine; Xₙᵢ and Yₙᵢ can be any two different nucleotide sequences that are of same length ni, where ni can be n1, n2, n3, n4, n5 or n6 - i.e., for example, Xₙ₁ and Yₙ₁ are two different nucleotide sequences of length n1 and n1, n2, n3, n4, n5 and n6 can be any integer between 0 and 30. In this embodiment, the set of carrier DNA molecules have two subsets (Subsets 1 and 2) of unmethylated carrier DNA molecules and two subsets (Subsets A and B) of methylated carrier DNA molecules and the number of CpG dinucleotides in all the subsets are the same - i.e., Subsets 1, 2, A and B have four CpG dinucleotides. But the sequence of the nucleotides adjacent to the CpG dinucleotides is different between the two subsets of unmethylated carrier DNA molecules - i.e., the sequence of the nucleotides (Xₙᵢ and Yₙᵢ) adjacent to the CpG dinucleotides are different in Subset 1 and Subset 2. Likewise, the sequence of the nucleotides adjacent to the CpG dinucleotides is different between the two subsets of methylated carrier DNA molecules - i.e., the sequence of the nucleotides (Xₙᵢ and Yₙᵢ) adjacent to the CpG dinucleotides are different in Subset A and Subset B. But the sequence of the nucleotides (Xₙᵢ) adjacent to the CpG dinucleotides is the same in Subset 1 and Subset A and also the same in Subset 1 and the sequence of the nucleotides (Yₙᵢ) adjacent to the CpG dinucleotides is the same in Subset 2 and Subset B. In some embodiments, the carrier DNA molecules can be of any length between 25 bp and 325 bp. In some embodiments, the carrier DNA molecules can be at least 50 bp, at least 60 bp, at least 80 bp, at least 100 bp, at least 120 bp, at least 150 bp, at least 200 bp, at least 250 bp or at least 300 bp. In some embodiments, ni can be a nucleotide sequence of length up to 5 bp.

In another aspect, the present disclosure provides a population of nucleic acids, comprising: (i) a set of carrier nucleic acid molecules, comprising: i) at least one subset of unmethylated carrier nucleic acid molecules; and/or (ii) at least one subset of methylated carrier nucleic acid molecules, wherein at least one end of the carrier nucleic acid molecules is modified to prevent ligation, wherein the unmethylated carrier nucleic acid molecule does not comprise methylated nucleotides and the methylated carrier nucleic acid molecule comprises one or more methylated nucleotides; and (ii) a polynucleotide sample obtained from a subject.

In some embodiments, carrier nucleic acid molecules can either have a sequence corresponding to regions of: (i) a viral genome, (ii) a bacterial genome, (iii) lambda phage genome, (iv) human genome, (iv) any naturally occurring sequence, (v) a non-naturally occurring sequence, (vi) non-human genome and/or (vii) a combination of any of the above. In some embodiments, the carrier nucleic acid molecules can comprise a non-naturally occurring sequence. In some embodiments, the carrier nucleic acid molecules can comprise a naturally occurring sequence. In some embodiments, the carrier DNA molecules can be generated by PCR. In some embodiments, the carrier nucleic acid molecules generated by PCR can be further modified either by treatment with a methyl transferase to incorporate the methyl group to one or more nucleotides in the carrier nucleic acid molecules. In some embodiments, the carrier nucleic acid molecules can be end labelled with a polymerase to incorporate a modified nucleoside so as to prevent the ligation of the carrier nucleic acid molecules to adapters. In some embodiments, the carrier nucleic acid molecules comprise non-naturally occurring nucleoside derivatives. In some embodiments, the carrier nucleic acid molecules can be labeled with biotin or fluorophore.

In some embodiments, the polynucleotide sample is obtained from tissue, blood, plasma, serum, urine, saliva, stool, cerebral spinal fluid, buccal swab or pleural tap. In some embodiments, the polynucleotide sample is obtained from tissue. In some embodiments, the polynucleotide sample obtained from the tissue is fragmented by enzymatic or mechanical means. In some embodiments, the polynucleotide sample is obtained from blood. In some embodiments, the polynucleotide sample obtained from the blood is a cell-free DNA sample. In some embodiments, the polynucleotide sample is a cell-free DNA sample.

In some embodiments, the polynucleotide sample is a DNA sample, RNA sample, cell-free polynucleotide sample, cell-free DNA sample or cell-free RNA sample. In some embodiments, the polynucleotide sample is a cell-free DNA sample.

In some embodiments, the amount of the at least one subset of methylated carrier nucleic acid molecules to the at least one subset of unmethylated carrier nucleic acid molecules is about 0:1, 0.1:99.9, 0.5:99.5, 0.75:99.25, 1:99, 1:95, 1:90, 1:80, 1:75, 1:70, 1:60, 1:50, 1:40, 1:30, 1:25, 1:20, 1:10, 1:5, 1:2, 1:1.15, 1:1, 1.15:1, 2:1, 5:1, 10:1, 20:1, 25:1, 30:1, 40:1, 50:1, 60:1, 70:1, 75:1, 80:1, 90:1, 95:1, 99:1,99.25:0.75, 99.5:0.5, 99.9:0.1 or 1:0 ratio. In some embodiments, amount of the polynucleotide sample to the set of carrier nucleic acid molecules is about 1: 0.1; 1:0.2, 1:0.3, 1:4, 1:0.5, 1:6, 1:7, 1:8, 1:0.9, 1:1, 1:1, 1:2, 1:3, 1:4, 1:5, 1:6, 1:7, 1:8, 1:9, 1:10 ratio, 1:20, 1:30, 1:40, 1:50, 1:60, 1:70, 1:80, 1:90, 1:100, 1:200, 1:300; 1:400, 1:500, 1:600, 1:700, 1:800, 1:900, 1:1000, 1:5000, 1:10,000, 1:100,000, 1:500,000, 1:10⁶, 1:10⁷, 1:10⁸ or 1:10⁹. In some embodiments, the amount is in terms of mass. In some embodiments, the amount is in terms of molarity.

In some embodiments, the polynucleotide sample is at least 1 ng, at least 5 ng, at least 10 ng, at least 15 ng, at least 20 ng, at least 30 ng, at least 50 ng, at least 75 ng, at least 100 ng, at least 150 ng, at least 200 ng, at least 250 ng, at least 300 ng, at least 350 ng, at least 400 ng, at least 450 ng, at least 500 ng, at least 750 ng or at least 1 µg. In some embodiments, the polynucleotide sample is up to 1 µg. In some embodiments, the polynucleotide sample is up to 200 ng. In some embodiments, the polynucleotide sample is up to 150 ng. In some embodiments, the polynucleotide sample is up to 100 ng. In some embodiments, the set of carrier nucleic acid molecules is added in a sufficient amount such that total amount of the polynucleotide sample and the set of carrier nucleic acid molecules is about 175 ng, 200 ng, 225 ng, 250 ng, 275 ng, 300 ng, 350 ng, 400 ng, 450 ng, 500 ng, 600 ng, 700 ng, 750 ng, 800 ng, 900 ng, 1 µg, 1.1 µg, 1.25 µg or 1.5 µg.

### III. General Features of the Methods

### A. Samples

A sample can be any biological sample isolated from a subject. Samples can include body tissues, whole blood, platelets, serum, plasma, stool, red blood cells, white blood cells or leucocytes, endothelial cells, tissue biopsies (e.g., biopsies from known or suspected solid tumors), cerebrospinal fluid, synovial fluid, lymphatic fluid, ascites fluid, interstitial or extracellular fluid (e.g., fluid from intercellular spaces), gingival fluid, crevicular fluid, bone marrow, pleural effusions, cerebrospinal fluid, saliva, mucous, sputum, semen, sweat, and urine. Samples may be bodily fluids, such as blood and fractions thereof, and urine. Such samples can include nucleic acids shed from tumors. The nucleic acids can include DNA and RNA and can be in double and single-stranded forms. A sample can be in the form originally isolated from a subject or can have been subjected to further processing to remove or add components, such as cells, enrich for one component relative to another, or convert one form of nucleic acid to another, such as RNA to DNA or single-stranded nucleic acids to double-stranded. Thus, for example, a bodily fluid for analysis can be plasma or serum containing cell-free nucleic acids, e.g., cell-free DNA (cfDNA).

In some embodiments, the sample volume of bodily fluid taken from a subject depends on the desired read depth for sequenced regions. Examples of volumes are about 0.4-40 milliliters (mL), about 5-20 mL, about 10-20 mL. For example, the volume can be about 0.5 mL, about 1 mL, about 5 mL, about 10 mL, about 20 mL, about 30 mL, about 40 mL, or more milliliters. A volume of sampled plasma is typically between about 5 mL to about 20 mL.

The sample can comprise various amounts of nucleic acid. Typically, the amount of nucleic acid in a given sample is equates with multiple genome equivalents. For example, a sample of about 30 nanograms (ng) DNA can contain about 10,000 (10⁴) haploid human genome equivalents and, in the case of cfDNA, about 200 billion (2 x 10¹¹) individual polynucleotide molecules. Similarly, a sample of about 100 ng of DNA can contain about 30,000 haploid human genome equivalents and, in the case of cfDNA, about 600 billion individual molecules.

In some embodiments, a sample comprises nucleic acids from different sources, e.g., from cells and from cell-free sources (e.g., blood samples, etc.). Typically, a sample includes nucleic acids carrying mutations. For example, a sample optionally comprises DNA carrying germline mutations and/or somatic mutations. Typically, a sample comprises DNA carrying cancer-associated mutations (e.g., cancer-associated somatic mutations).

Example amounts of cell-free nucleic acids in a sample before amplification typically range from about 1 femtogram (fg) to about 1 microgram (µg), e.g., about 1 picogram (pg) to about 200 nanograms (ng), about 1 ng to about 100 ng, about 10 ng to about 1000 ng. In some embodiments, a sample includes up to about 600 ng, up to about 500 ng, up to about 400 ng, up to about 300 ng, up to about 200 ng, up to about 100 ng, up to about 50 ng, or up to about 20 ng of cell-free nucleic acid molecules. Optionally, the amount is at least about 1 fg, at least about 10 fg, at least about 100 fg, at least about 1 pg, at least about 10 pg, at least about 100 pg, at least about 1 ng, at least about 10 ng, at least about 100 ng, at least about 150 ng, or at least about 200 ng of cell-free nucleic acid molecules. In some embodiments, the amount is up to about 1 fg, about 10 fg, about 100 fg, about 1 pg, about 10 pg, about 100 pg, about 1 ng, about 10 ng, about 100 ng, about 150 ng, about 200 ng, about 300 ng, about 400 ng, about 500 ng, aout 600 ng, about 700 ng, about 800 ng, about 900 ng or about 1 µg of cell-free nucleic acid molecules. In some embodiments, methods include obtaining between about 1 fg to about 200 ng cell-free nucleic acid molecules from samples.

Cell-free nucleic acids typically have a size distribution of between about 100 nucleotides in length and about 500 nucleotides in length, with molecules of about 110 nucleotides in length to about 230 nucleotides in length representing about 90% of molecules in the sample, with a mode of about 168 nucleotides length (in samples from human subjects) and a second minor peak in a range between about 240 nucleotides to about 440 nucleotides in length. In some embodiments, cell-free nucleic acids are from about 160 nucleotides to about 180 nucleotides in length, or from about 320 nucleotides to about 360 nucleotides in length, or from about 440 nucleotides to about 480 nucleotides in length.

In some embodiments, cell-free nucleic acids are isolated from bodily fluids through a partitioning step in which cell-free nucleic acids, as found in solution, are separated from intact cells and other non-soluble components of the bodily fluid. In some embodiments, partitioning includes techniques such as centrifugation or filtration. Alternatively, cells in bodily fluids may be lysed, and cell-free and cellular nucleic acids may be processed together. Generally, after addition of buffers and wash steps, cell-free nucleic acids may be precipitated with, for example, an alcohol. In some embodiments, additional clean-up steps are used, such as silica-based columns to remove contaminants or salts. Non-specific bulk carrier nucleic acids, for example, are optionally added throughout the reaction to optimize aspects of the example procedure, such as yield. After such processing, samples typically include various forms of nucleic acids including double-stranded DNA, single-stranded DNA and/or single-stranded RNA. Optionally, single-stranded DNA and/or single-stranded RNA are converted to double-stranded forms so that they are included in subsequent processing and analysis steps.

### B. Partitioning and Tagging

In some embodiments, the nucleic acid molecules (from the sample of polynucleotides) may be tagged with sample indexes and/or molecular barcodes (referred to generally as "tags"). Tags may be incorporated into or otherwise joined to adapters by chemical synthesis, ligation (e.g., blunt-end ligation or sticky-end ligation), or overlap extension polymerase chain reaction (PCR), among other methods. Such adapters may be ultimately joined to the target nucleic acid molecule. In other embodiments, one or more rounds of amplification cycles (e.g., PCR amplification) are generally applied to introduce sample indexes to a nucleic acid molecule using conventional nucleic acid amplification methods. The amplifications may be conducted in one or more reaction mixtures (e.g., a plurality of microwells in an array). Molecular barcodes and/or sample indexes may be introduced simultaneously, or in any sequential order. In some embodiments, molecular barcodes and/or sample indexes are introduced prior to and/or after sequence capturing steps are performed. In some embodiments, only the molecular barcodes are introduced prior to probe capturing and the sample indexes are introduced after sequence capturing steps are performed. In some embodiments, both the molecular barcodes and the sample indexes are introduced prior to performing probe-based capturing steps. In some embodiments, the sample indexes are introduced after sequence capturing steps are performed. In some embodiments, molecular barcodes are incorporated to the nucleic acid molecules (e.g. cfDNA molecules) in a sample through adapters via ligation (e.g., blunt-end ligation or sticky-end ligation). In some embodiments, sample indexes are incorporated to the nucleic acid molecules (e.g. cfDNA molecules) in a sample through overlap extension polymerase chain reaction (PCR). Typically, sequence capturing protocols involve introducing a single-stranded nucleic acid molecule complementary to a targeted nucleic acid sequence, e.g., a coding sequence of a genomic region and mutation of such region is associated with a cancer type.

In some embodiments, the tags may be located at one end or at both ends of the sample nucleic acid molecule. In some embodiments, tags are predetermined or random or semi-random sequence oligonucleotides. In some embodiments, the tags may be less than about 500, 200, 100, 50, 20, 10, 9, 8, 7, 6, 5, 4, 3, 2, or 1 nucleotides in length. The tags may be linked to sample nucleic acids randomly or non-randomly.

In some embodiments, each sample is uniquely tagged with a sample index or a combination of sample indexes. In some embodiments, each nucleic acid molecule of a sample or sub-sample is uniquely tagged with a molecular barcode or a combination of molecular barcodes. In other embodiments, a plurality of molecular barcodes may be used such that molecular barcodes are not necessarily unique to one another in the plurality (e.g., non-unique molecular barcodes). In these embodiments, molecular barcodes are generally attached (e.g., by ligation) to individual molecules such that the combination of the molecular barcode and the sequence it may be attached to creates a unique sequence that may be individually tracked. Detection of non-unique molecular barcodes in combination with endogenous sequence information (e.g., the beginning (start) and/or end (stop) genomic location/position corresponding to the sequence of the original nucleic acid molecule in the sample, start and stop genomic positions corresponding to the sequence of the original nucleic acid molecule in the sample, the beginning (start) and/or end (stop) genomic location/position of the sequence read that is mapped to the reference sequence, start and stop genomic positions of the sequence read that is mapped to the reference sequence, sub-sequences of sequence reads at one or both ends, length of sequence reads, and/or length of the original nucleic acid molecule in the sample) typically allows for the assignment of a unique identity to a particular molecule. In some embodiments, beginning region comprises the first 1, first 2, the first 5, the first 10, the first 15, the first 20, the first 25, the first 30 or at least the first 30 base positions at the 5' end of the sequencing read that align to the reference sequence. In some embodiments, the end region comprises the last 1, last 2, the last 5, the last 10, the last 15, the last 20, the last 25, the last 30 or at least the last 30 base positions at the 3' end of the sequencing read that align to the reference sequence. The length, or number of base pairs, of an individual sequence read are also optionally used to assign a unique identity to a given molecule. As described herein, fragments from a single strand of nucleic acid having been assigned a unique identity, may thereby permit subsequent identification of fragments from the parent strand, and/or a complementary strand.

In certain embodiments, the number of different tags used to uniquely identify a number of molecules, z, in a class can be between any of 2*z, 3*z, 4*z, 5*z, 6*z, 7*z, 8*z, 9*z, 10*z, 11 *z, 12*z, 13*z, 14*z, 15*z, 16*z, 17*z, 18*z, 19*z, 20*z or 100*z (e.g., lower limit) and any of 100,000*z, 10,000*z, 1000*z or 100*z (e.g., upper limit). In some embodiments, molecular barcodes are introduced at an expected ratio of a set of identifiers (e.g., a combination of unique or non-unique molecular barcodes) to molecules in a sample. One example format uses from about 2 to about 1,000,000 different molecular barcode sequences, or from about 5 to about 150 different molecular barcode sequences, or from about 20 to about 50 different molecular barcode sequences, ligated to both ends of a target molecule. Alternatively, from about 25 to about 1,000,000 different molecular barcode sequences may be used. For example, 20-50 x 20-50 molecular barcode sequences (i.e., one of the 20-50 different molecular barcode sequences can be attached to each end of the target molecule) can be used. Such numbers of identifiers are typically sufficient for different molecules having the same start and stop points to have a high probability (e.g., at least 94%, 99.5%, 99.99%, or 99.999%) of receiving different combinations of identifiers. In some embodiments, about 80%, about 90%, about 95%, or about 99% of molecules have the same combinations of molecular barcodes.

In some embodiments, the assignment of unique or non-unique molecular barcodes in reactions is performed using methods and systems described in, for example, U.S. Patent Application Nos. 20010053519, 20030152490, and 20110160078, and U.S. Patent Nos. 6,582,908, 7,537,898, 9,598,731, and 9,902,992. Alternatively, in some embodiments, different nucleic acid molecules of a sample may be identified using only endogenous sequence information (e.g., start and/or stop positions, sub-sequences of one or both ends of a sequence, and/or lengths).

In certain embodiments described herein, a population of different forms of nucleic acids (e.g., hypermethylated and hypomethylated DNA in a sample) can be physically partitioned prior to analysis, e.g., sequencing, or tagging and sequencing. This approach can be used to determine, for example, whether hypermethylation variable epigenetic target regions show hypermethylation characteristic of tumor cells or hypomethylation variable epigenetic target regions show hypomethylation characteristic of tumor cells. Additionally, by partitioning a heterogeneous nucleic acid population, one may increase rare signals, e.g., by enriching rare nucleic acid molecules that are more prevalent in one fraction (or partition) of the population. For example, a genetic variation present in hyper-methylated DNA but less (or not) in hypomethylated DNA can be more easily detected by partitioning a sample into hyper-methylated and hypo-methylated nucleic acid molecules. By analyzing multiple fractions of a sample, a multi-dimensional analysis of a single locus of a genome or species of nucleic acid can be performed and hence, greater sensitivity can be achieved.

In some instances, a heterogeneous nucleic acid sample is partitioned into two or more partitions (e.g., at least 3, 4, 5, 6 or 7 partitions). In some embodiments, each partition is differentially tagged - i.e., each partition can have a different set of molecular barcodes. Tagged partitions can then be pooled together for collective sample prep and/or sequencing. The partitioning-tagging-pooling steps can occur more than once, with each round of partitioning occurring based on a different characteristics (examples provided herein) and tagged using differential tags that are distinguished from other partitions and partitioning means.

Examples of characteristics that can be used for partitioning include sequence length, methylation level, nucleosome binding, sequence mismatch, immunoprecipitation, and/or proteins that bind to DNA. Resulting partitions can include one or more of the following nucleic acid forms: single-stranded DNA (ssDNA), double-stranded DNA (dsDNA), shorter DNA fragments and longer DNA fragments. In some embodiments, a heterogeneous population of nucleic acids is partitioned into nucleic acids with one or more epigenetic modifications and without the one or more epigenetic modifications. Examples of epigenetic modifications include presence or absence of methylation; level of methylation; type of methylation (e.g., 5-methylcytosine versus other types of methylation, such as adenine methylation and/or cytosine hydroxymethylation); and association and level of association with one or more proteins, such as histones. Alternatively, or additionally, a heterogeneous population of nucleic acids can be partitioned into nucleic acid molecules associated with nucleosomes and nucleic acid molecules devoid of nucleosomes. Alternatively, or additionally, a heterogeneous population of nucleic acids may be partitioned into single-stranded DNA (ssDNA) and double-stranded DNA (dsDNA). Alternatively, or additionally, a heterogeneous population of nucleic acids may be partitioned based on nucleic acid length (e.g., molecules of up to 160 bp and molecules having a length of greater than 160 bp).

In some instances, each partition (representative of a different nucleic acid form) is differentially tagged with molecular barcodes, and the partitions are pooled together prior to sequencing. In other instances, the different forms are separately sequenced. In some embodiments, a single tag can be used to label a specific partition. In some embodiments, multiple different tags can be used to label a specific partition. In embodiments employing multiple different tags to label a specific partition, the set of tags used to label one partition can be readily differentiated from the set of tags used to label other partitions. In some embodiments, a tag can be multifunctional - i.e., it can simultaneously act as a molecular identifier (i.e., molecular barcode), partition identifier (i.e., partition tag) and sample identifier (i.e., sample index). For example, if there are four DNA samples and each DNA sample is partitioned into three partitions, then the DNA molecules in each of the twelve partitions (i.e., twelve partitions for the four DNA samples in total) can be tagged with a separate set of tags such that the tag sequence attached to the DNA molecule reveals the identity of the DNA molecule, the partition it belongs to and the sample from which it was originated. In some embodiments, a tag can be used both as a molecular barcode and as a partition tag. For example, if a DNA sample is partitioned into three partitions, then DNA molecule in each partition is tagged with a separated set of tags such that the tag sequence attached to a DNA molecule reveals the identity of the DNA molecule and the partition it belongs to. In some embodiments, a tag can be used both as a molecular barcode and as a sample index. For example, if there are four DNA samples, then DNA molecules in each sample with be tagged with a separate set of tags that can be distinguishable from each sample such that the tag sequence attached to the DNA molecule serves as a molecule identifier and as a sample identifier.

In one embodiment, partition tagging comprises tagging molecules in each partition with a partition tag. After re-combining partitions and sequencing molecules, the partition tags identify the source partition. In another embodiment, different partitions are tagged with different sets of molecular tags, e.g., comprised of a pair of barcodes. In this way, each molecular barcode indicates the source partition as well as being useful to distinguish molecules within a partition. For example, a first set of 35 barcodes can be used to tag molecules in a first partition, while a second set of 35 barcodes can be used tag molecules in a second partition.

In some embodiments, after partitioning and tagging with partition tags, the molecules may be pooled for sequencing in a single run. In some embodiments, a sample tag is added to the molecules, e.g., in a step subsequent to addition of partition tags and pooling. Sample tags can facilitate pooling material generated from multiple samples for sequencing in a single sequencing run.

Alternatively, in some embodiments, partition tags may be correlated to the sample as well as the partition. As a simple example, a first tag can indicate a first partition of a first sample; a second tag can indicate a second partition of the first sample; a third tag can indicate a first partition of a second sample; and a fourth tag can indicate a second partition of the second sample.

While tags may be attached to molecules already partitioned based on one or more epigenetic characteristics, the final tagged molecules in the library may no longer possess that epigenetic characteristic. For example, while single stranded DNA molecules may be partitioned and tagged, the final tagged molecules in the library are likely to be double stranded. Similarly, while DNA may be subject to partition based on different levels of methylation, in the final library, tagged molecules derived from these molecules are likely to be unmethylated. Accordingly, the tag attached to molecule in the library typically indicates the characteristic of the "parent molecule" from which the ultimate tagged molecule is derived, not necessarily to characteristic of the tagged molecule, itself.

As an example, barcodes 1, 2, 3, 4, etc. are used to tag and label molecules in the first partition; barcodes A, B, C, D, etc. are used to tag and label molecules in the second partition; and barcodes a, b, c, d, etc. are used to tag and label molecules in the third partition. Differentially tagged partitions can be pooled prior to sequencing. Differentially tagged partitions can be separately sequenced or sequenced together concurrently, e.g., in the same flow cell of an Illumina sequencer.

After sequencing, analysis of reads to detect genetic variants can be performed on a partition-by-partition level, as well as a whole nucleic acid population level. Tags are used to sort reads from different partitions. Analysis can include in silico analysis to determine genetic and epigenetic variation (one or more of methylation, chromatin structure, etc.) using sequence information, genomic coordinates length, coverage and/or copy number. In some embodiments, higher coverage can correlate with higher nucleosome occupancy in genomic region while lower coverage can correlate with lower nucleosome occupancy or a nucleosome depleted region (NDR).

### C. Amplification

Sample nucleic acids may be flanked by adapters and amplified by PCR and other amplification methods using nucleic acid primers binding to primer binding sites in adapters flanking a DNA molecule to be amplified. In some embodiments, amplification methods involve cycles of extension, denaturation, and annealing resulting from thermocycling, or can be isothermal as, for example, in transcription mediated amplification. Other examples of amplification methods that may be optionally utilized include the ligase chain reaction, strand displacement amplification, nucleic acid sequence-based amplification, and self-sustained sequence-based replication.

Typically, the amplification reactions generate a plurality of non-uniquely or uniquely tagged nucleic acid amplicons with molecular barcodes and sample indexes at size ranging from about 150 nucleotides (nt), to about 700 nt, from 250 nt to about 350 nt, or from about 320 nt to about 550 nt. In some embodiments, the amplicons have a size of about 180 nt. In some embodiments, the amplicons have a size of about 200 nt.

### D. Enrichment/Capturing

In some embodiments, sequences are enriched prior to sequencing the nucleic acids. Enrichment optionally performed for specific target regions or nonspecifically ("target sequences"). In some embodiments, targeted regions of interest may be enriched/captured with nucleic acid capture probes ("baits") selected for one or more bait set panels using a differential tiling and capture scheme. A differential tiling and capture scheme generally uses bait sets of different relative concentrations to differentially tile (e.g., at different "resolutions") across genomic regions associated with the baits, subject to a set of constraints (e.g., sequencer constraints such as sequencing load, utility of each bait, etc.), and capture the targeted nucleic acids at a desired level for downstream sequencing. These targeted genomic regions of interest optionally include natural or synthetic nucleotide sequences of the nucleic acid construct. In some embodiments, biotin-labeled beads with probes to one or more regions of interest can be used to capture target sequences, and optionally followed by amplification of those regions, to enrich for the regions of interest.

Sequence capture typically involves the use of oligonucleotide probes that hybridize to the target nucleic acid sequence. In some embodiments, a probe set strategy involves tiling the probes across a region of interest. Such probes can be, for example, from about 60 to about 120 nucleotides in length. The set can have a depth (e.g., depth of coverage) of about 2X, 3X, 4X, 5X, 6X, 7X, 8X, 9X, 10X, 15X, 20X, 50X, or more than 50X. The effectiveness of sequence capture generally depends, in part, on the length of the sequence in the target molecule that is complementary (or nearly complementary) to the sequence of the probe.

In some embodiments, the enriched DNA molecules (or the captured set) may comprise DNA corresponding to a sequence-variable target region set and an epigenetic target region set. In some embodiments the quantity of captured sequence-variable target region DNA is greater than the quantity of the captured epigenetic target region DNA, when normalized for the difference in the size of the targeted regions (footprint size). In some embodiments, the compositions, methods and systems described in PCT Patent Application No. PCT/US2020/016120, which is hereby incorporated by reference in its entirety.

Alternatively, first and second captured sets may be provided, comprising, respectively, DNA corresponding to a sequence-variable target region set and DNA corresponding to an epigenetic target region set. The first and second captured sets may be combined to provide a combined captured set.

In a captured set comprising DNA corresponding to the sequence-variable target region set and the epigenetic target region set, including a combined captured set as discussed above, the DNA corresponding to the sequence-variable target region set may be present at a greater concentration than the DNA corresponding to the epigenetic target region set, e.g., a 1.1 to 1.2-fold greater concentration, a 1.2- to 1.4-fold greater concentration, a 1.4- to 1.6-fold greater concentration, a 1.6- to 1.8-fold greater concentration, a 1.8- to 2.0-fold greater concentration, a 2.0- to 2.2-fold greater concentration, a 2.2- to 2.4-fold greater concentration a 2.4- to 2.6-fold greater concentration, a 2.6- to 2.8-fold greater concentration, a 2.8- to 3.0-fold greater concentration, a 3.0- to 3.5-fold greater concentration, a 3.5- to 4.0, a 4.0- to 4.5-fold greater concentration, a 4.5- to 5.0-fold greater concentration, a 5.0- to 5.5-fold greater concentration, a 5.5- to 6.0-fold greater concentration, a 6.0- to 6.5-fold greater concentration, a 6.5- to 7.0-fold greater, a 7.0- to 7.5-fold greater concentration, a 7.5-to 8.0-fold greater concentration, an 8.0- to 8.5-fold greater concentration, an 8.5- to 9.0-fold greater concentration, a 9.0- to 9.5-fold greater concentration, 9.5- to 10.0-fold greater concentration, a 10- to 11-fold greater concentration, an 11- to 12-fold greater concentration a 12- to 13-fold greater concentration, a 13- to 14-fold greater concentration, a 14- to 15-fold greater concentration, a 15- to 16-fold greater concentration, a 16- to 17-fold greater concentration, a 17- to 18-fold greater concentration, an 18- to 19-fold greater concentration, or a 19- to 20-fold greater concentration. The degree of difference in concentrations accounts for normalization for the footprint sizes of the target regions, as discussed in the definition section.

### i. Epigenetic target region set

The epigenetic target region set may comprise one or more types of target regions likely to differentiate DNA from neoplastic (e.g., tumor or cancer) cells and from healthy cells, e.g., non-neoplastic circulating cells. Exemplary types of such regions are discussed in detail herein. In some embodiments, methods according to the disclosure comprise determining whether cfDNA molecules corresponding to the epigenetic target region set comprise or indicate cancer-associated epigenetic modifications (e.g., hypermethylation in one or more hypermethylation variable target regions; one or more perturbations of CTCF binding; and/or one or more perturbations of transcription start sites) and/or copy number variations (e.g., focal amplifications). The epigenetic target region set may also comprise one or more control regions, e.g., as described herein.

In some embodiments, the epigenetic target region set has a footprint of at least 100 kb, e.g., at least 200 kb, at least 300 kb, or at least 400 kb. In some embodiments, the epigenetic target region set has a footprint in the range of 100-1000 kb, e.g., 100-200 kb, 200-300 kb, 300-400 kb, 400-500 kb, 500-600 kb, 600-700 kb, 700-800 kb, 800-900 kb, and 900-1,000 kb.

### 1. Hypermethylation variable target regions

In some embodiments, the epigenetic target region set comprises one or more hypermethylation variable target regions. In general, hypermethylation variable target regions refer to regions where an increase in the level of observed methylation indicates an increased likelihood that a sample (e.g., of cfDNA) contains DNA produced by neoplastic cells, such as tumor or cancer cells. For example, hypermethylation of promoters of tumor suppressor genes has been observed repeatedly. See, e.g., Kang et al., Genome Biol. 18:53 (2017) and references cited therein.

An extensive discussion of methylation variable target regions in colorectal cancer is provided in Lam et al., Biochim Biophys Acta. 1866:106-20 (2016). These include VIM, SEPT9, ITGA4, OSM4, GATA4 and NDRG4. An exemplary set of hypermethylation variable target regions comprising the genes or portions thereof based on the colorectal cancer (CRC) studies is provided in Table 1. Many of these genes likely have relevance to cancers beyond colorectal cancer; for example, TP53 is widely recognized as a critically important tumor suppressor and hypermethylation-based inactivation of this gene may be a common oncogenic mechanism.

**Table 1. Exemplary hypermethylation target regions (genes or portions thereof) based on CRC studies.**

| **Gene Name** | **Additional Gene Name** | **Chromosome** |
|---|---|---|
| VIM | | chr10 |
| SEPT9 | | chr17 |
| CYCD2 | CCND2 | chr12 |
| TFPI2 | | chr7 |
| GATA4 | | chr8 |
| RARB2 | RARB | chr3 |
| p16INK4a | CDKN2A | chr9 |
| MGMT | MGMT | chr10 |
| APC | | chr5 |
| NDRG4 | | chr16 |
| HLTF | | chr3 |
| HPP1 | TMEFF2 | chr2 |
| hMLH1 | MLH1 | chr3 |
| RASSF1A | RASSF1 | chr3 |
| CDH13 | | chr16 |
| IGFBP3 | | chr7 |
| ITGA4 | | chr2 |

In some embodiments, the hypermethylation variable target regions comprise a plurality of genes or portions thereof listed in Table 1, e.g., at least 10%, 20%, 30%, 40%, 50%, 60%, 70%, 80%, 90%, or 100% of the genes or portions thereof listed in Table 1. For example, for each locus included as a target region, there may be one or more probes with a hybridization site that binds between the transcription start site and the stop codon (the last stop codon for genes that are alternatively spliced) of the gene. In some embodiments, the one or more probes bind within 300 bp upstream and/or downstream of the genes or portions thereof listed in Table 1, e.g., within 200 or 100 bp.

Methylation variable target regions in various types of lung cancer are discussed in detail, e.g., in Ooki et al., Clin. Cancer Res. 23:7141-52 (2017); Belinksy, Annu. Rev. Physiol. 77:453-74 (2015); Hulbert et al., Clin. Cancer Res. 23:1998-2005 (2017); Shi et al., BMC Genomics 18:901

(2017); Schneider et al., BMC Cancer. 11:102 (2011); Lissa et al., Transl Lung Cancer Res 5(5):492-504 (2016); Skvortsova et al., Br. J. Cancer. 94(10):1492-1495 (2006); Kim et al., Cancer Res. 61:3419-3424 (2001); Furonaka et al., Pathology International 55:303-309 (2005); Gomes et al., Rev. Port. Pneumol. 20:20-30 (2014); Kim et al., Oncogene. 20:1765-70 (2001); Hopkins-Donaldson et al., Cell Death Differ. 10:356-64 (2003); Kikuchi et al., Clin. Cancer Res. 11:2954-61 (2005); Heller et al., Oncogene 25:959-968 (2006); Licchesi et al., Carcinogenesis. 29:895-904 (2008); Guo et al., Clin. Cancer Res. 10:7917-24 (2004); Palmisano et al., Cancer Res. 63:4620-4625 (2003); and Toyooka et al., Cancer Res. 61:4556-4560, (2001).

An exemplary set of hypermethylation variable target regions comprising genes or portions thereof based on the lung cancer studies is provided in Table 2. Many of these genes likely have relevance to cancers beyond lung cancer; for example, Casp8 (Caspase 8) is a key enzyme in programmed cell death and hypermethylation-based inactivation of this gene may be a common oncogenic mechanism not limited to lung cancer. Additionally, a number of genes appear in both Tables 1 and 2, indicating generality.

**Table 2. Exemplary hypermethylation target regions (genes or portions thereof) based on lung cancer studies.**

| **Gene Name** | **Chromosome** |
|---|---|
| MARCH11 | chr5 |
| TAC1 | chr7 |
| TCF21 | chr6 |
| SHOX2 | chr3 |
| p16 | chr3 |
| Casp8 | chr2 |
| CDH13 | chr16 |
| MGMT | chr10 |
| MLH1 | chr3 |
| MSH2 | chr2 |
| TSLC1 | chr11 |
| APC | chr5 |
| DKK1 | chr10 |
| DKK3 | chr11 |
| LKB1 | chr11 |
| WIF1 | chr12 |
| RUNX3 | chr1 |
| GATA4 | chr8 |
| GATA5 | chr20 |
| PAX5 | chr9 |
| E-Cadherin | chr16 |
| H-Cadherin | chr16 |

Any of the foregoing embodiments concerning target regions identified in Table 2 may be combined with any of the embodiments described above concerning target regions identified in Table 1. In some embodiments, the hypermethylation variable target regions comprise a plurality of genes or portions thereof listed in Table 1 or Table 2, e.g., at least 10%, 20%, 30%, 40%, 50%, 60%, 70%, 80%, 90%, or 100% of the genes or portions thereof listed in Table 1 or Table 2.

Additional hypermethylation target regions may be obtained, e.g., from the Cancer Genome Atlas. Kang et al., Genome Biology 18:53 (2017), describe construction of a probabilistic method called Cancer Locator using hypermethylation target regions from breast, colon, kidney, liver, and lung. In some embodiments, the hypermethylation target regions can be specific to one or more types of cancer. Accordingly, in some embodiments, the hypermethylation target regions include one, two, three, four, or five subsets of hypermethylation target regions that collectively show hypermethylation in one, two, three, four, or five of breast, colon, kidney, liver, and lung cancers.

### ii. Hypomethylation variable target regions

Global hypomethylation is a commonly observed phenomenon in various cancers. See, e.g., Hon et al., Genome Res. 22:246-258 (2012) (breast cancer); Ehrlich, Epigenomics 1:239-259 (2009) (review article noting observations of hypomethylation in colon, ovarian, prostate, leukemia, hepatocellular, and cervical cancers). For example, regions such as repeated elements, e.g., LINE1 elements, Alu elements, centromeric tandem repeats, pericentromeric tandem repeats, and satellite DNA, and intergenic regions that are ordinarily methylated in healthy cells may show reduced methylation in tumor cells. Accordingly, in some embodiments, the epigenetic target region set includes hypomethylation variable target regions, where a decrease in the level of observed methylation indicates an increased likelihood that a sample (e.g., of cfDNA) contains DNA produced by neoplastic cells, such as tumor or cancer cells.

In some embodiments, hypomethylation variable target regions include repeated elements and/or intergenic regions. In some embodiments, repeated elements include one, two, three, four, or five of LINE1 elements, Alu elements, centromeric tandem repeats, pericentromeric tandem repeats, and/or satellite DNA.

Exemplary specific genomic regions that show cancer-associated hypomethylation include nucleotides 8403565-8953708 and 151104701-151106035 of human chromosome 1, e.g., according to the hg19 or hg38 human genome construct. In some embodiments, the hypomethylation variable target regions overlap or comprise one or both of these regions.

### iii. CTCF binding regions

CTCF is a DNA-binding protein that contributes to chromatin organization and often colocalizes with cohesin. Perturbation of CTCF binding sites has been reported in a variety of different cancers. *See, e.g.,* Katainen et al., Nature Genetics, doi:10.1038/ng.3335, published online 8 June 2015; Guo et al., Nat. Commun. 9:1520 (2018). CTCF binding results in recognizable patterns in cfDNA that can be detected by sequencing, e.g., through fragment length analysis. For example, details regarding sequencing-based fragment length analysis are provided in Snyder et al., Cell 164:57-68 (2016); WO 2018/009723; and US20170211143A1.

Thus, perturbations of CTCF binding result in variation in the fragmentation patterns of cfDNA. As such, CTCF binding sites represent a type of fragmentation variable target regions.

There are many known CTCF binding sites. See, e.g., the CTCFBSDB (CTCF Binding Site Database), available on the Internet at insulatordb.uthsc.edu/; Cuddapah et al., Genome Res. 19:24-32 (2009); Martin et al., Nat. Struct. Mol. Biol. 18:708-14 (2011); Rhee et al., Cell. 147:1408-19 (201). Exemplary CTCF binding sites are at nucleotides 56014955-56016161 on chromosome 8 and nucleotides 95359169-95360473 on chromosome 13, e.g., according to the hg19 or hg38 human genome construct.

Accordingly, in some embodiments, the epigenetic target region set includes CTCF binding regions. In some embodiments, the CTCF binding regions comprise at least 10, 20, 50, 100, 200, or 500 CTCF binding regions, or 10-20, 20-50, 50-100, 100-200, 200-500, or 500-1000 CTCF binding regions, e.g., such as CTCF binding regions described above or in one or more of CTCFBSDB or the Cuddapah et al., Martin et al., or Rhee et al. articles cited above.

In some embodiments, at least some of the CTCF sites can be methylated or unmethylated, wherein the methylation state is correlated with the whether or not the cell is a cancer cell. In some embodiments, the epigenetic target region set comprises at least 100 bp, at least 200 bp, at least 300 bp, at least 400 bp, at least 500 bp, at least 750 bp, at least 1000 bp upstream and/or downstream regions of the CTCF binding sites.

### iv. Transcription start sites

Transcription start sites may also show perturbations in neoplastic cells. For example, nucleosome organization at various transcription start sites in healthy cells of the hematopoietic lineage-which contributes substantially to cfDNA in healthy individuals-may differ from nucleosome organization at those transcription start sites in neoplastic cells. This results in different cfDNA patterns that can be detected by sequencing, for example, as discussed generally in Snyder et al., Cell 164:57-68 (2016); WO 2018/009723; and US20170211143A1.

Thus, perturbations of transcription start sites also result in variation in the fragmentation patterns of cfDNA. As such, transcription start sites also represent a type of fragmentation variable target regions.

Human transcriptional start sites are available from DBTSS (DataBase of Human Transcription Start Sites), available on the Internet at dbtss.hgc.jp and described in Yamashita et al., Nucleic Acids Res. 34(Database issue): D86-D89 (2006)

Accordingly, in some embodiments, the epigenetic target region set includes transcriptional start sites. In some embodiments, the transcriptional start sites comprise at least 10, 20, 50, 100, 200, or 500 transcriptional start sites, or 10-20, 20-50, 50-100, 100-200, 200-500, or 500-1000 transcriptional start sites, e.g., such as transcriptional start sites listed in DBTSS. In some embodiments, at least some of the transcription start sites can be methylated or unmethylated, wherein the methylation state is correlated with the whether or not the cell is a cancer cell. In some embodiments, the epigenetic target region set comprises at least 100 bp, at least 200 bp, at least 300 bp, at least 400 bp, at least 500 bp, at least 750 bp, at least 1000 bp upstream and/or downstream regions of the transcription start sites.

### v. Copy number variations; focal amplifications

Although copy number variations such as focal amplifications are somatic mutations, they can be detected by sequencing based on read frequency in a manner analogous to approaches for detecting certain epigenetic changes such as changes in methylation. As such, regions that may show copy number variations such as focal amplifications in cancer can be included in the epigenetic target region set and may comprise one or more of AR, BRAF, CCND1, CCND2, CCNE1, CDK4, CDK6, EGFR, ERBB2, FGFR1, FGFR2, KIT, KRAS, MET, MYC, PDGFRA, PIK3CA, and RAF1. For example, in some embodiments, the epigenetic target region set comprises at least 2, 3, 4, 5, 6, 7, 8, 9, 10, 11, 12, 13, 14, 15, 16, 17, or 18 of the foregoing targets.

### iv. Methylation control regions

It can be useful to include control regions to facilitate data validation. In some embodiments, the epigenetic target region set includes control regions that are expected to be methylated or unmethylated in essentially all samples, regardless of whether the DNA is derived from a cancer cell or a normal cell. In some embodiments, the epigenetic target region set includes control hypomethylated regions that are expected to be hypomethylated in essentially all samples. In some embodiments, the epigenetic target region set includes control hypermethylated regions that are expected to be hypermethylated in essentially all samples.

### b. Sequence-variable target region set

In some embodiments, the sequence-variable target region set comprises a plurality of regions known to undergo somatic mutations in cancer (referred to herein as cancer-associated mutations). Accordingly, methods may comprise determining whether cfDNA molecules corresponding to the sequence-variable target region set comprise cancer-associated mutations.

In some embodiments, the sequence-variable target region set targets a plurality of different genes or genomic regions ("panel") selected such that a determined proportion of subjects having a cancer exhibits a genetic variant or tumor marker in one or more different genes or genomic regions in the panel. The panel may be selected to limit a region for sequencing to a fixed number of base pairs. The panel may be selected to sequence a desired amount of DNA, e.g., by adjusting the affinity and/or amount of the probes as described elsewhere herein. The panel may be further selected to achieve a desired sequence read depth. The panel may be selected to achieve a desired sequence read depth or sequence read coverage for an amount of sequenced base pairs. The panel may be selected to achieve a theoretical sensitivity, a theoretical specificity, and/or a theoretical accuracy for detecting one or more genetic variants in a sample.

Probes for detecting the panel of regions can include those for detecting genomic regions of interest (hotspot regions) as well as nucleosome-aware probes (e.g., KRAS codons 12 and 13) and may be designed to optimize capture based on analysis of cfDNA coverage and fragment size variation impacted by nucleosome binding patterns and GC sequence composition. Regions used herein can also include non-hotspot regions optimized based on nucleosome positions and GC models.

Examples of listings of genomic locations of interest may be found in Table 3 and Table 4. In some embodiments, a sequence-variable target region set used in the methods of the present disclosure comprises at least a portion of at least 5, at least 10, at least 15, at least 20, at least 25, at least 30, at least 35, at least 40, at least 45, at least 50, at least 55, at least 60, at least 65, or 70 of the genes of Table 3. In some embodiments, a sequence-variable target region set used in the methods of the present disclosure comprises at least 5, at least 10, at least 15, at least 20, at least 25, at least 30, at least 35, at least 40, at least 45, at least 50, at least 55, at least 60, at least 65, or 70 of the SNVs of Table 3. In some embodiments, a sequence-variable target region set used in the methods of the present disclosure comprises at least 1, at least 2, at least 3, at least 4, at least 5, or 6 of the fusions of Table 3. In some embodiments, a sequence-variable target region set used in the methods of the present disclosure comprise at least a portion of at least 1, at least 2, or 3 of the indels of Table 3. In some embodiments, a sequence-variable target region set used in the methods of the present disclosure comprises at least a portion of at least 5, at least 10, at least 15, at least 20, at least 25, at least 30, at least 35, at least 40, at least 45, at least 50, at least 55, at least 60, at least 65, at least 70, or 73 of the genes of Table 4. In some embodiments, a sequence-variable target region set used in the methods of the present disclosure comprises at least 5, at least 10, at least 15, at least 20, at least 25, at least 30, at least 35, at least 40, at least 45, at least 50, at least 55, at least 60, at least 65, at least 70, or 73 of the SNVs of Table 4. In some embodiments, a sequence-variable target region set used in the methods of the present disclosure comprises at least 1, at least 2, at least 3, at least 4, at least 5, or 6 of the fusions of Table 4. In some embodiments, a sequence-variable target region set used in the methods of the present disclosure comprises at least a portion of at least 1, at least 2, at least 3, at least 4, at least 5, at least 6, at least 7, at least 8, at least 9, at least 10, at least 11, at least 12, at least 13, at least 14, at least 15, at least 16, at least 17, or 18 of the indels of Table 4. Each of these genomic locations of interest may be identified as a backbone region or hot-spot region for a given panel. An example of a listing of hot-spot genomic locations of interest may be found in Table 5. The coordinates in Table 5 are based on the hg19 assembly of the human genome, but one skilled in the art will be familiar with other assemblies and can identify coordinate sets corresponding to the indicated exons, introns, codons, etc. in an assembly of their choice. In some embodiments, a sequence-variable target region set used in the methods of the present disclosure comprises at least a portion of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or at least 20 of the genes of Table 5. Each hot-spot genomic region is listed with several characteristics, including the associated gene, chromosome on which it resides, the start and stop position of the genome representing the gene's locus, the length of the gene's locus in base pairs, the exons covered by the gene, and the critical feature (e.g., type of mutation) that a given genomic region of interest may seek to capture.

**Table 3**

| **Point Mutations (SNVs) and Indels** | | | | | | **Fusions** |
|---|---|---|---|---|---|---|
| AKT1 | ALK | APC | AR | ARAF | ARID1A | ALK |
| ATM | BRAF | BRCA1 | BRCA2 | CCND1 | CCND2 | FGFR2 |
| CCNE1 | CDH1 | CDK4 | CDK6 | CDKN2A | CDKN2B | FGFR3 |
| CTNNB1 | EGFR | ERBB2 | ESR1 | EZH2 | FBXW7 | NTRK1 |
| FGFR1 | FGFR2 | FGFR3 | GATA3 | GNA11 | GNAQ | RET |
| GNAS | HNF1A | HRAS | IDH1 | IDH2 | JAK2 | ROS1 |
| JAK3 | KIT | KRAS | MAP2K1 | MAP2K2 | MET | |
| MLH1 | MPL | MYC | NF1 | NFE2L2 | NOTCH 1 | |
| NPM1 | NRAS | NTRK1 | PDGFRA | PIK3CA | PTEN | |
| PTPN 11 | RAF1 | RB1 | RET | RHEB | RHOA | |
| RIT1 | ROS1 | SMAD4 | SMO | SRC | STK11 | |
| TERT | TP53 | TSC1 | VHL | | | |

**Table 4**

| **Point Mutations (SNVs) and Indels** | | | | | | **Fusions** |
|---|---|---|---|---|---|---|
| AKT1 | ALK | APC | AR | ARAF | ARIDIA | ALK |
| ATM | BRAF | BRCA1 | BRCA2 | CCND1 | CCND2 | FGFR2 |
| CCNE1 | CDH1 | CDK4 | CDK6 | CDKN2A | DDR2 | FGFR3 |
| CTNNB1 | EGFR | ERBB2 | ESR1 | EZH2 | FBXW7 | NTRK1 |
| FGFR1 | FGFR2 | FGFR3 | GATA3 | GNA11 | GNAQ | RET |
| GNAS | HNF1A | HRAS | IDH1 | IDH2 | JAK2 | ROS1 |
| JAK3 | KIT | KRAS | MAP2K1 | MAP2K2 | MET | |
| MLH1 | MPL | MYC | NF1 | NFE2L2 | NOTCH1 | |
| NPM1 | NRAS | NTRK1 | PDGFRA | PIK3CA | PTEN | |
| PTPN 11 | RAF1 | RB1 | RET | RHEB | RHOA | |
| RIT1 | ROS1 | SMAD4 | SMO | MAPK1 | STK11 | |
| TERT | TP53 | TSC1 | VHL | MAPK3 | MTOR | |
| NTRK3 | | | | | | |

**Table 5**

| Gene | **Chromosome** | **Start Position** | **Stop Position** | **Length (bp)** | **Exons/ Introns Covered** | **Feature** |
|---|---|---|---|---|---|---|
| ALK | chr2 | 29446405 | 29446655 | 250 | intron 19 | Fusion |
| ALK | chr2 | 29446062 | 29446197 | 135 | intron 20 | Fusion |
| ALK | chr2 | 29446198 | 29446404 | 206 | exon 20 | Fusion |
| ALK | chr2 | 29447353 | 29447473 | 120 | intron 19 | Fusion |
| ALK | chr2 | 29447614 | 29448316 | 702 | intron 19 | Fusion |
| ALK | chr2 | 29448317 | 29448441 | 124 | exon 19 | Fusion |
| ALK | chr2 | 29449366 | 29449777 | 411 | intron 18 | Fusion |
| ALK | chr2 | 29449778 | 29449950 | 172 | exon 18 | Fusion |
| BRAF | chr7 | 140453064 | 140453203 | 139 | exon 15 | BRAF V600 |
| CTNNB1 | chr3 | 41266007 | 41266254 | 247 | exon 3 | S37 |
| EGFR | chr7 | 55240528 | 55240827 | 299 | exons 18 and 19 | G719 and deletions |
| EGFR | chr7 | 55241603 | 55241746 | 143 | exon 20 | Insertions/T790M |
| EGFR | chr7 | 55242404 | 55242523 | 119 | exon 21 | L858R |
| ERBB2 | chr17 | 37880952 | 37881174 | 222 | exon 20 | Insertions |
| ESR1 | chr6 | 152419857 | 152420111 | 254 | exon 10 | V534, P535, L536, Y537, D538 |
| FGFR2 | chr10 | 123279482 | 123279693 | 211 | exon 6 | S252 |
| GATA3 | chr10 | 8111426 | 8111571 | 145 | exon 5 | SS / Indels |
| GATA3 | chr10 | 8115692 | 8116002 | 310 | exon 6 | SS / Indels |
| GNAS | chr20 | 57484395 | 57484488 | 93 | exon 8 | R844 |
| IDH1 | chr2 | 209113083 | 209113394 | 311 | exon 4 | R132 |
| IDH2 | chr15 | 90631809 | 90631989 | 180 | exon 4 | R140, R172 |
| KIT | chr4 | 55524171 | 55524258 | 87 | exon 1 | |
| KIT | chr4 | 55561667 | 55561957 | 290 | exon 2 | |
| KIT | chr4 | 55564439 | 55564741 | 302 | exon 3 | |
| KIT | chr4 | 55565785 | 55565942 | 157 | exon 4 | |
| KIT | chr4 | 55569879 | 55570068 | 189 | exon 5 | |
| KIT | chr4 | 55573253 | 55573463 | 210 | exon 6 | |
| KIT | chr4 | 55575579 | 55575719 | 140 | exon 7 | |
| KIT | chr4 | 55589739 | 55589874 | 135 | exon 8 | |
| KIT | chr4 | 55592012 | 55592226 | 214 | exon 9 | |
| KIT | chr4 | 55593373 | 55593718 | 345 | exons 10 and 11 | 557, 559, 560, 576 |
| KIT | chr4 | 55593978 | 55594297 | 319 | exons 12 and 13 | V654 |
| KIT | chr4 | 55595490 | 55595661 | 171 | exon 14 | T670, S709 |
| KIT | chr4 | 55597483 | 55597595 | 112 | exon 15 | D716 |
| KIT | chr4 | 55598026 | 55598174 | 148 | exon 16 | L783 |
| KIT | chr4 | 55599225 | 55599368 | 143 | exon 17 | C809, R815, D816, L818, D820, S821F, N822, Y823 |
| KIT | chr4 | 55602653 | 55602785 | 132 | exon 18 | A829P |
| KIT | chr4 | 55602876 | 55602996 | 120 | exon 19 | |
| KIT | chr4 | 55603330 | 55603456 | 126 | exon 20 | |
| KIT | chr4 | 55604584 | 55604733 | 149 | exon 21 | |
| KRAS | chr12 | 25378537 | 25378717 | 180 | exon 4 | A146 |
| KRAS | chr12 | 25380157 | 25380356 | 199 | exon 3 | Q61 |
| KRAS | chr12 | 25398197 | 25398328 | 131 | exon 2 | G12/G13 |
| MET | chr7 | 116411535 | 116412255 | 720 | exon 13, exon 14, intron 13, intron 14 | MET exon 14 SS |
| NRAS | chr1 | 115256410 | 115256609 | 199 | exon 3 | Q61 |
| NRAS | chr1 | 115258660 | 115258791 | 131 | exon 2 | G12/G13 |
| PIK3CA | chr3 | 178935987 | 178936132 | 145 | exon 10 | E545K |
| PIK3CA | chr3 | 178951871 | 178952162 | 291 | exon 21 | H1047R |
| PTEN | chr10 | 89692759 | 89693018 | 259 | exon 5 | R130 |
| SMAD4 | chr18 | 48604616 | 48604849 | 233 | exon 12 | D537 |
| TERT | chr5 | 1294841 | 1295512 | 671 | promoter | chr5:1295228 |
| TP53 | chr17 | 7573916 | 7574043 | 127 | exon 11 | Q331, R337, R342 |
| TP53 | chr17 | 7577008 | 7577165 | 157 | exon 8 | R273 |
| TP53 | chr17 | 7577488 | 7577618 | 130 | exon 7 | R248 |
| TP53 | chr17 | 7578127 | 7578299 | 172 | exon 6 | R213/Y220 |
| TP53 | chr17 | 7578360 | 7578564 | 204 | exon 5 | R175 / Deletions |
| TP53 | chr17 | 7579301 | 7579600 | 299 | exon 4 | |
| | | | | 12574 (total target region) | | |
| | | | | 16330 (total probe coverage) | | |

Additionally, or alternatively, suitable target region sets are available from the literature. For example, Gale et al., PLoS One 13: e0194630 (2018), describes a panel of 35 cancer-related gene targets that can be used as part or all of a sequence-variable target region set. These 35 targets are AKT1, ALK, BRAF, CCND1, CDK2A, CTNNB1, EGFR, ERBB2, ESR1, FGFR1, FGFR2, FGFR3, FOXL2, GATA3, GNA11, GNAQ, GNAS, HRAS, IDH1, IDH2, KIT, KRAS, MED12, MET, MYC, NFE2L2, NRAS, PDGFRA, PIK3CA, PPP2R1A, PTEN, RET, STK11, TP53, and U2AF1.

In some embodiments, the sequence-variable target region set comprises target regions from at least 10, 20, 30, or 35 cancer-related genes, such as the cancer-related genes listed above.

### E. Sequencing

Sample nucleic acids, optionally flanked by adapters, with or without prior amplification are generally subjected to sequencing. Sequencing methods or commercially available formats that are optionally utilized include, for example, Sanger sequencing, high-throughput sequencing, pyrosequencing, sequencing-by-synthesis, single-molecule sequencing, nanopore-based sequencing, semiconductor sequencing, sequencing-by-ligation, sequencing-by-hybridization, RNA-Seq (Illumina), Digital Gene Expression (Helicos), next generation sequencing (NGS), Single Molecule Sequencing by Synthesis (SMSS) (Helicos), massively-parallel sequencing, Clonal Single Molecule Array (Solexa), shotgun sequencing, Ion Torrent, Oxford Nanopore, Roche Genia, Maxim-Gilbert sequencing, primer walking, sequencing using PacBio, SOLiD, Ion Torrent, or Nanopore platforms. Sequencing reactions can be performed in a variety of sample processing units, which may include multiple lanes, multiple channels, multiple wells, or other means of processing multiple sample sets substantially simultaneously. Sample processing units can also include multiple sample chambers to enable the processing of multiple runs simultaneously.

The sequencing reactions can be performed on one or more nucleic acid fragment types or regions known to contain markers of cancer or of other diseases. The sequencing reactions can also be performed on any nucleic acid fragment present in the sample. The sequence reactions may be performed on at least about 5%, 10%, 15%, 20%, 25%, 30%, 40%, 50%, 60%, 70%, 80%, 90%, 95%, 99%, 99.9%, or 100% of the genome. In other cases, sequence reactions may be performed on less than about 5%, 10%, 15%, 20%, 25%, 30%, 40%, 50%, 60%, 70%, 80%, 90%, 95%, 99%, 99.9%, or 100% of the genome.

Simultaneous sequencing reactions may be performed using multiplex sequencing techniques. In some embodiments, cell-free polynucleotides are sequenced with at least about 1000, 2000, 3000, 4000, 5000, 6000, 7000, 8000, 9000, 10000, 50000, or 100,000 sequencing reactions. In other embodiments, cell-free polynucleotides are sequenced with less than about 1000, 2000, 3000, 4000, 5000, 6000, 7000, 8000, 9000, 10000, 50000, or 100,000 sequencing reactions. Sequencing reactions are typically performed sequentially or simultaneously. Subsequent data analysis is generally performed on all or part of the sequencing reactions. In some embodiments, data analysis is performed on at least about 1000, 2000, 3000, 4000, 5000, 6000, 7000, 8000, 9000, 10000, 50000, or 100,000 sequencing reactions. In other embodiments, data analysis may be performed on less than about 1000, 2000, 3000, 4000, 5000, 6000, 7000, 8000, 9000, 10000, 50000, or 100,000 sequencing reactions. An example of a read depth is from about 1000 to about 50000 reads per locus (e.g., base position).

### F. Analysis

Sequencing may generate a plurality of sequencing reads or reads. Sequencing reads or reads may include sequences of nucleotide data less than about 150 bases in length, or less than about 90 bases in length. In some embodiments, reads are between about 80 bases and about 90 bases, e.g., about 85 bases in length. In some embodiments, methods of the present disclosure are applied to very short reads, e.g., less than about 50 bases or about 30 bases in length. Sequencing read data can include the sequence data as well as meta information. Sequence read data can be stored in any suitable file format including, for example, VCF files, FASTA files, or FASTQ files.

FASTA may refer to a computer program for searching sequence databases, and the name FASTA may also refer to a standard file format. For example, FASTA is described by, for example, Pearson & Lipman, 1988, Improved tools for biological sequence comparison, PNAS 85 2444-2448. A sequence in FASTA format begins with a single-line description, followed by lines of sequence data. The description line is distinguished from the sequence data by a greater-than (">") symbol in the first column. The word following the ">" symbol is the identifier of the sequence, and the rest of the line is the description (both are optional). There should be no space between the ">" and the first letter of the identifier. It is recommended that all lines of text be shorter than 80 characters. The sequence ends if another line starting with a ">" appears; this indicates the start of another sequence.

The FASTQ format is a text-based format for storing both a biological sequence (usually nucleotide sequence) and its corresponding quality scores. It is similar to the FASTA format but with quality scores following the sequence data. Both the sequence letter and quality score are encoded with a single ASCII character for brevity. The FASTQ format is a *de facto* standard for storing the output of high throughput sequencing instruments such as the Illumina Genome Analyzer, as described by, for example, Cock et al. ("The Sanger FASTQ file format for sequences with quality scores, and the Solexa/Illumina FASTQ variants," Nucleic Acids Res 38(6):1767-1771, 2009).

For FASTA and FASTQ files, meta information includes the description line and not the lines of sequence data. In some embodiments, for FASTQ files, the meta information includes the quality scores. For FASTA and FASTQ files, the sequence data begins after the description line and is present typically using some subset of IUPAC ambiguity codes optionally with "-". In an embodiment, the sequence data may use the A, T, C, G, and N characters, optionally including "-" or U as-needed (e.g., to represent gaps or uracil).

In some embodiments, the at least one master sequence read file and the output file are stored as plain text files (e.g., using encoding such as ASCII; ISO/IEC 646; EBCDIC; UTF-8; or UTF-16). A computer system provided by the present disclosure may include a text editor program capable of opening the plain text files. A text editor program may refer to a computer program capable of presenting contents of a text file (such as a plain text file) on a computer screen, allowing a human to edit the text (e.g., using a monitor, keyboard, and mouse). Examples of text editors include, without limitation, Microsoft Word, emacs, pico, vi, BBEdit, and TextWrangler. The text editor program may be capable of displaying the plain text files on a computer screen, showing the meta information and the sequence reads in a human-readable format (e.g., not binary encoded but instead using alphanumeric characters as they may be used in print or human writing).

While methods have been discussed with reference to FASTA or FASTQ files, methods and systems of the present disclosure may be used to compress any suitable sequence file format including, for example, files in the Variant Call Format (VCF) format. A typical VCF file may include a header section and a data section. The header contains an arbitrary number of meta-information lines, each starting with characters '##', and a TAB delimited field definition line starting with a single '#' character. The field definition line names eight mandatory columns and the body section contains lines of data populating the columns defined by the field definition line. The VCF format is described by, for example, Danecek et al. ("The variant call format and VCF tools," Bioinformatics 27(15):2156-2158, 2011), The header section may be treated as the meta information to write to the compressed files and the data section may be treated as the lines, each of which will be stored in a master file only if unique.

Some embodiments provide for the assembly of sequencing reads. In assembly by alignment, for example, the sequencing reads are aligned to each other or aligned to a reference sequence. By aligning each read, in turn to a reference genome, all of the reads are positioned in relationship to each other to create the assembly. In addition, aligning or mapping the sequencing read to a reference sequence can also be used to identify variant sequences within the sequencing read. Identifying variant sequences can be used in combination with the methods and systems described herein to further aid in the diagnosis or prognosis of a disease or condition, or for guiding treatment decisions.

In some embodiments, any or all of the steps are automated. Alternatively, methods of the present disclosure may be embodied wholly or partially in one or more dedicated programs, for example, each optionally written in a compiled language such as C++, then compiled and distributed as a binary. Methods of the present disclosure may be implemented wholly or in part as modules within, or by invoking functionality within, existing sequence analysis platforms. In some embodiments, methods of the present disclosure include a number of steps that are all invoked automatically responsive to a single starting cue (e.g., one or a combination of triggering events sourced from human activity, another computer program, or a machine). Thus, the present disclosure provides methods in which any or the steps or any combination of the steps can occur automatically responsive to a cue. "Automatically" generally means without intervening human input, influence, or interaction (e.g., responsive only to original or pre-cue human activity).

The methods of the present disclosure may also encompass various forms of output, which includes an accurate and sensitive interpretation of a subject's nucleic acid sample. The output of retrieval can be provided in the format of a computer file. In some embodiments, the output is a FASTA file, a FASTQ file, or a VCF file. The output may be processed to produce a text file, or an XML file containing sequence data such as a sequence of the nucleic acid aligned to a sequence of the reference genome. In other embodiments, processing yields output containing coordinates or a string describing one or more mutations in the subject nucleic acid relative to the reference genome. Alignment strings may include Simple UnGapped Alignment Report (SUGAR), Verbose Useful Labeled Gapped Alignment Report (VULGAR), and Compact Idiosyncratic Gapped Alignment Report (CIGAR) (as described by, for example, Ning et al., Genome Research 11(10):1725-9, 200:). These strings may be implemented, for example, in the Exonerate sequence alignment software from the European Bioinformatics Institute (Hinxton, UK).

In some embodiments, a sequence alignment is produced-such as, for example, a sequence alignment map (SAM) or binary alignment map (BAM) file-comprising a CIGAR string (the SAM format is described, e.g., by Li et al., "The Sequence Alignment/Map format and SAMtools," Bioinformatics, 25(16):2078-9, 2009). In some embodiments, CIGAR displays or includes gapped alignments one-per-line. CIGAR is a compressed pairwise alignment format reported as a CIGAR string. A CIGAR string may be useful for representing long (e.g., genomic) pairwise alignments. A CIGAR string may be used in SAM format to represent alignments of reads to a reference genome sequence.

A CIGAR string may follow an established motif. Each character is preceded by a number, giving the base counts of the event. Characters used can include M, I, D, N, and S (M=match; I=insertion; D=deletion; N=gap; S=substitution). The CIGAR string defines the sequence of matches/mismatches and deletions (or gaps). For example, the CIGAR string 2MD3M2D2M may indicate that the alignment contains 2 matches, 1 deletion (number 1 is omitted in order to save some space), 3 matches, 2 deletions, and 2 matches.

In some embodiments, a nucleic acid population is prepared for sequencing by enzymatically forming blunt-ends on double-stranded nucleic acids with single-stranded overhangs at one or both ends. In these embodiments, the population is typically treated with an enzyme having a 5'-3' DNA polymerase activity and a 3'-5' exonuclease activity in the presence of the nucleotides (e.g., A, C, G, and T or U). Examples of enzymes or catalytic fragments thereof that may be optionally used include Klenow large fragment and T4 polymerase. At 5' overhangs, the enzyme typically extends the recessed 3' end on the opposing strand until it is flush with the 5' end to produce a blunt end. At 3' overhangs, the enzyme generally digests from the 3' end up to and sometimes beyond the 5' end of the opposing strand. If this digestion proceeds beyond the 5' end of the opposing strand, the gap can be filled in by an enzyme having the same polymerase activity that is used for 5' overhangs. The formation of blunt ends on double-stranded nucleic acids facilitates, for example, the attachment of adapters and subsequent amplification.

In some embodiments, nucleic acid populations are subjected to additional processing, such as the conversion of single-stranded nucleic acids to double-stranded nucleic acids and/or conversion of RNA to DNA (e.g., complementary DNA or cDNA). These forms of nucleic acid are also optionally linked to adapters and amplified.

With or without prior amplification, nucleic acids subject to the process of forming blunt-ends described above, and optionally other nucleic acids in a sample, can be sequenced to produce sequenced nucleic acids. A sequenced nucleic acid can refer either to the sequence of a nucleic acid (e.g., sequence information) or a nucleic acid whose sequence has been determined. Sequencing can be performed so as to provide sequence data of individual nucleic acid molecules in a sample either directly or indirectly from a consensus sequence of amplification products of an individual nucleic acid molecule in the sample.

In some embodiments, double-stranded nucleic acids with single-stranded overhangs in a sample after blunt-end formation are linked at both ends to adapters including barcodes, and the sequencing determines nucleic acid sequences as well as in-line barcodes introduced by the adapters. The blunt-end DNA molecules are optionally ligated to a blunt end of an at least partially double-stranded adapter (e.g., a Y-shaped or bell-shaped adapter). Alternatively, blunt ends of sample nucleic acids and adapters can be tailed with complementary nucleotides to facilitate ligation (for e.g., sticky-end ligation).

The nucleic acid sample is typically contacted with a sufficient number of adapters that there is a low probability (e.g., less than about 1 or 0.1 %) that any two copies of the same nucleic acid receive the same combination of adapter barcodes from the adapters linked at both ends. The use of adapters in this manner may permit identification of families of nucleic acid sequences with the same start and stop points on a reference nucleic acid and linked to the same combination of barcodes. Such a family may represent sequences of amplification products of a nucleic acid in the sample before amplification. The sequences of family members can be compiled to derive consensus nucleotide(s) or a complete consensus sequence for a nucleic acid molecule in the original sample, as modified by blunt-end formation and adapter attachment. In other words, the nucleotide occupying a specified position of a nucleic acid in the sample can be determined to be the consensus of nucleotides occupying that corresponding position in family member sequences. Families can include sequences of one or both strands of a double-stranded nucleic acid. If members of a family include sequences of both strands from a double-stranded nucleic acid, sequences of one strand may be converted to their complements for purposes of compiling sequences to derive consensus nucleotide(s) or sequences. Some families include only a single member sequence. In this case, this sequence can be taken as the sequence of a nucleic acid in the sample before amplification. Alternatively, families with only a single member sequence can be eliminated from subsequent analysis.

Nucleotide variations (e.g., SNVs or indels) in sequenced nucleic acids can be determined by comparing sequenced nucleic acids with a reference sequence. The reference sequence is often a known sequence, e.g., a known whole or partial genome sequence from a subject (e.g., a whole genome sequence of a human subject). The reference sequence can be, for example, hG19 or hG38. The sequenced nucleic acids can represent sequences determined directly for a nucleic acid in a sample, or a consensus of sequences of amplification products of such a nucleic acid, as described above. A comparison can be performed at one or more designated positions on a reference sequence. A subset of sequenced nucleic acids can be identified including a position corresponding with a designated position of the reference sequence when the respective sequences are maximally aligned. Within such a subset it can be determined which, if any, sequenced nucleic acids include a nucleotide variation at the designated position, and optionally which if any, include a reference nucleotide (e.g., same as in the reference sequence). If the number of sequenced nucleic acids in the subset including a nucleotide variant exceeding a selected threshold, then a variant nucleotide can be called at the designated position. The threshold can be a simple number, such as at least 1, 2, 3, 4, 5, 6, 7, 8, 9, or 10 sequenced nucleic acids within the subset including the nucleotide variant or it can be a ratio, such as at least 0.5, 1, 2, 3, 4, 5, 10, 15, or 20, of sequenced nucleic acids within the subset that include the nucleotide variant, among other possibilities. The comparison can be repeated for any designated position of interest in the reference sequence. Sometimes a comparison can be performed for designated positions occupying at least about 20, 100, 200, or 300 contiguous positions on a reference sequence, e.g., about 20-500, or about 50-300 contiguous positions.

Additional details regarding nucleic acid sequencing, including the formats and applications described herein, are also provided in, for example, Levy et al., Annual Review of Genomics and Human Genetics, 17: 95-115 (2016), Liu et al., J. of Biomedicine and Biotechnology, Volume 2012, Article ID 251364:1-11 (2012), Voelkerding et al., Clinical Chem., 55: 641-658 (2009), MacLean et al., Nature Rev. Microbiol., 7: 287-296 (2009), Astier et al., J Am Chem Soc., 128(5):1705-10 (2006), U.S. Pat. No. 6,210,891, U.S. Pat. No. 6,258,568, U.S. Pat. No. 6,833,246, U.S. Pat. No. 7,115,400, U.S. Pat. No. 6,969,488, U.S. Pat. No. 5,912,148, U.S. Pat. No. 6,130,073, U.S. Pat. No. 7,169,560, U.S. Pat. No. 7,282,337, U.S. Pat. No. 7,482,120, U.S. Pat. No. 7,501,245, U.S. Pat. No. 6,818,395, U.S. Pat. No. 6,911,345, U.S. Pat. No. 7,501,245, U.S. Pat. No. 7,329,492, U.S. Pat. No. 7,170,050, U.S. Pat. No. 7,302,146, U.S. Pat. No. 7,313,308, and U.S. Pat. No. 7,476,503.

### IV. Computer Systems (not claimed)

Methods of the present disclosure can be implemented using, or with the aid of, computer systems. For example, such methods may comprise: (i) adding a set of carrier nucleic acid molecules to the polynucleotide sample to generate a first sample; wherein the set of carrier nucleic acid molecules comprises: (a) at least one subset of unmethylated carrier nucleic acid molecules; and/or (b) at least one subset of methylated carrier nucleic acid molecules, wherein at least one end of the carrier nucleic acid molecules is modified to prevent ligation, wherein the unmethylated carrier nucleic acid molecule does not comprise methylated nucleotides and the methylated carrier nucleic acid molecule comprises one or more methylated nucleotides; (ii) partitioning the first sample into at least two partitioned sets using capturing agent that binds selectively to methylated polynucleotides, thereby generating a partitioned sample;(iii) processing at least a portion of the partitioned sample to generate processed sample, wherein the processing comprises at least one of the following: (a) tagging, (b) amplifying and (c) enriching molecules for specific regions of interest; (iv) sequencing at least a portion of the processed sample to generate a set of sequencing reads; and (v) analyzing at least a portion of the set of sequencing reads to detect the presence or absence of tumor. In this embodiment, the system comprises components for adding carrier nucleic acid molecules, partitioning, tagging, amplifying, enriching and sequencing.

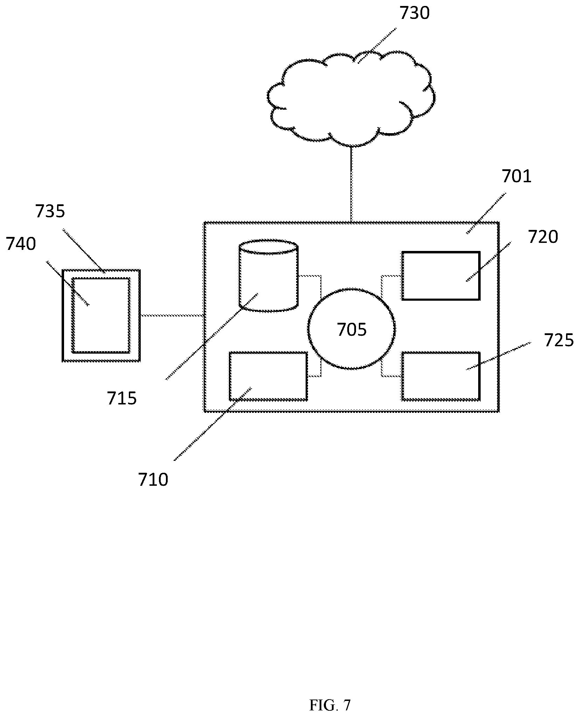

FIG. 7 shows a computer system 701 that is programmed or otherwise configured to implement the methods of the present disclosure. The computer system 701 can regulate various aspects sample preparation, sequencing, and/or analysis. In some examples, the computer system 701 is configured to perform sample preparation and sample analysis, including nucleic acid sequencing.

The computer system 701 includes a central processing unit (CPU, also "processor" and "computer processor" herein) 705, which can be a single core or multi core processor, or a plurality of processors for parallel processing. The computer system 701 also includes memory or memory location 710 (e.g., random-access memory, read-only memory, flash memory), electronic storage unit 715 (e.g., hard disk), communication interface 720 (e.g., network adapter) for communicating with one or more other systems, and peripheral devices 725, such as cache, other memory, data storage, and/or electronic display adapters. The memory 710, storage unit 715, interface 720, and peripheral devices 725 are in communication with the CPU 705 through a communication network or bus (solid lines), such as a motherboard. The storage unit 715 can be a data storage unit (or data repository) for storing data. The computer system 701 can be operatively coupled to a computer network 730 with the aid of the communication interface 720. The computer network 730 can be the Internet, an internet and/or extranet, or an intranet and/or extranet that is in communication with the Internet. The computer network 730 in some cases is a telecommunication and/or data network. The computer network 730 can include one or more computer servers, which can enable distributed computing, such as cloud computing. The computer network 730, in some cases with the aid of the computer system 701, can implement a peer-to-peer network, which may enable devices coupled to the computer system 701 to behave as a client or a server.

The CPU 705 can execute a sequence of machine-readable instructions, which can be embodied in a program or software. The instructions may be stored in a memory location, such as the memory 710. Examples of operations performed by the CPU 705 can include fetch, decode, execute, and writeback.

The storage unit 715 can store files, such as drivers, libraries, and saved programs. The storage unit 715 can store programs generated by users and recorded sessions, as well as output(s) associated with the programs. The storage unit 715 can store user data, e.g., user preferences and user programs. The computer system 701 in some cases can include one or more additional data storage units that are external to the computer system 701, such as located on a remote server that is in communication with the computer system 701 through an intranet or the Internet. Data may be transferred from one location to another using, for example, a communication network or physical data transfer (e.g., using a hard drive, thumb drive, or other data storage mechanism).

The computer system 701 can communicate with one or more remote computer systems through the network 730. For embodiment, the computer system 701 can communicate with a remote computer system of a user (e.g., operator). Examples of remote computer systems include personal computers (e.g., portable PC), slate or tablet PC's (e.g., Apple^{®} iPad, Samsung^{®} Galaxy Tab), telephones, Smart phones (e.g., Apple^{®} iPhone, Android-enabled device, Blackberry^{®}), or personal digital assistants. The user can access the computer system 701 via the network 730.

Methods as described herein can be implemented by way of machine (e.g., computer processor) executable code stored on an electronic storage location of the computer system 701, such as, for example, on the memory 710 or electronic storage unit 715. The machine executable or machine-readable code can be provided in the form of software. During use, the code can be executed by the processor 705. In some cases, the code can be retrieved from the storage unit 715 and stored on the memory 710 for ready access by the processor 705. In some situations, the electronic storage unit 715 can be precluded, and machine-executable instructions are stored on memory 710.

In an aspect, the present disclosure provides a non-transitory computer-readable medium comprising computer-executable instructions which, when executed by at least one electronic processor, perform a method comprising: (i) adding a set of carrier nucleic acid molecules to the polynucleotide sample to generate a first sample; wherein the set of carrier nucleic acid molecules comprises: (a) at least one subset of unmethylated carrier nucleic acid molecules; and/or (b) at least one subset of methylated carrier nucleic acid molecules, wherein at least one end of the carrier nucleic acid molecules is modified to prevent ligation, wherein the unmethylated carrier nucleic acid molecule does not comprise methylated nucleotides and the methylated carrier nucleic acid molecule comprises one or more methylated nucleotides; (ii) partitioning the first sample into at least two partitioned sets using capturing agent that binds selectively to methylated polynucleotides, thereby generating a partitioned sample;(iii) processing at least a portion of the partitioned sample to generate processed sample, wherein the processing comprises at least one of the following: (a) tagging, (b) amplifying and (c) enriching molecules for specific regions of interest; (iv)sequencing at least a portion of the processed sample to generate a set of sequencing reads; and (v) analyzing at least a portion of the set of sequencing reads to detect the presence or absence of tumor. In this embodiment, the computer readable medium comprises computer-executable instructions required for adding carrier nucleic acid molecules, partitioning, tagging, amplifying, enriching and sequencing.

The code can be pre-compiled and configured for use with a machine have a processer adapted to execute the code or can be compiled during runtime. The code can be supplied in a programming language that can be selected to enable the code to execute in a pre-compiled or as-compiled fashion.

Aspects of the systems and methods provided herein, such as the computer system 701, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming.

All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical, and electromagnetic waves, such as those used across physical interfaces between local devices, through wired and optical landline networks, and over various air-links. The physical elements that carry such waves, such as wired or wireless links, optical links, or the like, also may be considered as media bearing the software. As used herein, unless restricted to non-transitory, tangible "storage" media, terms such as computer or machine "readable medium" refer to any medium that participates in providing instructions to a processor for execution.

Hence, a machine-readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards, paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 701 can include or be in communication with an electronic display that comprises a user interface (UI) for providing, for example, one or more results of sample analysis. Examples of UIs include, without limitation, a graphical user interface (GUI) and web-based user interface.

Additional details relating to computer systems and networks, databases, and computer program products are also provided in, for example, Peterson, Computer Networks: A Systems Approach, Morgan Kaufmann, 5th Ed. (2011), Kurose, Computer Networking: A Top-Down Approach, Pearson, 7th Ed. (2016), Elmasri, Fundamentals of Database Systems, Addison Wesley, 6th Ed. (2010), Coronel, Database Systems: Design, Implementation, & Management, Cengage Learning, 11th Ed. (2014), Tucker, Programming Languages, McGraw-Hill Science/Engineering/Math, 2nd Ed. (2006), and Rhoton, Cloud Computing Architected: Solution Design Handbook, Recursive Press (201:).

### V. Applications

### A. Cancer and Other Diseases

In some embodiments, the disease under consideration is a type of cancer. Non-limiting examples of such cancers include biliary tract cancer, bladder cancer, transitional cell carcinoma, urothelial carcinoma, brain cancer, gliomas, astrocytomas, breast carcinoma, metaplastic carcinoma, cervical cancer, cervical squamous cell carcinoma, rectal cancer, colorectal carcinoma, colon cancer, hereditary nonpolyposis colorectal cancer, colorectal adenocarcinomas, gastrointestinal stromal tumors (GISTs), endometrial carcinoma, endometrial stromal sarcomas, esophageal cancer, esophageal squamous cell carcinoma, esophageal adenocarcinoma, ocular melanoma, uveal melanoma, gallbladder carcinomas, gallbladder adenocarcinoma, renal cell carcinoma, clear cell renal cell carcinoma, transitional cell carcinoma, urothelial carcinomas, Wilms tumor, leukemia, acute lymphocytic leukemia (ALL), acute myeloid leukemia (AML), chronic lymphocytic leukemia (CLL), chronic myeloid leukemia (CML), chronic myelomonocytic leukemia (CMML), liver cancer, liver carcinoma, hepatoma, hepatocellular carcinoma, cholangiocarcinoma, hepatoblastoma, Lung cancer, non-small cell lung cancer (NSCLC), mesothelioma, B-cell lymphomas, non-Hodgkin lymphoma, diffuse large B-cell lymphoma, Mantle cell lymphoma, T cell lymphomas, non-Hodgkin lymphoma, precursor T-lymphoblastic lymphomalleukemia, peripheral T cell lymphomas, multiple myeloma, nasopharyngeal carcinoma (NPC), neuroblastoma, oropharyngeal cancer, oral cavity squamous cell carcinomas, osteosarcoma, ovarian carcinoma, pancreatic cancer, pancreatic ductal adenocarcinoma, pseudopapillary neoplasms, acinar cell carcinomas. Prostate cancer, prostate adenocarcinoma, skin cancer, melanoma, malignant melanoma, cutaneous melanoma, small intestine carcinomas, stomach cancer, gastric carcinoma, gastrointestinal stromal tumor (GIST), uterine cancer, or uterine sarcoma.

Non-limiting examples of other genetic-based diseases, disorders, or conditions that are optionally evaluated using the methods and systems disclosed herein include achondroplasia, alpha-1 antitrypsin deficiency, antiphospholipid syndrome, autism, autosomal dominant polycystic kidney disease, Charcot-Marie-Tooth (CMT), cri du chat, Crohn's disease, cystic fibrosis, Dercum disease, down syndrome, Duane syndrome, Duchenne muscular dystrophy, Factor V Leiden thrombophilia, familial hypercholesterolemia, familial mediterranean fever, fragile X syndrome, Gaucher disease, hemochromatosis, hemophilia, holoprosencephaly, Huntington's disease, Klinefelter syndrome, Marfan syndrome, myotonic dystrophy, neurofibromatosis, Noonan syndrome, osteogenesis imperfecta, Parkinson's disease, phenylketonuria, Poland anomaly, porphyria, progeria, retinitis pigmentosa, severe combined immunodeficiency (scid), sickle cell disease, spinal muscular atrophy, Tay-Sachs, thalassemia, trimethylaminuria, Turner syndrome, velocardiofacial syndrome, WAGR syndrome, Wilson disease, or the like.

### B. Methods of determining a risk of cancer recurrence in a test subject and/or classifying a test subject as being a candidate for a subsequent cancer treatment

In some embodiments, a method provided herein is a method of determining a risk of cancer recurrence in a test subject. In some embodiments, a method provided herein is a method of classifying a test subject as being a candidate for a subsequent cancer treatment.

Any of such methods may comprise collecting DNA (e.g., originating or derived from a tumor cell) from the test subject diagnosed with the cancer at one or more preselected timepoints following one or more previous cancer treatments to the test subject. The subject may be any of the subjects described herein. The DNA may be cfDNA. The DNA may be obtained from a tissue sample.

Any of such methods may comprise capturing a plurality of sets of target regions from DNA from the subject, wherein the plurality of target region sets comprises a sequence-variable target region set and an epigenetic target region set, whereby a captured set of DNA molecules is produced. The capturing step may be performed according to any of the embodiments described elsewhere herein.

In any of such methods, the previous cancer treatment may comprise surgery, administration of a therapeutic composition, and/or chemotherapy.

Any of such methods may comprise sequencing the captured DNA molecules, whereby a set of sequence information is produced. The captured DNA molecules of the sequence-variable target region set may be sequenced to a greater depth of sequencing than the captured DNA molecules of the epigenetic target region set.

Any of such methods may comprise detecting a presence or absence of DNA originating or derived from a tumor cell at a preselected timepoint using the set of sequence information. The detection of the presence or absence of DNA originating or derived from a tumor cell may be performed according to any of the embodiments thereof described elsewhere herein.

Methods of determining a risk of cancer recurrence in a test subject may comprise determining a cancer recurrence score that is indicative of the presence or absence, or amount, of the DNA originating or derived from the tumor cell for the test subject. The cancer recurrence score may further be used to determine a cancer recurrence status. The cancer recurrence status may be at risk for cancer recurrence, e.g., when the cancer recurrence score is above a predetermined threshold. The cancer recurrence status may be at low or lower risk for cancer recurrence, e.g., when the cancer recurrence score is above a predetermined threshold. In particular embodiments, a cancer recurrence score equal to the predetermined threshold may result in a cancer recurrence status of either at risk for cancer recurrence or at low or lower risk for cancer recurrence.

Methods of classifying a test subject as being a candidate for a subsequent cancer treatment may comprise comparing the cancer recurrence score of the test subject with a predetermined cancer recurrence threshold, thereby classifying the test subject as a candidate for the subsequent cancer treatment when the cancer recurrence score is above the cancer recurrence threshold or not a candidate for therapy when the cancer recurrence score is below the cancer recurrence threshold. In particular embodiments, a cancer recurrence score equal to the cancer recurrence threshold may result in classification as either a candidate for a subsequent cancer treatment or not a candidate for therapy. In some embodiments, the subsequent cancer treatment comprises chemotherapy or administration of a therapeutic composition.

Any of such methods may comprise determining a disease-free survival (DFS) period for the test subject based on the cancer recurrence score; for example, the DFS period may be 1 year, 2 years, 3, years, 4 years, 5 years, or 10 years.

In some embodiments, the set of sequence information comprises sequence-variable target region sequences, and determining the cancer recurrence score may comprise determining at least a first subscore indicative of the amount of SNVs, insertions/deletions, CNVs and/or fusions present in sequence-variable target region sequences.

In some embodiments, a number of mutations in the sequence-variable target regions chosen from 1, 2, 3, 4, or 5 is sufficient for the first subscore to result in a cancer recurrence score classified as positive for cancer recurrence. In some embodiments, the number of mutations is chosen from 1, 2, or 3.

In some embodiments, the set of sequence information comprises epigenetic target region sequences, and determining the cancer recurrence score comprises determining a second subscore indicative of the changes in the epigenetic features in the epigenetic target region sequences e.g., methylation of hypermethylation variable target regions and/or perturbed fragmentation of fragmentation variable target regions, where "perturbed" means different from DNA found in a corresponding sample from a healthy subject.

In any embodiment where a cancer recurrence score is classified as positive for cancer recurrence, the cancer recurrence status of the subject may be at risk for cancer recurrence and/or the subject may be classified as a candidate for a subsequent cancer treatment.

In some embodiments, the cancer is any one of the types of cancer described elsewhere herein, e.g., colorectal cancer

### C. Therapies and Related Administration

In certain embodiments, the methods disclosed herein relate to identifying and administering customized therapies to patients given the status of a nucleic acid variant as being of somatic or germline origin. In some embodiments, essentially any cancer therapy (e.g., surgical therapy, radiation therapy, chemotherapy, and/or the like) may be included as part of these methods. Typically, customized therapies include at least one immunotherapy (or an immunotherapeutic agent). Immunotherapy refers generally to methods of enhancing an immune response against a given cancer type. In certain embodiments, immunotherapy refers to methods of enhancing a T cell response against a tumor or cancer.

In certain embodiments, the status of a nucleic acid variant from a sample from a subject as being of somatic or germline origin may be compared with a database of comparator results from a reference population to identify customized or targeted therapies for that subject. Typically, the reference population includes patients with the same cancer or disease type as the test subject and/or patients who are receiving, or who have received, the same therapy as the test subject. A customized or targeted therapy (or therapies) may be identified when the nucleic variant and the comparator results satisfy certain classification criteria (e.g., are a substantial or approximate match).

In certain embodiments, the customized therapies described herein are typically administered parenterally (e.g., intravenously or subcutaneously). Pharmaceutical compositions containing a immunotherapeutic agent are typically administered intravenously. Certain therapeutic agents are administered orally. However, customized therapies (e.g., immunotherapeutic agents, etc.) may also be administered by any method known in the art, including, for example, buccal, sublingual, rectal, vaginal, intraurethral, topical, intraocular, intranasal, and/or intraauricular, which administration may include tablets, capsules, granules, aqueous suspensions, gels, sprays, suppositories, salves, ointments, or the like.

### EXAMPLES

### Example 1: Partitioning of cell-free DNA samples

Two DNA samples (Sample 1 and Sample 2) obtained from GM12878 cell line by extracting DNA released into the culture medium (i.e., the DNA was extracted from the supernatant of the culture medium after the cells were separated out by centrifugation) are analyzed here. In this example, each cell-free DNA sample is divided into two aliquots and each aliquot has 10 ng of cell-free DNA. To one aliquot of the sample, 140 ng of carrier DNA molecules are added. In this embodiment, the carrier DNA molecules used are double stranded and about 50% of the carrier DNA molecules are methylated and 50% of carrier DNA molecules are unmethylated. Both the methylated and unmethylated carrier DNA molecules have the same sequence and the carrier DNA molecules have three CpG dinucleotides. All the cytosines in the three CpG dinucleotides of the methylated carrier DNA molecules are methylated. To the other aliquot of the sample, carrier DNA molecules are not added.

Two aliquots (with and without carrier DNA molecules) from Sample 1 and Sample 2 are combined with methyl binding domain (MBD) buffers and magnetic beads conjugated with a MBDs protein (MethylMiner Methylated DNA Enrichment Kit (ThermoFisher Scientific)) and incubated overnight. Methylated DNA molecules (if present, in the cell-free DNA samples and in the carrier DNA molecules) are bound by the MBD protein during this incubation. Non-methylated or less methylated DNA is washed away from the beads with buffers containing increasing concentrations of salt. Finally, a high salt buffer is used to wash the heavily methylated DNA away from the MBD protein. These washes result in three partitions (three partitioned sets - hypo, intermediate and hyper) of increasingly methylated DNA. The partitioned DNA present in the partitioned set comprises of DNA from the cell-free DNA sample and carrier DNA molecules. The partitioned DNA in the three partitioned sets are cleaned, to remove salt, and concentrated in preparation for the enzymatic steps of library preparation.

After concentrating the DNA in the partitioned sets, the end overhangs of partitioned DNA are extended, and adenosine residues are added to the 3' ends of fragments. The 5' end of each fragment is phosphorylated. These modifications make the partitioned DNA ligatable. DNA ligase and adapters are added to ligate each partitioned DNA molecule with an adapter on each end. These adapters contain non-unique barcodes and each partitioned set is ligated with adapters having non-unique barcodes that is distinguishable from the barcodes in the adapters used in the other partitioned sets. After ligation, the 3 partitioned sets are pooled together and are amplified by PCR. Since the ends of the carrier DNA molecules are modified to prevent ligation, the adapters will not ligated to the carrier DNA molecules. Hence, the carrier DNA molecules will not be amplified as these molecules are not ligated with the adapters and the adapters contain the primer binding site.

Following PCR, amplified DNA is again cleaned and concentrated prior to enrichment. Once concentrated, the amplified DNA is combined with salt buffer and biotinylated RNA probes targeting specific regions of interest and this mixture is incubated overnight. The biotinylated RNA probes are captured by streptavidin magnetic beads and separated from the amplified DNA that was not eluted by a series of salt washes, thereby enriching the sample. In this step, if any leftover carrier DNA molecules are still present in the sample, the probes will not bind to the carrier DNA molecules as the carrier DNA molecules do not have sequence similarity to bind to the probes (i.e., the carrier DNA molecules have a sequence from a non-human genome, in this example) and the carrier DNA molecules will not be captured, thus ensuring that the carrier DNA molecules will not be sequenced.

After enrichment, sample indexes are incorporated to the enriched molecules via PCR amplification. After PCR amplification, the amplified molecules from different samples (within a batch) are pooled together and are sequenced using Illumina NovaSeq sequencer. The sequence reads generated by the sequencer are then analyzed using bioinformatic tools/algorithms. The analysis step comprises determining the methylation status of the molecules. For example, specific regions of interest have been previously determined to be unmethylated in healthy individuals and methylated in individuals with malignant tumors. In this example, the analysis step comprises determining whether the molecules in these regions of interest are methylated or not. This is determined based on the number of CpG residues in the molecule and the partitioned set in which the molecule gets partitioned, which in turn will be used to detect the presence or absence of tumor.

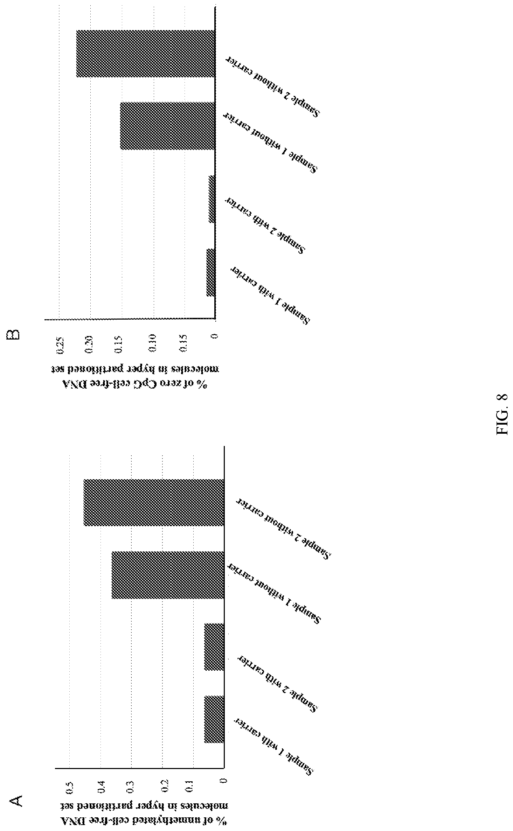

FIGs. 8A and 8B show the graphical representations of the partitioning of cell-free DNA molecules from the samples in the presence and absence of carrier DNA molecules. In the human genome, it has been found that the methylation status of certain regions does not vary/change often and always remains the same or remains consistent with different subjects and/or different types of disease/disease stages. For example, certain human genomic regions can be either predominantly methylated or predominantly unmethylated irrespective of whether the subject has cancer or not. FIG. 8A shows the percentage of cell-free DNA molecules of certain human genomic regions that are known to be predominantly unmethylated in the hyper partitioned set. FIG. 8A clearly shows that the unmethylated cell-free DNA molecules that are unlikely to be in the hyper partitioned set have partitioned into the hyper partitioned set. And addition of the carrier DNA molecules to the sample has drastically reduced the amount of cell-free DNA molecules that are unmethylated being partitioned into the hyper partitioned set. For example, in Sample 1 without carrier DNA molecules, the percentage of cell-free DNA molecules of certain human genomic regions that are known to be predominantly unmethylated in the hyper partitioned set is 0.37% (*Sample 1 without carrier* in FIG. 8A) whereas upon adding the carrier DNA molecules, it has been reduced to 0.07% (*Sample 1 with carrier* in FIG. 8A). Likewise in Sample 2, the percentage of cell-free DNA molecules (in the absence of carrier DNA molecules) of certain human genomic regions that are known to be predominantly unmethylated in the hyper partitioned set is 0.46% (*Sample 2 without carrier* in FIG. 8A) whereas upon adding the carrier DNA molecules, it has been reduced to 0.06% (*Sample 2 with carrier* in FIG. 8A). FIG. 8B shows the partitioning of cell-free DNA molecules with zero CpG dinucleotides in the hyper partitioned set. Ideally, these molecules should not be partitioned into the hyper partitioned set. FIG. 8B clearly shows that about 0.15 % and 0.22% of cell-free DNA molecules with zero CpG dinucleotides in Sample 1 (*Sample 1 without carrier* in FIG. 8B) and Sample 2 (*Sample 2 without carrier* in FIG. 8B) respectively are partitioned into the hyper partitioned set. But upon adding the carrier DNA molecules, the percentage of cell-free DNA molecules with zero CpG dinucleotides in the hyper partitioned set has been reduced to 0.014% and 0.01% in Sample 1 (S*ample 1 with carrier* in FIG. 8B) and Sample 2 (*Sample 2 with carrier* in FIG. 8B) respectively. FIG. 8 clearly shows that the use of carrier DNA molecules increases the confidence of the partitioning assay by reducing the mis-partitioning of unmethylated DNA molecules, assay noise, and hence improving the molecular specificity of the partitioning assay which will lead to improved clinical performance.

It is intended that the following claims define the scope of the invention and that methods and structures within the scope of these claims be covered thereby.

If different versions of a sequence are associated with an accession number at different times, the version associated with the accession number at the effective filing date of this application is meant. The effective filing date means the earlier of the actual filing date or filing date of a priority application referring to the accession number, if applicable. Likewise, if different versions of a publication, website or the like are published at different times, the version most recently published at the effective filing date of the application is meant, unless otherwise indicated.

## Claims

1. A method of detecting the presence or absence of tumor in a subject comprising:
(i) obtaining a polynucleotide sample from the subject;
(ii) adding a set of carrier nucleic acid molecules to the polynucleotide sample to generate a first sample; wherein the set of carrier nucleic acid molecules comprises:
(a) at least one subset of unmethylated carrier nucleic acid molecules; and
(b) at least one subset of methylated carrier nucleic acid molecules
wherein at least one end of the carrier nucleic acid molecules is modified to prevent ligation, wherein the unmethylated carrier nucleic acid molecule does not comprise methylated nucleotides, and the methylated carrier nucleic acid molecule comprises one or more methylated nucleotides;
(iii) partitioning the first sample into at least two partitioned sets using capturing agent that binds selectively to methylated polynucleotides, thereby generating a partitioned sample;
(iv) processing at least a portion of the partitioned sample to generate processed sample, wherein the processing comprises at least one of the following: (a) tagging, (b) amplifying and (c) enriching molecules for specific regions of interest;
(v) sequencing at least a portion of the processed sample to generate a set of sequencing reads; and
(vi) analyzing at least a portion of the set of sequencing reads to detect the presence or absence of tumor.

2. The method of claim 1, wherein the carrier nucleic acid molecules are between 25 bp and 325 bp in length.

3. The method of claim 1 or claim 2, wherein
a) a first subset and a second subset of the at least one subset of unmethylated carrier nucleic acid molecules comprise the same nucleotide sequence;
b) a first subset and a second subset of the at least one subset of unmethylated carrier nucleic acid molecules comprise different nucleotide sequence; or
c) a first subset and a second subset of the at least one subset of unmethylated carrier nucleic acid molecules are of different lengths.

4. The method of claim 3, wherein the first subset and the second subset of the at least one subset of unmethylated carrier nucleic acid molecules comprise one or more CpG dinucleotides in the nucleotide sequence; for example wherein:
a) position of the one or more CpG dinucleotides in the first subset is different from position of the one or more CpG dinucleotides in the second subset of the at least one subset of unmethylated carrier nucleic acid molecules;
b) number of the CpG dinucleotides in the first subset is different from number of CpG dinucleotides in the second subset of the at least one subset of unmethylated carrier nucleic acid molecules; or
c) sequence of nucleotides adjacent to the one or more CpG dinucleotides in the first subset is different from sequence of nucleotides adjacent to the one or more CpG dinucleotides in the second subset of the at least one subset of unmethylated carrier nucleic acid molecules.

5. The method of any one of the preceding claims, wherein
a) the one or more methylated nucleotides is selected from the group consisting of: (i) 5-methylcytosine, (ii) 6-methyladenine, (iii) hydroxymethyl cytosine, (iv) methyl uracil, and (v) any other methylated nucleotide; and/or
b) number of methylated nucleotides is 1, 2, 3, 4, 5, 6, 7, 8, 9, 10, 12, 14, 15, 16, 17, 18, 19 or at least 20.

6. The method of any one of the preceding claims, wherein
a) a first subset and a second subset of the at least one subset of methylated carrier nucleic acid molecules comprise same nucleotide sequence;
b) a first subset and a second subset of the at least one subset of methylated carrier nucleic acid molecules comprise different nucleotide sequence; or
c) a first subset and a second subset of the at least one subset of methylated carrier nucleic acid molecules are of different lengths.

7. The method of claim 6, wherein
a) the first subset and the second subset of the at least one subset of methylated carrier nucleic acid molecules comprise one or more CpG dinucleotides in the nucleotide sequence, for example wherein the one or more CpG dinucleotides comprises one or more methylated cytosines;
b) position of the one or more methylated nucleotides in the first subset is different from position of the one or more methylated nucleotides in the second subset of the at least one subset of methylated carrier nucleic acid molecules;
c) number of the methylated nucleotides in the first subset is different from number of methylated nucleotides in the second subset of the at least one subset of methylated carrier nucleic acid molecules; or
d) sequence of nucleotides adjacent to the one or more methylated nucleotides in the first subset is different from sequence of nucleotides adjacent to the one or more methylated nucleotides in the second subset of the at least one subset of methylated carrier nucleic acid molecules.

8. The method of any one of the preceding claims, wherein
a) amount of the at least one subset of methylated carrier nucleic acid molecules to the at least one subset of unmethylated carrier nucleic acid molecules is about 0:1, 0.1:99.9, 0.5:99.5, 0.75:99.25, 1:99, 1:95, 1:90, 1:80, 1:75, 1:70, 1:60, 1:50, 1:40, 1:30, 1:25, 1:20, 1:10, 1:5, 1:2, 1:1.15, 1:1, 1.15:1, 2:1, 5:1, 10:1, 20:1, 25:1, 30:1, 40:1, 50:1, 60:1, 70:1, 75:1, 80:1, 90:1, 95:1, 99:1,99.25:0.75, 99.5:0.5, 99.9:0.1 or 1:0 ratio; and/or
b) amount of the polynucleotide sample to the set of carrier nucleic acid molecules is about 1: 0.1; 1:0.2, 1:0.3, 1:4, 1:0.5, 1:6, 1:7, 1:8, 1:0.9, 1:1, 1:1, 1:2, 1:3, 1:4, 1:5, 1:6, 1:7, 1:8, 1:9, 1:10, 1:20, 1:30, 1:40, 1:50, 1:60, 1:70, 1:80, 1:90, 1:100, 1:200, 1:300; 1:400, 1:500, 1:600, 1:700, 1:800, 1:900, 1:1000, 1:5000, 1:10,000, 1:100,000, 1:500,000, 1:10⁶, 1:10⁷, 1:10⁸ or 1:10⁹ ratio.

9. The method of any one of the preceding claims, wherein
a) the polynucleotide sample is up to 1 µg;
b) the polynucleotide sample is up to 200 ng;
c) the polynucleotide sample is up to 150 ng; or
d) the polynucleotide sample is up to 100 ng.

10. The method of any one of the preceding claims, wherein the set of carrier nucleic acid molecules is added in a sufficient amount such that total amount of the polynucleotide sample and the set of carrier nucleic acid molecules is about 175 ng, 200 ng, 225 ng, 250 ng, 275 ng, 300 ng, 350 ng, 400 ng, 450 ng, 500 ng, 600 ng, 700 ng, 750 ng, 800 ng, 900 ng, 1 µg, 1.1 µg, 1.25 µg or 1.5 µg.

11. The method of any one of the preceding claims, wherein
a) sequence of the carrier nucleic acid molecule is selected from the group consisting of: (i) a sequence from a viral genome, (ii) a sequence from a bacterial genome, (iii) a sequence from a lambda genome, and (iv) a sequence from a non-human genome; and/or
b) the carrier nucleic acid molecule is a synthetic DNA.

12. The method of any one of the preceding claims, wherein the at least one end of the carrier nucleic acid molecules
a) comprises C3 (propyl group) spacers;
b) comprises dideoxy nucleotides; or
c) comprises any chemical modification that prevents the hydroxyl group from acting as a nucleophile.

13. The method of any one of the preceding claims, wherein the carrier nucleic acid molecules comprise uracil nucleosides;
the method optionally further comprising adding uracil deglycosylase and DNA glycosylase-lyase prior to the amplifying.

14. The method of any one of the preceding claims, wherein
a) 5' end of the carrier nucleic acid molecules comprises at least one of the following modifications: (i) inverted (5'- 5') such as dideoxy thymine, dideoxy cytosine, dideoxy guanine or dideoxy adenine; (ii) propyl group, or (iii) other organic functional groups, for example, but not limited to, benzyl, ethyl or methyl; and/or
b) 3' end of the carrier nucleic acid molecules comprises at least one of the following modifications: (i) any dideoxy base such as dideoxy thymine, dideoxy cytosine, dideoxy guanine or dideoxy adenine, that can be added enzymatically or during synthesis; (ii) propyl group, or (iii) other organic functional groups, for example, but not limited to, benzyl, ethyl or methyl.

15. The method of any one of the preceding claims, wherein
a) the carrier nucleic acid molecules are double stranded molecules;
b) the first sample is not subjected to a denaturing step;
c) the sequence of nucleotides adjacent to the one or more CpG nucleotides or methylated nucleotides in the first subset and/or the second subset can be the sequence of 1 nucleotide, 2 nucleotides, 3 nucleotides, 4 nucleotides or 5 nucleotides adjacent to the one or more CpG nucleotides or methylated nucleotides; and/or
d) the sequencing step comprises sequencing at least a portion of the processed sample from at least two partitioned sets.

16. The method of any one of the preceding claims, wherein the polynucleotide sample is obtained from blood, optionally wherein the polynucleotide sample from the blood is a cell-free DNA sample.

17. The method of any one of the preceding claims, wherein the results of the method are used as an input to generate a report, optionally wherein:
(i) the report is in a paper or electronic format;
(ii) information on, and/or information derived from, the partitioning of nucleic acid molecules, as determined by the method, is displayed in the report; and/or
(iii) the method further comprises a step of communicating the report to a third party, such as the subject from whom the sample derived or a health care practitioner.

## Patentansprüche

1. Verfahren zum Nachweis des Vorliegens oder Nichtvorliegens eines Tumors bei einem Individuum, umfassend:
(i) Erhalten einer Polynukleotidprobe von dem Individuum;
(ii) Hinzufügen eines Satzes Trägernukleinsäuremoleküle zu der Polynukleotidprobe, so dass eine erste Probe erzeugt wird; wobei der Satz Trägernukleinsäuremoleküle Folgendes umfasst:
(a) mindestens eine Teilmenge von unmethylierten Trägernukleinsäuremolekülen; und
(b) mindestens eine Teilmenge von methylierten Trägernukleinsäuremolekülen,
wobei mindestens ein Ende der Trägernukleinsäuremoleküle zum Verhindern von Ligation modifiziert ist, wobei das unmethylierte Trägernukleinsäuremolekül keine methylierten Nukleotide umfasst und das methylierte Trägernukleinsäuremolekül ein oder mehrere methylierte Nukleotide umfasst;
(iii) Teilen der ersten Probe in mindestens zwei geteilte Sätze unter Verwendung eines Einfangmittels, das selektiv an methylierte Polynukleotide bindet, wodurch eine geteilte Probe erzeugt wird;
(iv) Prozessieren wenigstens eines Teils der geteilten Probe, so dass eine prozessierte Probe erzeugt wird, wobei das Prozessieren mindestens eines der Folgenden umfasst: (a) Versehen mit einem Tag, (b) Amplifizieren und (c) Anreichern von Molekülen für spezifische Regionen von Interesse;
(v) Sequenzieren wenigstens eines Teils der prozessierten Probe, so dass ein Satz Sequenzierung-Reads erzeugt wird; und
(vi) Analysieren mindestens eines Teils des Satzes Sequenzierung-Reads zum Nachweis des Vorliegens oder Nichtvorliegens eines Tumors.

2. Verfahren nach Anspruch 1, wobei die Trägernukleinsäuremoleküle eine Länge zwischen 25 bp und 325 bp aufweist.

3. Verfahren nach Anspruch 1 oder Anspruch 2, wobei
a) eine erste Teilmenge und eine zweite Teilmenge der mindestens einen Teilmenge von unmethylierten Trägernukleinsäuremolekülen die gleiche Nukleotidsequenz umfassen;
b) eine erste Teilmenge und eine zweite Teilmenge der mindestens einen Teilmenge von unmethylierten Trägernukleinsäuremolekülen unterschiedliche Nukleotidsequenzen umfassen; oder
c) eine erste Teilmenge und eine zweite Teilmenge der mindestens einen Teilmenge von unmethylierten Trägernukleinsäuremolekülen unterschiedliche Längen aufweisen.

4. Verfahren nach Anspruch 3, wobei die erste Teilmenge und die zweite Teilmenge der mindestens einen Teilmenge von unmethylierten Trägernukleinsäuremolekülen ein oder mehrere CpG-Dinukleotide in der Nukleotidsequenz umfassen; wobei zum Beispiel:
a) die Position des einen oder der mehreren CpG-Dinukleotide in der ersten Teilmenge von der Position des einen oder der mehreren CpG-Dinukleotide in der zweiten Teilmenge der mindestens einen Teilmenge von unmethylierten Trägernukleinsäuremolekülen verschieden ist;
b) die Anzahl der CpG-Dinukleotide in der ersten Teilmenge von der Anzahl der CpG-Dinukleotide in der zweiten Teilmenge der mindestens einen Teilmenge von unmethylierten Trägernukleinsäuremolekülen verschieden ist; oder
c) die Sequenz von Nukleotiden, die an das eine oder die mehreren CpG-Dinukleotide in der ersten Teilmenge grenzen, von der Sequenz von Nukleotiden, die an das eine oder die mehreren CpG-Dinukleotide in der zweiten Teilmenge der mindestens einen Teilmenge von unmethylierten Trägernukleinsäuremolekülen grenzen, verschieden ist.

5. Verfahren nach einem der vorhergehenden Ansprüche, wobei
a) das eine bzw. die mehreren methylierte(n) Nukleotid(e) ausgewählt ist bzw. sind aus der Gruppe bestehend aus: (i) 5-Methylcytosin, (ii) 6-Methyladenin, (iii) Hydroxymethylcytosin, (iv) Methyluracil und (v) irgendeinem anderen methylierten Nukleotid; und/oder
b) die Anzahl methylierter Nukleotide 1, 2, 3, 4, 5, 6, 7, 8, 9, 10, 12, 14, 15, 16, 17, 18, 19 oder mindestens 20 beträgt.

6. Verfahren nach einem der vorhergehenden Ansprüche, wobei
a) eine erste Teilmenge und eine zweite Teilmenge der mindestens einen Teilmenge von methylierten Trägernukleinsäuremolekülen die gleiche Nukleotidsequenz umfassen;
b) eine erste Teilmenge und eine zweite Teilmenge der mindestens einen Teilmenge von methylierten Trägernukleinsäuremolekülen unterschiedliche Nukleotidsequenzen umfassen; oder
c) eine erste Teilmenge und eine zweite Teilmenge der mindestens einen Teilmenge von methylierten Trägernukleinsäuremolekülen unterschiedliche Längen aufweisen.

7. Verfahren nach Anspruch 6, wobei
a) die erste Teilmenge und die zweite Teilmenge der mindestens einen Teilmenge von methylierten Trägernukleinsäuremolekülen ein oder mehrere CpG-Dinukleotide in der Nukleotidsequenz umfassen, wobei zum Beispiel das eine bzw. die mehreren CpG-Dinukleotid(e) ein oder mehrere methylierte Cytosine umfasst bzw. umfassen;
b) die Position des einen oder der mehreren methylierten Nukleotide in der ersten Teilmenge von der Position des einen oder der mehreren methylierten Nukleotide in der zweiten Teilmenge der mindestens einen Teilmenge von methylierten Trägernukleinsäuremolekülen verschieden ist;
c) die Anzahl der methylierten Nukleotide in der ersten Teilmenge von der Anzahl der methylierten Nukleotide in der zweiten Teilmenge der mindestens einen Teilmenge von methylierten Trägernukleinsäuremolekülen verschieden ist; oder
d) die Sequenz von Nukleotiden, die an das eine oder die mehreren methylierten Nukleotide in der ersten Teilmenge grenzen, von der Sequenz von Nukleotiden, die an das eine oder die mehreren methylierten Nukleotide in der zweiten Teilmenge der mindestens einen Teilmenge von methylierten Trägernukleinsäuremolekülen grenzen, verschieden ist.

8. Verfahren nach einem der vorhergehenden Ansprüche, wobei
a) die Menge der mindestens einen Teilmenge von methylierten Trägernukleinsäuremolekülen zu der mindestens einen Teilmenge von unmethylierten Trägernukleinsäuremolekülen im Verhältnis von etwa 0:1, 0,1:99,9, 0,5:99,5, 0,75:99,25, 1:99, 1:95, 1:90, 1:80, 1:75, 1:70, 1:60, 1:50, 1:40, 1:30, 1:25, 1:20, 1:10, 1:5, 1:2, 1:1,15, 1:1, 1,15:1, 2:1, 5:1, 10:1, 20:1, 25:1, 30:1, 40:1, 50:1, 60:1, 70:1, 75:1, 80:1, 90:1, 95:1, 99:1, 99,25:0,75, 99,5:0,5, 99,9:0,1 oder 1:0 vorliegt; und/oder
b) die Menge der Polynukleotidprobe zu dem Satz Trägernukleinsäuremoleküle im Verhältnis von etwa 1:0,1; 1:0,2, 1:0,3, 1:4, 1:0,5, 1:6, 1:7, 1:8, 1:0,9, 1:1, 1:1, 1:2, 1:3, 1:4, 1:5, 1:6, 1:7, 1:8, 1:9, 1:10, 1:20, 1:30, 1:40, 1:50, 1:60, 1:70, 1:80, 1:90, 1:100, 1:200, 1:300; 1:400, 1:500, 1:600, 1:700, 1:800, 1:900, 1:1000, 1:5000, 1:10.000, 1:100.000, 1:500.000, 1:10⁶, 1:10⁷, 1:10⁸ oder 1:10⁹ vorliegt.

9. Verfahren nach einem der vorhergehenden Ansprüche, wobei
a) die Polynukleotidprobe bis zu 1 µg groß ist;
b) die Polynukleotidprobe bis zu 200 ng groß ist;
c) die Polynukleotidprobe bis zu 150 ng groß ist; oder
d) die Polynukleotidprobe bis zu 100 ng groß ist.

10. Verfahren nach einem der vorhergehenden Ansprüche, wobei der Satz Trägernukleinsäuremoleküle in einer hinreichenden Menge zugegeben wird, so dass die Gesamtmenge der Polynukleotidprobe und des Satzes Trägernukleinsäuremoleküle etwa 175 ng, 200 ng, 225 ng, 250 ng, 275 ng, 300 ng, 350 ng, 400 ng, 450 ng, 500 ng, 600 ng, 700 ng, 750 ng, 800 ng, 900 ng, 1 µg, 1,1 µg, 1,25 µg oder 1,5 µg beträgt.

11. Verfahren nach einem der vorhergehenden Ansprüche, wobei
a) die Sequenz des Trägernukleinsäuremoleküls ausgewählt ist aus der Gruppe bestehend aus: (i) einer Sequenz aus einem Virusgenom, (ii) einer Sequenz aus einem Bakteriengenom, (iii) einer Sequenz aus einem Lambda-Genom und (iv) einer Sequenz aus einem nichtmenschlichen Genom; und/oder
b) es sich bei dem Trägernukleinsäuremolekül um eine synthetische DNA handelt.

12. Verfahren nach einem der vorhergehenden Ansprüche, wobei das mindestens eine Ende der Trägernukleinsäuremoleküle
a) C3(Propylgruppe)-Spacer umfasst;
b) Didesoxynukleotide umfasst; oder
c) irgendeine chemische Modifikation, die verhindert, dass die Hydroxylgruppe als Nukleophil wirkt, umfasst.

13. Verfahren nach einem der vorhergehenden Ansprüche, wobei die Trägernukleinsäuremoleküle Uracilnukleoside umfassen;
wobei das Verfahren gegebenenfalls ferner Zugeben von Uracil-Deglykosylase und DNA-Glykosylase-Lyase vor dem Amplifizieren umfasst.

14. Verfahren nach einem der vorhergehenden Ansprüche, wobei
a) das 5'-Ende der Trägernukleinsäuremoleküle mindestens eine der folgenden Modifikationen umfasst: (i) invertierte (5'- 5') wie Didesoxythymin, Didesoxycytosin, Didesoxyguanin oder Didesoxyadenin; (ii) Propylgruppe oder (iii) andere organische funktionelle Gruppen, zum Beispiel, aber nicht beschränkt auf Benzyl, Ethyl oder Methyl; und/oder
b) das 3'-Ende der Trägernukleinsäuremoleküle mindestens eine der folgenden Modifikationen umfasst: (i) irgendeine Didesoxybase wie Didesoxythymin, Didesoxycytosin, Didesoxyguanin oder Didesoxyadenin, die sich enzymatisch oder während der Synthese addieren lässt; (ii) Propylgruppe oder (iii) andere organische funktionelle Gruppen, zum Beispiel, aber nicht beschränkt auf Benzyl, Ethyl oder Methyl.

15. Verfahren nach einem der vorhergehenden Ansprüche, wobei
a) es sich bei den Trägernukleinsäuremolekülen um Doppelstrangmoleküle handelt;
b) die erste Probe keinem Denaturierungsschritt unterzogen wird;
c) es sich bei der Sequenz von Nukleotiden, die an das eine oder die mehreren CpG-Nukleotide oder methylierten Nukleotide in der ersten Teilmenge und/oder der zweiten Teilmenge grenzen, um die Sequenz von 1 Nukleotid, 2 Nukleotiden, 3 Nukleotiden, 4 Nukleotiden oder 5 Nukleotiden, die an das eine oder die mehreren CpG-Nukleotide oder methylierten Nukleotide grenzen, handeln kann; und/oder
d) der Sequenzierungsschritt Sequenzieren wenigstens eines Teils der prozessierten Probe aus mindestens zwei geteilten Sätzen umfasst.

16. Verfahren nach einem der vorhergehenden Ansprüche, wobei die Polynukleotidprobe aus Blut erhalten wird, gegebenenfalls wobei es sich bei der Polynukleotidprobe aus dem Blut um eine zellfreie DNA-Probe handelt.

17. Verfahren nach einem der vorhergehenden Ansprüche, wobei die Ergebnisse des Verfahrens als Eingabe zur Erstellung einen Berichts verwendet werden, gegebenenfalls wobei:
(i) der Bericht in Papierform oder einem elektronischen Format vorliegt;
(ii) Informationen über die Teilung von Nukleinsäuremolekülen und/oder Informationen, die daraus gewonnen wurden, wie mit dem Verfahren bestimmt, im Bericht präsentiert werden; und/oder
(iii) das Verfahren ferner einen Schritt der Übermittlung des Berichts an einen Dritten, etwa das Individuum, von dem die Probe stammte, oder eine medizinsche Fachperson, umfasst.

## Revendications

1. Procédé de détection de la présence ou de l'absence d'une tumeur chez un sujet comprenant :
(i) l'obtention d'un échantillon de polynucléotide du sujet ;
(ii) l'ajout d'un ensemble de molécules d'acides nucléiques porteuses à l'échantillon de polynucléotide pour produire un premier échantillon ; l'ensemble de molécules d'acides nucléiques porteuses comprenant :
(a) au moins un sous-ensemble de molécules d'acides nucléiques porteuses non méthylées ; et
(b) au moins un sous-ensemble de molécules d'acides nucléiques porteuses méthylées
dans lequel au moins une extrémité des molécules d'acides nucléiques porteuses est modifiée pour empêcher une ligature, la molécule d'acide nucléique porteuse non méthylée ne comprenant pas de nucléotides méthylés, et la molécule d'acide nucléique porteuse méthylée comprenant un ou plusieurs nucléotides méthylés ;
(iii) le partitionnement du premier échantillon en au moins deux ensembles partitionnés au moyen d'un agent de capture qui se lie sélectivement à des polynucléotides méthylés, produisant ainsi un échantillon partitionné ;
(iv) le traitement d'au moins une partie de l'échantillon partitionné pour produire un échantillon traité, le traitement comprenant au moins l'un parmi les suivants : (a) marquage, (b) amplification et (c) enrichissement de molécules pour des régions d'intérêt spécifiques ;
(v) le séquençage d'au moins une partie de l'échantillon traité pour générer un ensemble de lectures de séquençage ; et
(vi) l'analyse d'au moins une partie de l'ensemble de lectures de séquençage pour détecter la présence ou l'absence de tumeur.

2. Procédé selon la revendication 1, dans lequel les molécules d'acides nucléiques porteuses ont une longueur comprise entre 25 pb et 325 pb.

3. Procédé selon la revendication 1 ou la revendication 2, dans lequel
a) un premier sous-ensemble et un second sous-ensemble de l'au moins un sous-ensemble de molécules d'acides nucléiques porteuses non méthylées comprennent la même séquence nucléotidique ;
b) un premier sous-ensemble et un second sous-ensemble de l'au moins un sous-ensemble de molécules d'acides nucléiques porteuses non méthylées comprennent une séquence nucléotidique différente ; ou
c) un premier sous-ensemble et un second sous-ensemble de l'au moins un sous-ensemble de molécules d'acides nucléiques porteuses non méthylées sont de longueurs différentes.

4. Procédé selon la revendication 3, dans lequel le premier sous-ensemble et le second sous-ensemble de l'au moins un sous-ensemble de molécules d'acides nucléiques porteuses non méthylées comprennent un ou plusieurs dinucléotides CpG dans la séquence nucléotidique ; par exemple dans lequel :
a) la position du ou des dinucléotides CpG dans le premier sous-ensemble est différente de la position du ou des dinucléotides CpG dans le second sous-ensemble de l'au moins un sous-ensemble de molécules d'acides nucléiques porteuses non méthylées ;
b) le nombre de dinucléotides CpG dans le premier sous-ensemble est différent du nombre de dinucléotides CpG dans le second sous-ensemble de l'au moins un sous-ensemble de molécules d'acides nucléiques porteuses non méthylées ; ou
c) la séquence de nucléotides adjacente au ou aux dinucléotides CpG dans le premier sous-ensemble est différente de la séquence de nucléotides adjacente au ou aux dinucléotides CpG dans le second sous-ensemble de l'au moins un sous-ensemble de molécules d'acides nucléiques porteuses non méthylées.

5. Procédé selon l'une quelconque des revendications précédentes, dans lequel
a) le ou les nucléotides méthylés sont choisis dans le groupe constitué par : (i) 5-méthylcytosine, (ii) 6-méthyladénine, (iii) hydroxyméthylcytosine, (iv) méthyluracile, et (v) tout autre nucléotide méthylé ; et/ou
b) le nombre de nucléotides méthylés est 1, 2, 3, 4, 5, 6, 7, 8, 9, 10, 12, 14, 15, 16, 17, 18, 19 ou au moins 20.

6. Procédé selon l'une quelconque des revendications précédentes, dans lequel
a) un premier sous-ensemble et un second sous-ensemble de l'au moins un sous-ensemble de molécules d'acides nucléiques porteuses méthylées comprennent la même séquence nucléotidique ;
b) un premier sous-ensemble et un second sous-ensemble de l'au moins un sous-ensemble de molécules d'acides nucléiques porteuses méthylées comprennent une séquence nucléotidique différente ; ou
c) un premier sous-ensemble et un second sous-ensemble de l'au moins un sous-ensemble de molécules d'acides nucléiques porteuses méthylées sont de longueurs différentes.

7. Procédé selon la revendication 6, dans lequel
a) le premier sous-ensemble et le second sous-ensemble de l'au moins un sous-ensemble de molécules d'acides nucléiques porteuses méthylées comprennent un ou plusieurs dinucléotides CpG dans la séquence nucléotidique, par exemple, le ou les dinucléotides CpG comprenant une ou plusieurs cytosines méthylées ;
b) la position du ou des nucléotides méthylés dans le premier sous-ensemble est différente de la position du ou des nucléotides méthylés dans le second sous-ensemble de l'au moins un sous-ensemble de molécules d'acides nucléiques porteuses méthylées ;
c) le nombre de nucléotides méthylés dans le premier sous-ensemble est différent du nombre de nucléotides méthylés dans le second sous-ensemble de l'au moins un sous-ensemble de molécules d'acides nucléiques porteuses méthylées ; ou
d) la séquence de nucléotides adjacente au ou aux nucléotides méthylés dans le premier sous-ensemble est différente de la séquence de nucléotides adjacente au ou aux nucléotides méthylés dans le second sous-ensemble de l'au moins un sous-ensemble de molécules d'acides nucléiques porteuses méthylées.

8. Procédé selon l'une quelconque des revendications précédentes, dans lequel
a) la quantité de l'au moins un sous-ensemble de molécules d'acides nucléiques porteuses méthylées par rapport à l'au moins un sous-ensemble de molécules d'acides nucléiques porteuses non méthylées est d'un rapport d'environ 0:1, 0,1:99,9, 0,5:99,5,0,75:99,25, 1:99, 1:95, 1:90, 1:80, 1:75, 1:70, 1:60, 1:50, 1:40, 1:30, 1:25, 1:20, 1:10, 1:5, 1:2, 1:1,15, 1:1, 1,15:1, 2:1, 5:1, 10:1, 20:1, 25:1, 30:1, 40:1, 50:1, 60:1, 70:1, 75:1, 80:1, 90:1, 95:1, 99:1, 99,25:0,75, 99,5:0,5, 99,9:0,1 ou 1:0 ; et/ou
b) la quantité de l'échantillon de polynucléotide par rapport à l'ensemble des molécules d'acides nucléiques porteuses est d'un rapport d'environ 1:0,1 ; 1:0,2, 1:0,3, 1:4, 1:0,5, 1:6, 1:7, 1:8, 1:0,9, 1:1, 1:1, 1:2, 1:3, 1:4, 1:5, 1:6, 1:7, 1:8, 1:9, 1:10, 1:20, 1:30, 1:40, 1:50, 1:60, 1:70, 1:80, 1:90, 1:100, 1:200, 1:300 ; 1:400, 1:500, 1:600, 1:700, 1:800, 1:900, 1:1000, 1:5000, 1:10 000, 1:100 000, 1:500 000, 1:10⁶, 1:10⁷, 1:10⁸ ou 1:10⁹.

9. Procédé selon l'une quelconque des revendications précédentes, dans lequel
a) l'échantillon de polynucléotide est jusqu'à 1 µg ;
b) l'échantillon de polynucléotide est jusqu'à 200 ng ;
c) l'échantillon de polynucléotide est jusqu'à 150 ng ; ou
d) l'échantillon de polynucléotide est jusqu'à 100 ng.

10. Procédé selon l'une quelconque des revendications précédentes, dans lequel l'ensemble de molécules d'acides nucléiques porteuses est ajouté en une quantité suffisante telle que la quantité totale de l'échantillon de polynucléotide et l'ensemble de molécules d'acides nucléiques porteuses soit d'environ 175 ng, 200 ng, 225 ng, 250 ng, 275 ng, 300 ng, 350 ng, 400 ng, 450 ng, 500 ng, 600 ng, 700 ng, 750 ng, 800 ng, 900 ng, 1 µg, 1,1 µg, 1,25 µg ou 1,5 µg.

11. Procédé selon l'une quelconque des revendications précédentes, dans lequel
a) la séquence de la molécule d'acide nucléique porteuse est choisie dans le groupe constitué par : (i) une séquence d'un génome viral, (ii) une séquence d'un génome bactérien, (iii) une séquence d'un génome lambda, et (iv) une séquence d'un génome non humain ; et/ou
b) la molécule d'acide nucléique porteuse est un ADN synthétique.

12. Procédé selon l'une quelconque des revendications précédentes, dans lequel l'au moins une extrémité des molécules d'acides nucléiques porteuses
a) comprend des espaceurs C3 (groupe propyle) ;
b) comprend des didésoxynucléotides ; ou
c) comprend toute modification chimique qui empêche le groupe hydroxyle d'agir comme nucléophile.

13. Procédé selon l'une quelconque des revendications précédentes, dans lequel les molécules d'acides nucléiques porteuses comprennent des nucléosides d'uracile ;
le procédé comprenant en outre éventuellement l'ajout d'uracile déglycosylase et d'ADN glycosylase-lyase avant l'amplification.

14. Procédé selon l'une quelconque des revendications précédentes, dans lequel
a) l'extrémité 5' des molécules d'acides nucléiques porteuses comprend au moins une des modifications suivantes : (i) (5'- 5') inversées telles que la didésoxythymine, la didésoxycytosine, la didésoxyguanine ou la didésoxyadénine ; (ii) groupe propyle, ou (iii) autres groupes fonctionnels organiques, par exemple, mais sans s'y limiter, benzyle, éthyle ou méthyle ; et/ou
b) l'extrémité 3' des molécules d'acides nucléiques porteuses comprend au moins une des modifications suivantes : (i) toute didésoxybase telle que la didésoxythymine, la didésoxycytosine, la didésoxyguanine ou la didésoxyadénine, qui peut être ajoutée enzymatiquement ou pendant la synthèse ; (ii) groupe propyle, ou (iii) autres fonctionnels organiques, par exemple, mais sans s'y limiter, benzyle, éthyle ou méthyle.

15. Procédé selon l'une quelconque des revendications précédentes, dans lequel
a) les molécules d'acides nucléiques porteuses sont des molécules à double brin ;
b) le premier échantillon n'est pas soumis à une étape de dénaturation ;
c) la séquence de nucléotides adjacente au ou aux nucléotides CpG ou nucléotides méthylés dans le premier sous-ensemble et/ou le second sous-ensemble peut être la séquence de 1 nucléotide, 2 nucléotides, 3 nucléotides, 4 nucléotides ou 5 nucléotides adjacents au ou aux nucléotides CpG ou nucléotides méthylés ; et/ou
d) l'étape de séquençage comprend le séquençage d'au moins une partie de l'échantillon traité à partir d'au moins deux ensembles partitionnés.

16. Procédé selon l'une quelconque des revendications précédentes, dans lequel l'échantillon de polynucléotide est obtenu à partir de sang, éventuellement dans lequel l'échantillon de polynucléotide provenant du sang est un échantillon d'ADN acellulaire.

17. Procédé selon l'une quelconque des revendications précédentes, dans lequel les résultats du procédé sont utilisés comme entrée pour générer un rapport, éventuellement dans lequel :
(i) le rapport est sur un format papier ou électronique ;
(ii) des informations sur le et/ou des informations dérivées du, partitionnement des molécules d'acides nucléiques, comme déterminé par le procédé, sont affichées dans le rapport ; et/ou
(iii) le procédé comprend en outre une étape de communication du rapport à un tiers, tel que le sujet dont l'échantillon provient ou un praticien de la santé.
